# EUROPEAN PATENT APPLICATION

(11) **EP 3 819 300 A1**
(43) Date of publication of application: **12.05.2021**
(21) Application number: 19925556.3
(22) Date of filing: 06.09.2019
(51) Int. Cl.: C07D 495/04, A61K 31/4365, C07K 16/28, A61P 35/00

(54) **BIOMARKER-BASED THERAPEUTIC COMPOSITION**

(71) Applicant: Wellmarker Bio Co., Ltd., Seoul 05855 (KR)
(72) Inventor: HONG, Seung-Woo, Seoul 05855 (KR); MOON, Jai-Hee, Seoul 05855 (KR); SHIN, Jae-Sik, Seoul 05855 (KR); KIM, Joseph, Seoul 05855 (KR); PARK, Yoon-Sun, Seoul 05855 (KR); LEE, Min-Ki, Seoul 05855 (KR); JEONG, Joon-Yee, Seoul 05855 (KR); LEE, So-Hee, Seoul 05855 (KR); CHOI, Soon-Jin, Seoul 05855 (KR)
(74) Representative: HGF
(86) International application number: PCT/KR2019/011570
(87) International publication number: WO 2021/045279

(57) **Abstract**

The present invention provides an anticancer agent for treating a patient who is resistant to a protein kinase inhibitor, the anticancer agent comprising, as an active ingredient, a thienopyridine derivative compound or a pharmaceutically acceptable salt thereof. Here, the patient may be a patient carrying active RON. In addition, the patient may be a patient carrying normal KRAS. In addition, the anticancer agent may be applied to a patient who is resistant to an EGFR inhibitor. In particular, the anticancer agent may be usefully used to treat a patient who is resistant to the therapeutic agent cetuximab.

## Description

### Technical Field

The present invention relates to a thienopyridine derivative applicable to a cancer patient carrying active RON kinase, and to a pharmaceutical composition comprising the same.

### Background Art

Cetuximab is an inhibitor of epidermal growth factor receptor (EGFR). In particular, since EGFR is overexpressed in cancer cells of metastatic colon cancer, metastatic non-small cell lung cancer, and head and neck cancer, cetuximab is mainly used for the treatment of such diseases. However, cetuximab cannot be used for patients who are resistant to the anticancer agent. Resistance to cetuximab occurs for several reasons. Typically, cancer cells with mutations in a protein such as KRAS which is located in the EGFR signaling system, are resistant to cetuximab. Therefore, a new anticancer agent suitable for patients who are resistant to conventional anticancer agents is required for effective cancer treatment.

Meanwhile, one of the most important proteins in intracellular signaling systems is a protein kinase. The protein kinases phosphorylate other proteins to regulate the activity, location, and function of the proteins, thereby controlling intracellular signaling processes. Examples of the protein kinase include AB1, ACK, ALK, ARG, ARK5, AURORA, AX1, BMX, c-MET, c-RAF, c-SRC, EGFR, FAK, FES, FGFR, FLT3, GSK3, IGF, IKK, JAK, LCK, LIMK, LYN, MEK, MER, MK-2, RET, RON, ROS, RSE, and the like. Mutations in these protein kinases may lead to diseases such as cancer, immune diseases, neurological diseases, metabolic diseases, and infections.

Among these, abnormal c-MET activation in cancer cells has been reported to be closely related to aggravation of the prognosis of anticancer therapy. In particular, overexpression and mutations of c-MET have been observed in several cancer diseases such as non-small cell lung cancer (NSCLC). Tumor invasion and metastasis are the leading cause of death in cancer patients, and thus inhibiting c-MET signaling may be effective in cancer treatment. In particular, RON (recepteur d'origine nantais) refers to a protein receptor belonging to the c-MET family, and is a receptor for macrophage-stimulating protein (MSP), a serum protein, which is secreted by the liver and regulates the action of macrophages. The activity of RON plays an important role in the development, progression, and metastasis of tumors. In particular, overexpression or hyperactivity thereof in colon cancer and breast cancer has been reported to contribute to inducing tumor invasion and metastasis and inhibiting apoptosis.

Therefore, a substance capable of specifically inhibiting activity of abnormally activated RON may be used to effectively treat various diseases related to RON, in particular, tumors such as colon cancer. Accordingly, there is an urgent need to develop an anticancer agent that can inhibit activity of abnormally activated RON.

### Disclosure of Invention

### Technical Problem

An object of the present invention is to provide a pharmaceutical composition for treating cancer, comprising as an active ingredient a compound capable of inhibiting activity of abnormally activated RON or a pharmaceutically acceptable salt thereof. In particular, an object of the present invention is to provide a pharmaceutical composition that can effectively exert its action in a patient who is resistant to an anticancer agent such as cetuximab.

### Solution to Problem

In order to achieve the above objects, there is provided an anticancer agent for treating a patient who is resistant to a protein kinase inhibitor such as cetuximab, the anticancer agent comprising, as an active ingredient, a thienopyridine derivative compound represented by Formula 1 or a pharmaceutically acceptable salt thereof.

### Advantageous Effects of Invention

The anticancer pharmaceutical composition according to the present disclosure is applicable to cancer patients carrying active RON kinase. In particular, the pharmaceutical composition may be usefully used to treat patients who are resistant to cetuximab that is conventionally used for anticancer therapy.

### Brief Description of Drawings

Fig. 1 is a graph showing cell death rate (%) depending on concentrations (1 µM and 5 µM) of sample compounds in a colon cancer tumor cell line (KM12C) carrying RON mutant (Δ160).
Fig. 2 illustrates results obtained by subjecting the KM12C cell line carrying RON mutant (Δ160) to treatment with Example 18 at multiple concentrations, and then identifying decreased expression of active RON (pTyr-RON).
Fig. 3 illustrates results obtained by performing kinase assay for RON mutant (Δ160).
Fig. 4 illustrates results obtained by performing kinase assay for RON mutant (Δ155).
Fig. 5 illustrates results obtained by causing Colo320HSR cell line to overexpress RON mutants (mt #1: Δ160, mt #2: Δ155), and then identifying efficacy of Example 18.
Fig. 6 illustrates results obtained by causing Colo320HSR cell line to overexpress RON mutants (mt #1: Δ160, mt #2: Δ155), and then identifying efficacy of Example 18 with Western blotting.
Fig. 7 illustrates results obtained by subjecting the KM12C cell line carrying RON mutant (Δ160) to treatment with Example 18, and then identifying decreased expression of active RON (pTyr-RON) so that the mechanism of action of Example 18 is analyzed.
Fig. 8 illustrates results obtained by identifying tumor growth inhibition efficacy of Example 18 in an animal model transplanted with the KM12C cell line.
Fig. 9 illustrates results obtained by identifying tumor growth inhibition efficacy of Example 18 in an animal model transplanted with the KM12C cell line.
Fig. 10 illustrates results obtained by identifying changes in mouse body weight in a case where Example 18 is administered to an animal model transplanted with the KM12C cell line.
Fig. 11 illustrates results obtained by identifying changes in expression of active RON (pTyr-RON), p-ERK, and cleaved caspase3 in a case where an animal model transplanted with the KM12C cell line is subjected to treatment with Example 18.
Fig. 12 illustrates results obtained by administering Example 18 to an animal model transplanted with the KM12C cell line, extracting tumors after the end of the administration, and identifying changes in expression of active RON (pTyr-RON) and cleaved caspase3.
Fig. 13 illustrates results obtained by analyzing tumor growth inhibition efficacy of Example 18 in a case where Example 18 is administered to an animal model transplanted with the KM12C cell.
Fig. 14 illustrates results obtained by administering Example 18 to an animal model transplanted with the KM12C cell, and then analyzing the condition of mice and the size of tumors extracted from the respective mice.
Fig. 15 illustrates results obtained by observing changes in mouse body weight in a case where Example 18 is administered to an animal model transplanted with the KM12C cell line.
Fig. 16 illustrates results obtained by identifying an effective dose concentration in an animal model transplanted with the KM12C cell line.
Fig. 17 illustrates results obtained by identifying an effective dose concentration in an animal model transplanted with the KM12C cell line.
Fig. 18 illustrates results obtained by analyzing efficacy of Example 18 in an animal model transplanted with a colon cancer patient's tissue.
Fig. 19 illustrates results obtained by administering Example 18 to mice transplanted with a colon cancer patient's tissue, and then analyzing the condition of the mice and the size of tumors extracted from the respective mice.
Fig. 20 illustrates results obtained by analyzing efficacy of Example 18 in an animal model transplanted with a colon cancer patient's tissue.
Fig. 21 illustrates results obtained by analyzing the size of tumors extracted from an animal model transplanted with a colon cancer patient's tissue.
Fig. 22 illustrates results obtained by analyzing cell killing efficacy of Example 18, depending on the expression of active RON, in colon cancer patient-derived cell lines.
Fig. 23 illustrates results obtained by analyzing efficacy of Example 18, depending on the expression of active RON, in colon cancer patient-derived cell lines.
Fig. 24 illustrates results obtained by analyzing cell killing efficacy of Example 18 in a colon cancer patient-derived cell line in which RON is not expressed.
Fig. 25 illustrates results obtained by analyzing efficacy of Example 18 in a colon cancer patient-derived cell line in which RON is not expressed.
Fig. 26 illustrates results obtained by performing genetic analysis for biomarkers (RON mutants) in colon cancer cell lines.
Fig. 27 illustrates results obtained by performing genetic analysis for RON mutants in a Korean colon cancer patient group.
Fig. 28 illustrates results obtained by performing genetic analysis for RON and KRAS mutants in a Korean colon cancer patient group.
Fig. 29 illustrates results obtained by performing genetic analysis for RON mutants in a Caucasian colon cancer patient group.
Fig. 30 illustrates results obtained by performing genetic analysis for RON and KRAS mutants in a Caucasian colon cancer patient group.
Fig. 31 illustrates results obtained by performing genetic analysis to identify RON and KRAS mutants in tissues from an animal model transplanted with tumor cells in colon cancer patients.

### Best Mode for Carrying out the Invention

Hereinafter, the present invention will be described in more detail.

### Definition of terms

As used herein, the term "halogen" refers to F, Cl, Br, or I, unless otherwise stated.

The term "alkyl," unless otherwise specified, refers to a linear or branched saturated hydrocarbon radical. For example, "C₁₋₁₀ alkyl" means an alkyl having a skeleton consisting of 1 to 10 carbon atoms. Specifically, C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "haloalkyl" refers to an alkyl substituted with one or more halogen atoms. Specifically, haloalkyl may be an alkyl substituted with two or more halogen atoms of the same kind or substituted with two or more kinds of halogen atoms.

The term "heterocycle" refers to an aromatic or non-aromatic ring having one or more heteroatoms, which may be saturated or unsaturated and may be monocyclic or polycyclic. For example, "4- to 10-membered heterocycle" means a heterocycle having a skeleton consisting of 4 to 10 atoms in total which include a heteroatom and a carbon atom. Specifically, examples of the 4- to 10-membered heterocycle may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, and the like.

The term "heteroatom" refers to an atom other than carbon (C), and may specifically be nitrogen (N), oxygen (O), or sulfur (S) atom.

The term "substitution" refers to replacing a hydrogen atom in a molecular structure with a substituent such that a chemically stable compound results therefrom without exceeding the valence on the designated atom. For example, the phrase "group A is substituted with substituent B" means that a hydrogen atom bonded to an atom such as carbon atom constituting the skeleton of group A is replaced with substituent B, so that group A and substituent B form a covalent bond.

### Pharmaceutical composition

In an aspect of the present invention, there is provided a pharmaceutical composition for treating a cancer patient who is resistant to a protein kinase inhibitor, the composition comprising, as an active ingredient, a compound represented by Formula 1, or a pharmaceutically acceptable salt thereof:

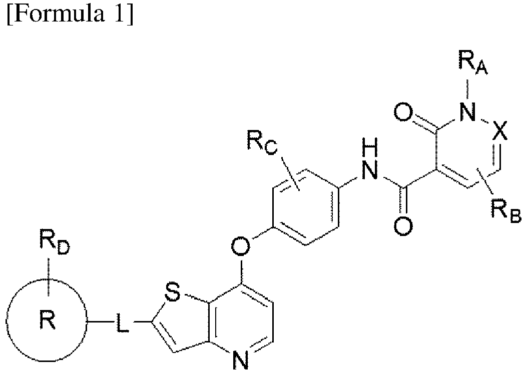

In the formula,
R_{A} is C₁₋₁₀ alkyl, phenyl, or benzyl, where R_{A} optionally has one or more substituents selected from halogen and C₁₋₁₀alkoxy;
X is -C(-R_{B}')= or -N=;
R_{B} and R_{B}' are each independently H, halogen, C₁₋₁₀ alkyl, or C₁₋₁₀ alkoxy;
Rc is H, halogen, or C₁₋₁₀ alkyl;
L is a single bond or C₁₋₆ alkylene;
R is a 5- to 8-membered heterocycle having 1 or 2 heteroatoms selected from N and S;
R_{D} is H, C₁₋₁₀ alkyl, -(CH₂)ₙY-R_{G}, or -CH₂-NR_{E}R_{F};
R_{E} and R_{F} are each independently H, C₁₋₁₀ alkyl, or -(CH₂)ₙ-Y-R_{G}, or R_{E} and R_{F} are linked to each other to form a 4- to 10-membered heterocycle together with the N atom to which they are attached;
wherein n is an integer from 0 to 10; Y is -O-, -C(=O)-, -C(=O)-O-, -S-, or - S(=O)₂-; R_{G} is linear or branched C₁₋₁₀ alkyl, provided that R_{G} is unsubstituted or substituted with one or more substituents selected from the group consisting of halogen, amino, hydroxyl, and C₁₋₆ alkoxy; and
the 4- to 10-membered heterocycle optionally further contains one or two heteroatoms selected from the group consisting of N, O, and S, in addition to the N atom to which R_{E} and R_{F} are attached, and is unsubstituted or substituted with one or more substituents selected from halogen and C₁₋₆ alkyl.

According to an embodiment, in Formula 1, R_{A} may be methyl, phenyl, halobenzyl, or halophenyl.

According to an embodiment, in Formula 1, R_{B} and R_{B}' may be each independently H, methyl, methoxy, or ethoxy.

According to an embodiment, in Formula 1, X may be -C(-R_{B}')=; and R_{B} and R_{B}' may be each independently H, methyl, methoxy, or ethoxy, and, optionally, R_{B} and R_{B}' are not H at the same time.

According to an embodiment, in Formula 1, Rc may be H or halogen. Here, the halogen may be F, Cl, Br, or I.

According to an embodiment, in Formula 1, L may be a single bond or methylene; and R may be imidazolyl, pyrazolyl, pyridinyl, piperazinyl, or thiazolyl.

According to an embodiment, in Formula 1, R_{D} may be H, methyl, ethyl, methoxymethyl, or -CH₂-NR_{E}R_{F}; and R_{E} and R_{F} may be each independently H, C₁₋₆ alkyl, or -C₁₋₆ alkylene-O-C₁₋₁₀ alkyl, and, optionally, R_{E} and R_{F} are not H at the same time.

According to an embodiment, in Formula 1, R_{E} and R_{F} together with the N atom to which they are attached may form 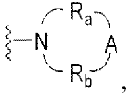 where Rₐ and R_{b} may be each independently C₁₋₃ alkylene; and A may be -N(-C₁₋₆ alkyl)- or -O-.

According to an embodiment, in Formula 1, R_{A} may be methyl, phenyl, halobenzyl, or halophenyl; X may be -CH=; R_{B} may be H, methyl, methoxy, or ethoxy; Rc may be H or halogen; L may be a single bond or methylene; R may be imidazolyl, pyrazolyl, pyridinyl, thiazolyl, or piperazinyl; R_{D} may be H, methyl, ethyl, methoxymethyl, or -CH₂-NR_{E}R_{F}, where R_{E} may be -C₂H₄-O-CH₃ and R_{F} may be H or methyl, or R_{E} and R_{F} may be linked to each other to form morpholine or methylpiperazinyl together with the N atom to which they are attached. Here, the halogen may be F, Cl, Br, or I.

The present invention includes pharmaceutically acceptable salts of the compounds represented by Formula 1. The pharmaceutically acceptable salts should have low toxicity to humans and should not have any adverse effects on the biological activity and physicochemical properties of their parent compounds.

For example, the pharmaceutically acceptable salt may be an acid addition salt formed with a pharmaceutically acceptable free acid. As the free acid, an inorganic acid or an organic acid may be used, where the inorganic acid may be hydrochloric acid, sulfuric acid, nitric acid, phosphoric acid, perchloric acid, bromic acid, or the like; and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, or the like.

The acid addition salt may be prepared by a conventional method, for example, by dissolving the compound of Formula 1 in an excess amount of an acidic aqueous solution and precipitating the resulting salt using a water-miscible organic solvent such as methanol, ethanol, acetone, or acetonitrile.

In addition, the pharmaceutically acceptable salt may be an alkali metal salt (for example, sodium salt) or an alkaline earth metal salt (for example, potassium salt). The alkali metal salt or alkaline earth metal salt may be obtained, for example, by dissolving the compound of Formula 1 in an excess amount of an alkali metal hydroxide or alkaline earth metal hydroxide solution, filtering off the undissolved compound salt, and then evaporating and drying the filtrate.

Further, the compounds of the present invention may have a chiral carbon center, and thus may exist in the form of R or S isomers, racemic compounds, individual enantiomers or mixtures thereof, or individual diastereomers or mixtures thereof. All such stereoisomers and mixtures thereof may fall within the scope of the present invention.

In addition, the compounds of the present invention may include hydrates and solvates of the compounds of Formula 1. The hydrates and solvates may be prepared using known methods and are preferably non-toxic and water-soluble. In particular, the hydrates and solvates may preferably be formed by being combined with one to five molecules of water and an alcoholic solvent (in particular, ethanol, or the like), respectively.

According to an embodiment, specific examples of the compound represented by Formula 1 are as follows:
1) 4-Ethoxy-N-(3-fluoro-4-(2-(1-methyl-1H-imidazol-4-yl)thieno[3,2-b]pyridin-7-yloxy)phenyl)-1-(4-fluorophenyl)-2-oxo-1,2-dihydropyridine-3-carboxamide;
2) 4-Ethoxy-N-(3-fluoro-4-(2-(1-methyl-1H-imidazol-4-yl)thieno[3,2-b]pyridin-7-yloxy)phenyl)-1-methyl-2-oxo-1,2-dihydropyridine-3-carboxamide;
3) 4-Ethoxy-N-(3-fluoro-4-(2-(1-methyl-1H-imidazol-2-yl)thieno[3,2-b]pyridin-7-yloxy)phenyl)-1-(4-fluorophenyl)-2-oxo-1,2-dihydropyridine-3-carboxamide;
4) 4-Ethoxy-N-(3-fluoro-4-(2-(1-methyl-1H-pyrazol-4-yl)thieno[3,2-b]pyridin-7-yloxy)phenyl)-1-(4-fluorophenyl)-2-oxo-1,2-dihydropyridine-3-carboxamide;
5) 4-Ethoxy-N-(3-fluoro-4-(2-(pyridin-2-yl)thieno[3,2-b]pyridin-7-yloxy)phenyl)-1-(4-fluorophenyl)-2-oxo-1,2-dihydropyridine-3-carboxamide;
6) 4-Ethoxy-N-(3-fluoro-4-(2-(pyridin-3-yl)thieno[3,2-b]pyridin-7-yloxy)phenyl)-1-(4-fluorophenyl)-2-oxo-1,2-dihydropyridine-3-carboxamide;
7) 4-Ethoxy-N-(3-fluoro-4-(2-(thiazol-2-yl)thieno[3,2-b]pyridin-7-yloxy)phenyl)-1-(4-fluorophenyl)-2-oxo-1,2-dihydropyridine-3-carboxamide;
8) 4-Ethoxy-N-(4-(2-(1-ethyl-1H-imidazol-4-yl)thieno[3,2-b]pyridin-7-yloxy)-3-fluorophenyl)-1-(4-fluorobenzyl)-2-oxo-1,2-dihydropyridine-3-carboxamide;
9) 4-Methoxy-N-(3-fluoro-4-(2-(1-(methoxymethyl)-1H-imidazol-4-yl)thieno [3,2-b]pyridine-7-yloxy)phenyl)-1-phenyl-2-oxo-1,2-dihydropyridine-3-carboxamide;
10) 4-Ethoxy-N-(3-fluoro-4-(2-(piperazin-1-ylmethyl)thieno[3,2-b]pyridin-7-yloxy)phenyl)-1-(4-fluorophenyl)-2-oxo-1,2-dihydropyridine-3-carboxamide;
11) 4-Ethoxy-N-(3-fluoro-4-(2-((4-methylpiperazin-l-yl)methyl)thieno[3,2-b]pyridin-7-yloxy)phenyl)-1-(4-fluorophenyl)-2-oxo-1,2-dihydropyridine-3-carboxamide;
12) 4-Ethoxy-N-(3-fluoro-4-{ [2-(5-{ [(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide;
13) 4-Ethoxy-N-(3-fluoro-4-{ [2-(5-{ [(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3-carboxamide;
14) N-(3-Chloro-4-{ [2-(5-{ [(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3-carboxamide;
15) 4-Ethoxy-N-(3-fluoro-4-{ [2-(5-{ [(2-methoxyethyl)amino]methyl}pyridin-2-yl)thieno[3,2-b]pyridin-7-yl]oxy}phenyl)-1-(3-fluorophenyl)-2-oxo-1,2-dihydropyridine-3-carboxamide;
16) N-(3-Fluoro-4-{ [2-(5-{ [(2-methoxyethyl)amino]methyl}pyridin-2-yl)thieno[3,2-b]pyridine-7-yl]oxy}phenyl)-1-(4-fluorophenyl)-4-methyl-2-oxo-1,2-dihydropyridine-3-carboxamide;
17) 4-Ethoxy-N-(3-fluoro-4-{ [2-(5-{ [(2-methoxyethyl) (methyl)amino]methyl }pyridin-2-yl)thieno[3,2-b]pyridin-7-yl]oxy}phenyl)-1-(4-fluorophenyl)-2-oxo-1,2-dihydropyridine-3-carboxamide;
18) 4-Ethoxy-N-[3-fluoro-4-({2-[5-(morpholinomethyl)pyridin- 2-yl]thieno[3,2-blpyridin-7-yl}oxy)phenyl]-1-(4-fluorophenyl)-2-oxo-1,2-dihydropyridine-3-carboxamide;
19) 4-Ethoxy-N-[3-fluoro-4-([2-[5-(morpholinomethyl)pyridin-2-yl]thieno[3,2-b]pyridin-7-yl}oxy)phenyl]-2-oxo-1-phenyl-1,2-dihydropyridine-3-carboxamide;
20) 4-Ethoxy-N-{3-fluoro-4-[(2-{5-[(4-methylpiperazin-1-yl)methyl]pyridin-2-yl}thieno[3,2-b]pyridin-7-yl)oxy]phenyl}-1-(4-fluorophenyl)-2-oxo-1,2-dihydropyridine-3-carboxamide; and
21) 4-Ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

### Application range of pharmaceutical composition

The protein kinases are enzymes that phosphorylate other proteins to regulate the activity, location, and function of the proteins, thereby controlling various intracellular processes. Abnormalities in control function of these protein kinases are closely associated with disease mechanisms such as in cancer, immune diseases, neurological diseases, metabolic diseases, and infections. Examples of the protein kinase include AB1, ACK, ALK, ARG, ARK5, AURORA, AXI, BMX, BTK, CDK, CHK, c-KIT, c-MET, c-RAF, c-SRC, EGFR, FAK, FES, FGFR, FLT3, GSK3, IGF, IKK, JAK, LCK, LIMK, LYN, MEK, MER, MK-2, P38ALPHA, PDGFR, PDK, PIM, PKA, PKB, PKCR, PLK-1/3, RET, ROS, RSE, TIE, TRK, TYRO3, VEGFR, YES, and the like. Here, the protein kinase may preferably be EGFR.

In addition, a cancer patient to which the pharmaceutical composition is applicable may carry active RON (Recepteur d'Origine Nantais). In addition, the patient may be a patient carrying normal KRAS. Preferably, the pharmaceutical composition may be applied to a patient carrying active RON and normal KRAS. In particular, the patient may be a patient who is resistant to cetuximab.

The RON (Recepteur d'Origine Nantais) refers to a protein receptor belonging to the MET (c-MET) family, and is a receptor for MSP, a serum protein, which is secreted by the liver and regulates the action of macrophages. As used herein, the term "active RON" means RON which is activated at all times, unlike normal RON. The activity degree of such RON is regulated by alternative splicing, one of the important processes for regulating gene expression in eukaryotes.

Examples of the active RON may include RONΔ155, RONΔ160, RONA165, and the like. The active RON is a form generated by skipping of exons through splicing and is constitutively activated at all times without any ligand. Therefore, an embodiment of the active RON carried by the cancer patient may be, but is not limited to, any one selected from the group consisting of RONΔ155, RONΔ160, RONA165, and combinations thereof. In addition, the active RON may be mutated RON. The "mutated RON" means that some amino acids of RON are substituted, deleted, or inserted so that RON is activated at all times. In addition, in a case where MSP is present, even normal RON is also activated. The pharmaceutical composition of the present invention may act on the active RON, thereby inhibiting activity of the active RON.

In addition, the pharmaceutical composition may be a composition for treating any one cancer selected from the group consisting of breast cancer, lung cancer, gastric cancer, prostate cancer, uterine cancer, ovarian cancer, kidney cancer, pancreatic cancer, liver cancer, colon cancer, skin cancer, head and neck cancer, and thyroid cancer. Here, the cancer may carry active RON. Preferably, the cancer may carry any one active RON selected from the group consisting of RONΔ155, RONΔ160, and RONΔ165.

### Formulation of pharmaceutical composition

The pharmaceutical composition of the present invention may contain, as an active ingredient, the compound represented by Formula 1 or a pharmaceutically acceptable salt thereof, in an amount of about 0.1 wt% to about 90 wt%, specifically about 0.1 wt% to about 75 wt%, and more specifically about 1 wt% to about 50 wt%, based on the total weight of the composition.

The pharmaceutical composition of the present invention may contain conventional, non-toxic pharmaceutically acceptable additives which are combined into preparations according to conventional methods. For example, the pharmaceutical composition may further contain a pharmaceutically acceptable carrier, diluent, or excipient.

Examples of additives used in the composition of the present invention may include sweeteners, binders, solvents, solubilizers, wetting agents, emulsifiers, isotonic agents, absorbents, disintegrants, antioxidants, preservatives, lubricants, glidants, fillers, flavors, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, tragacanth gum, alginic acid, sodium alginate, methyl cellulose, sodium carboxymethyl cellulose, agar, water, ethanol, polyethylene glycol, polyvinylpyrrolidone, sodium chloride, calcium chloride, orange essence, strawberry essence, vanilla flavor, and the like.

The composition of the present invention may be formulated in various preparation forms for oral administration (for example, tablets, pills, powders, capsules, syrups, or emulsions) or parenteral administration (for example, intramuscular, intravenous, or subcutaneous injection).

Preferably, the composition of the present invention may be formulated into preparations for oral administration, and examples of the additives for this purpose may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like. Specifically, examples of the glidant include colloidal silicon dioxide, magnesium silicate, and the like; examples of the diluent include microcrystalline cellulose, Fast Flo lactose, lactose anhydrous, lactose monohydrate, silicified MCC HD 90, and the like; examples of the disintegrant include croscarmellose sodium, crospovidone, and the like; and examples of the lubricant include magnesium stearate, sodium lauryl sulfate, stearic acid, and the like.

Further, as liquid preparations for oral administration, suspensions, emulsions, syrups, and the like may be exemplified, and the liquid preparations may contain various excipients such as wetting agents, sweeteners, fragrances, preservatives, and the like, in addition to water and liquid paraffin which are commonly used as simple diluents.

In addition, examples of preparations for parenteral administration may include sterilized aqueous solutions, non-aqueous solutions, suspensions, emulsions, freeze-dried preparations, and suppositories. For the non-aqueous solutions and the suspensions, propylene glycol, polyethylene glycol, vegetable oil such as olive oil, injectable ester such as ethyl oleate, or the like may be used. As the suppository base, Witepsol™, macrogol, Tween™ 61, cacao butter, laurin fat, glycerogelatin, or the like may be used. On the other hand, injections may contain conventional additives such as solubilizers, isotonic agents, suspending agents, emulsifiers, stabilizers, and preservatives.

### Dosage and usage of pharmaceutical composition

The compound or composition of the present invention may be administered to a patient in a therapeutically effective amount or in a pharmaceutically effective amount.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. The level of the effective amount may be determined depending on factors including the patient's health condition, type and severity of disease, activity of drug, the patient's sensitivity to drug, mode of administration, time of administration, route of administration and excretion rate, duration of treatment, formulation or simultaneously used drugs, and other factors well known in the medical field.

The compound or composition of the present invention may be administered as an individual therapeutic agent or in combination with other therapeutic agents, may be administered sequentially or simultaneously with a conventional therapeutic agent, and may be administered in single or multiple doses. It is important that taking into account all of the above factors, the amount which is a minimum amount that allows a maximum effect to be obtained without adverse effects is administered, and such an amount may be easily determined by those skilled in the art.

Specifically, the effective amount of the compound in the composition of the present invention may vary depending on the patient's age, sex, and body weight; and in general, about 0.1 mg to about 1,000 mg or about 5 mg to about 200 mg per kg body weight may be administered daily or every other day, or once to three times a day. However, the effective amount may be increased or decreased depending on route of administration, severity of disease, the patient's sex, body weight, age, and the like, and thus the scope of the present invention is not limited thereto.

Preferably, the compound or composition of the present invention may be administered for tumor therapy in combination with chemotherapy, radiation therapy, immunotherapy, hormone therapy, bone marrow transplantation, stem cell replacement therapy, other biological therapies, surgical intervention, or combinations thereof. For example, the compound or composition of the present invention may be used as adjunctive therapy in conjunction with other long-term treatment strategies, or may be used to maintain the patient's condition after tumor regression or chemoprophylactic therapy in severe patients.

### Pharmaceutical composition intended for combined administration

The pharmaceutical composition of the present invention may further contain at least one active ingredient, and examples of the further-contained active ingredient may include, but are not limited to, anti-proliferative compounds such as aromatase inhibitors, anti-estrogens, topoisomerase I inhibitors, topoisomerase II inhibitors, microtubule active compounds, alkylating compounds, histone deacetylase inhibitors, compounds that induce cell differentiation processes, cyclooxygenase inhibitors, MMP inhibitors, mTOR inhibitors, anti-neoplastics, anti-metabolites, platin compounds, compounds that target/decrease activity of proteins or lipid kinases, anti-angiogenic compounds, compounds that target, decrease, or inhibit activity of proteins or lipid phosphatases, gonadorelin agonists, anti-androgens, methionine aminopeptidase inhibitors, bisphosphonates, biological response modifiers, anti-proliferative antibodies, heparanase inhibitors, Ras oncogenic isotype inhibitors, telomerase inhibitors, proteasome inhibitors, compounds used for treatment of hematologic malignancies, compounds which target, decrease or inhibit activity of Flt-3, Hsp90 inhibitors, kinesin spindle protein inhibitors, MEK inhibitors, leucovorin, EDG binding agents, anti-leukemia compounds, ribonucleotide reductase inhibitors, S-adenosylmethionine decarboxylase inhibitors, hemostatic steroids, corticosteroids, other chemotherapeutic compounds, and photosensitizing compounds.

The further-contained active ingredient may be a known anticancer agent. Non-limiting examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anticancer antibiotics such as dactinomycin (actinomycin D), doxorubicin (adriamycin), daunorubicin, idarubicin, mitoxantrone, plicamycin, mitomycin C, and bleomycin; and plant alkaloids such as vincristine, vinblastine, paclitaxel, docetaxel, etoposide, teniposide, topotecan, and irinotecan; and the like.

In another aspect of the present invention, there is provided a pharmaceutical composition for treating cancer, comprising the compound represented by Formula 1 or a pharmaceutically acceptable salt thereof, wherein the pharmaceutical composition further contains a protein kinase inhibitor.

### Treatment method using pharmaceutical composition

In yet another aspect of the present invention, there is provided a method for treating a patient who is resistant to a protein kinase inhibitor, comprising steps of i) identifying whether a cancer patient carries active RON; and ii) administering, to a patient carrying active RON, an anticancer agent that comprises, as an active ingredient, the compound represented by Formula I as described above, or a pharmaceutically acceptable salt thereof.

Here, the protein kinase is as described above, and the active RON may be any one selected from the group consisting of RONΔ155, RONΔ160, RONA165, and combinations thereof.

### Mode for the Invention

Hereinafter, the present invention will be described in more detail by way of the following examples. However, the following examples are intended to only illustrate the present invention for easier understanding, and the scope of the present invention is not limited only thereto.

### Preparation Example 1: Preparation of 4-ethoxy-1-(4-fluorophenyl)-2-oxo-1,2-dihydropyridine-3-carboxylic acid

### Step 1: Preparation of ethyl 4-ethoxy- 2-oxo-1,2-dihydropyridine- 3-carboxylate

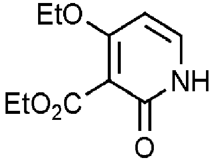

Triethyl orthoacetate (249.6 mL, 1.32 mmol) and acetic acid (19.6 mL, 0.33 mol) were added to ethyl cyanoacetate (70.5 mL, 0.66 mol), and stirring was performed at 120°C for 12 hours or longer. The reaction mixture was concentrated and N,N-dimethylformamide diethyl acetal (DMFDEA) (141 mL, 0.55 mol) was added thereto. Stirring was performed at 70°C for 2 hours or longer. 500 mL of acetic acid and 60 mL of distilled water were added to the reaction mixture, and the resultant was refluxed for 12 hours or longer. The reaction mixture was cooled to room temperature, and saturated aqueous sodium bicarbonate solution and water were added thereto. The resultant was extracted with a mixed solvent of dichloromethane and methanol (9:1). The organic layer was dried over anhydrous magnesium sulfate, filtered, and concentrated under reduced pressure. 100 mL of ethyl acetate was added thereto, and the resultant was concentrated. The resulting solid was filtered to obtain the compound, ethyl 4-ethoxy-2-oxo-1,2-dihydropyridine-3-carboxylate (37 g, yield: 26%).
¹H NMR (400 MHz, DMSO-d₆) δ 11.61 (bs, 1H), 7.46 (d, J = 7.6 Hz, 1H), 6.21 (d, J = 7.6 Hz, 1H), 4.14(m, 4H), 1.22 (m, 6H)
MS (ESI) m/z 212.12 (M + H)⁺

### Step 2: Preparation of ethyl 4-ethoxy-1-(4-fluorophenyl)-2-oxo-1,2-dihydropyridine-3-carboxylate

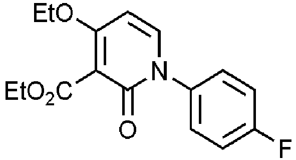

100 mL of N,N-dimethylformamide was added to cesium carbonate (114 g, 0.35 mol), and the resultant was filled with nitrogen gas. Stirring was stirred at room temperature for 10 minutes. The compound obtained in Step 1, ethyl 4-ethoxy-2-oxo-1,2-dihydropyridine-3-carboxylate (10.2 g, 0.07 mol), dissolved in 200 mL of N,N-dimethylformamide, was added to the mixture, and copper iodide (10 g, 0.05 mol), 58.3 g (0.26 mol) of 4-fluoro-iodobenzene, and the compound (37 g, 0.17 mol) obtained above were added thereto. Stirring was performed at 110°C for 24 hours. After completion of the reaction, the resultant was cooled to room temperature, ethyl acetate was added thereto, and stirring was performed for 10 minutes. The reaction mixture was filtered through a celite pad and extracted with water and ethyl acetate. The organic layer was dried over anhydrous magnesium sulfate and filtered. Then, the solvent was removed under reduced pressure.

### Step 3: Preparation of 4-ethoxy-1-(4-fluorophenyl)-2-oxo-1,2-dihydropyridine-3-carboxylic acid

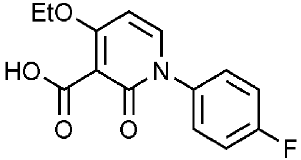

The compound of Step 2 from which the solvent was completely removed was dissolved in 200 mL of ethanol, and 400 mL of 3N aqueous hydrogen chloride solution was added thereto. Stirring was performed at 60°C for 24 hours. The resulting solid was filtered to obtain 4-ethoxy-1-(4-fluorophenyl)-2-oxo-1,2-dihydropyridine-3-carboxylic acid (21 g, two-step yield: 43%).
¹H NMR (400 MHz, DMSO-d₆) δ 7.95 (d, J = 76 Hz, 1H), 7.48 (m, 2H), 7.35 (m, 2H), 6.58 (d, J = 8 Hz, 1H), 4.28 (q, J = 68 Hz, 2H), 1.32 (t, J = 68 Hz, 3H)
MS (ESI) m/z 276.09 (M + H)⁺

### Preparation Example 2: Preparation of 4-ethoxy-2-oxo-1-phenyl-1,2-dihydropyridine-3-carboxylic acid

The following compound was prepared in the same manner as in Preparation Example 1, except that iodobenzene was used in place of of 4-fluoro-iodobenzene in Step 2 of Preparation Example 1. 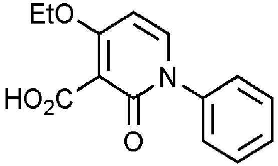 ¹H NMR (400 MHz, DMSO-d6) δ 7.98 (d, J = 8 Hz, 1H), 7.56-7.41 (m, 5H), 6.60 (d, J = 7.6 Hz, 1H), 4.30(q, J = 7.2 Hz, 2H), 1.34 (t, J = 7.2 Hz, 3H)
MS (ESI) m/z 260.05 (M + H)⁺

### Preparation Example 3: Preparation of 1-(4-fluorophenyl)-4-methoxy-2-oxo-1,2-dihydropyridine-3-carboxylic acid

### Step 1: Preparation of methyl 4-methoxy-1-(4-fluorophenyl)-2-oxo-1,2-dihydropyridine-3-carboxylate

Ethyl 4-ethoxy-1-(4-fluorophenyl)-2-oxo-1,2-dihydropyridine-3-carboxylate (0.37 g, 1.20 mmol) was subjected to treatment with 28% NaOMe solution, and then stirred at room temperature for 10 minutes. Removal of solvent was done by concentration under reduced pressure to obtain the following compound (0.26 g, yield: 77%).

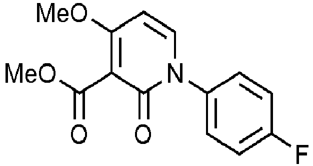

### Step 2: Preparation of 4-methoxy-1-(4-fluorophenyl)-2-oxo-1,2-dihydropyridine-3-carboxylic acid

Methyl 4-methoxy-1-(4-fluorophenyl)-2-oxo-1,2-dihydropyridine-3-carboxylate (0.33 g, 1.18 mmol) was subjected to treatment with 5 mL of ethanol, and then 10 mL of 2.75 N hydrochloric acid solution was added dropwise thereto at room temperature. Stirring was performed at 60°C for 4 hours, and then the resulting solid was filtered to obtain the following compound (0.16 g, yield: 52%). 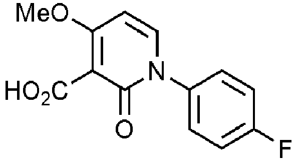 ¹H NMR (400 MHz, DMSO-d₆) δ 13.89 (bs, 1H), 8.02 (d, J = 7.6 Hz, 1H), 7.53-7.48 (m, 2H), 7.40-7.34 (m, 2H), 6.63 (d, J = 8 Hz, 1H), 4.02 (s, 3H)
MS (ESI) m/z 264.06 (M + H)⁺

### Preparation Example 4: Preparation of 4-methoxy-2-oxo-1-phenyl-1,2-dihydropyridine-3-carboxylic acid

The following compound was prepared in the same manner as in Preparation Example 3, except that ethyl 4-ethoxy-1-(4-fluorophenyl)-2-oxo-1,2-dihydropyridine-3-carboxylate used in Preparation Example 2 was used in place of ethyl 4-ethoxy-1-phenyl-2-oxo-1,2-dihydropyridine-3-carboxylate in Step 1 of Preparation Example 3. 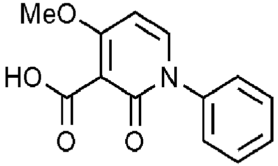 ¹H NMR (400 MHz, DMSO-d₆) δ 7.96 (d, J = 7.8 Hz, 1H), 7.55-7.45 (m, 5H), 6.58 (d, J = 78 Hz, 1H), 3.99 (s, 3H)
MS (ESI) m/z 246.06 (M + H)⁺

### Preparation Example 5: Preparation of 4-ethoxy-1-methyl-2-oxo-1,2-dihydropyridine-3-carboxylic acid

30 mL of N,N-dimethylformamide and cesium carbonate (15.4 g, 0.05 mol) were added to the compound (ethyl 4-ethoxy-2-oxo-1,2-dihydropyridine-3-carboxylate) (5 g, 23.7 mmol) obtained in Step 1 of Preparation Example 1, and stirring was performed at room temperature for 10 minutes. Iodomethane (6.7 g, 47.3 mmol) was added thereto and stirring was performed at room temperature for 5 hours. After completion of the reaction, the mixture was concentrated, 50 mL of ethyl acetate was added thereto, and then the resultant was concentrated again. 50 mL of isopropyl ether was added to the concentrated mixture and stirring was performed. The resultant was filtered to obtain a solid in the form of a slurry. 20 mL of tetrahydrofuran and 10 mL of 3N aqueous hydrogen chloride solution were added to the obtained solid. The resultant was stirred at 80°C for 5 hours and concentrated. 180 mL of dichloromethane and 10 mL of methanol were added thereto, and the organic layer was separated. Anhydrous magnesium sulfate was added to the separated organic layer, and the resultant was filtered to obtain a filtrate.

The obtained filtrate was concentrated, and the resulting solid was filtered to obtain the following compound (3 g, yield: 64%). 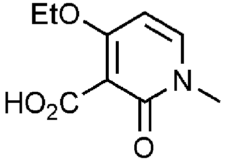 ¹H NMR (400 MHz, DMSO-d₆) δ 8.04 (d, J = 8 Hz, 1H), 6.56 (d, J = 7.6 Hz, 1H), 4.24 (q, J = 6.8 Hz, 1H), 1.32 (t, J = 7.2 Hz, 1H)
MS (ESI) m/z 198.17 (M + H)⁺

### Preparation Example 6: Preparation of 4-ethoxy-1-(4-fluorobenzyl)-2-oxo-1,2-dihydropyridine-3-carboxylic acid

The following compound was prepared in the same manner as in Preparation Example 5, except that 4-fluorobenzylbromide and sodium hydride were used in place of iodomethane and cesium carbonate. 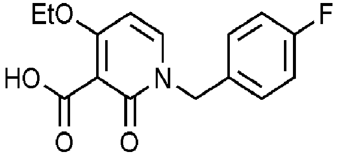 ¹H NMR (400 MHz, CDCl₃) δ 7.50 (d, J = 7.6 Hz, 1H), 7.31 (d, J = 5.2 Hz, 2H), 7.29 (d, J = 5.2 Hz, 2H), 6.29 (d, J = 7.6 Hz, 1H), 5.15 (s, 2H), 4.28 (q, J = 7.2 Hz, 2H), 1.52 (t, J = 7.2 Hz, 3H)
MS (ESI) m/z 289.98 (M + H)⁺

### Preparation Example 7: Preparation of 4-ethoxy-1-(3-fluorophenyl)-2-oxo-1,2-dihydropyridine-3-carboxylic acid

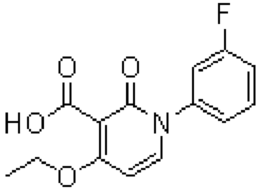

Preparation was done according to the synthesis route of Preparation Example 1, except that 3-fluoro-iodobenzene was used in place of 4-fluoro-iodobenzene in Step 2, to obtain the title compound (192 mg, yield: 73%, white solid).
¹H NMR (500 MHz, DMSO-d₆) δ 13.70 (brs, 1H), 7.99 (d, J = 10.0 Hz, 1H), 7.60-7.56 (m, 1H), 7.43 (dt, J = 10.0 and 5.0 Hz, 1H), 7.36 (td, J = 10.0 and 5.0 Hz, 1H), 7.30 (dd, J = 10.0 and 5.0 Hz, 1H), 6.61 (d, J = 10.0 Hz, 1H), 4.30 (qt, J = 5.0 Hz, 2H), 1.34 (t, J = 5.0 Hz, 3H)

### Preparation Example 8: Preparation of 4-methyl-2-oxo-1-(4-fluorophenyl)-1,2-dihydropyridine-3-carboxylic acid

### Step 1: Synthesis of ethyl 2-(4-fluoroanilinocarbonyl)-3-methyl-2-butylate

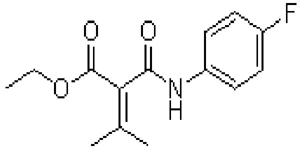

A mixture of 4-fluoroaniline (0.20 mL, 2.05 mmol), diethyl isopropylidenemalonate (1.60 mL, 8.20 mmol), and imidazole (139 mg, 2.05 mmol) was stirred at 200°C for 5 hours. The resultant was cooled to room temperature, and concentrated under reduced pressure. The resulting residue was purified by column chromatography to obtain the title compound (151 mg, yield: 28%, yellow oil).
¹H NMR (500 MHz, DMSO-d₆) δ 10.25 (s, 1H), 7.65-7.62 (m, 2H), 7.17-7.13 (m, 2H), 4.12 (qt, J = 5.0 Hz, 2H), 2.16 (s, 3H), 1.88 (s, 3H), 1.16 (t, J = 5.0 Hz, 3H)

### Step 2: Synthesis of ethyl 4-methyl-2-oxo-1-(4-fluorophenyl)-1,2-dihydropyridine-3-carboxylate

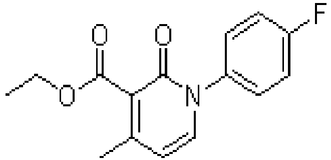

A mixture of the compound (96 mg, 0.36 mmol) prepared in Step 1 and 1,1-dimethoxy-N,N-dimethylmethanamine (72 µL, 0.54 mmol) were stirred at 100°C for 1 hour. The resultant was cooled to room temperature, and concentrated under reduced pressure. The resulting residue was purified by column chromatography to obtain the title compound (34 mg, yield: 34%, brown solid).
¹H NMR (500 MHz, CDCl₃) δ 7.36-7.33 (m, 2H), 7.27-7.26 (m, 1H), 7.17-7.13 (m, 2H), 6.13 (d, J = 5.0 Hz, 1H), 4.39 (qt, J = 5.0 Hz, 2H), 2.28 (s, 3H), 1.37 (t, J = 5.0 Hz, 3H)

### Step 3: Synthesis of 4-methyl-2-oxo-1-(4-fluorophenyl)-1,2-dihydropyridine-3-carboxylic acid

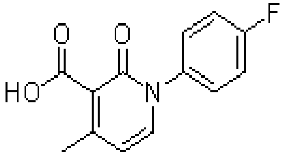

Reaction was allowed to proceed in the same manner as in Step 3 of Preparation Example 1, except that the compound (34 mg, 0.12 mmol) prepared in Step 2 was used as a starting material, thereby obtaining the title compound (29 mg, yield: 100%, yellow solid).
¹H NMR (500 MHz, DMSO-d₆) δ 7.90 (d, J = 10.0 Hz, 1H), 7.56-7.52 (m, 2H), 7.42-7.37 (m, 2H), 6.56 (d, J = 10.0 Hz, 1H), 2.52 (s, 3H)

### Example 1: Preparation of 4-ethoxy-N-(3-fluoro-4-(2-(1-methyl-1H-imidazol-4-yl)thieno[3,2-b]pyridine-7-yloxy)phenyl)-1-(4-fluorophenyl)-2-oxo-1,2-dihydropyridine-3-carboxamide

### Step 1: Preparation of 7-chlorothieno[3,2-b]pyridine

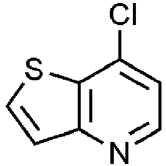

25 mL of phosphoryl chloride was heated to 60°C and thieno[3,2-b]pyridin-7-ol (15 g, 99.2 mmol) was added thereto. Stirring was performed at the same temperature for 1 hour, and then the resultant was cooled to room temperature. The reaction mixture was concentrated under reduced pressure, and 100 mL of ice water was added thereto so that complete dissolution was allowed to occur. Ammonia water was added thereto to achieve basification, and the resulting solid was filtered. The filtered solid was dissolved in ethyl acetate and filtered. The filtrate was dried over anhydrous magnesium sulfate and concentrated under reduced pressure, to obtain the white solid, 7-chlorothieno[3,2-b]pyridine (15 g, 89%).
¹H NMR (400 MHz, DMSO-d₆) δ 8.66 (d, J = 4.8 Hz, 1H), 8.26 (d, J = 56 Hz, 1H), 7.67 (d, J = 5.6 Hz, 1H), 7.58 (d, J = 4.8 Hz, 1H)
MS (ESI) m/z 169.92 (M + H)⁺

### Step 2: Preparation of 7-chloro-2-(1-methyl-1H-imidazol-4-yl)thieno[3,2-b]pyridine

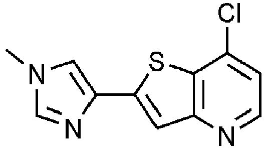

7-Chlorothieno[3,2-b]pyridine (50 g, 294.8 mmol) was dissolved in 500 mL of tetrahydrofuran, and then the resultant was cooled to -78°C. n-Butyl lithium (25 M hexane solution) (118 mL, 294.8 mmol) was added slowly thereto at the same temperature, and stirring was performed for 10 minutes. Zinc chloride (1M diethyl ether solution) (295 mL, 294.8 mmol) was added slowly thereto and stirring was performed for 10 minutes. The temperature of the resultant was slowly elevated to room temperature, and stirring was performed for 1 hour. Then, tetrakistriphenylphosphinepalladium (28 g, 29.5 mmol) and 4-iodo-1-methyl-1H-imidazole (51 g, 245.6 mmol) were added thereto, and the resultant was stirred at reflux for 4 hours. The reaction mixture was cooled to room temperature and concentrated under reduced pressure. 500 mL of ethyl acetate and 20 mL of water were added thereto, stirring was performed, and the resultant was filtered through a celite pad. The filtrate was concentrated under reduced pressure, dichloromethane was added thereto, and then the resultant was acidified with 1N aqueous hydrogen chloride solution. The resulting solid was filtered. The filtered solid was washed with ammonia water and water to obtain the off-white solid, 7-chloro-2-(1-methyl-1H-imidazol-4-yl)thieno[3,2-b]pyridine (29 g, yield: 39 %).
¹H NMR (400 MHz, DMSO-d₆) δ 8.55 (d, J = 4.8 Hz, 1H), 7.90 (S, 1H), 7.75 (s, 1H), 7.74 (s, 1H), 7.45(d, J = 4.8 Hz, 1H), 3.73 (s, 3H)
MS (ESI) m/z 249.98 (M + H)+, 252.02 (M + Na)⁺

### Step 3: Preparation of 7-(2-fluoro-4-nitrophenoxy)-2-(1-methyl-1H-imidazol-4-yl)thieno[3,2-b]pyridine

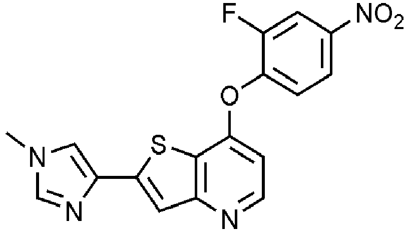

64 g (464.5 mmol) of anhydrous potassium carbonate and 2-fluoro-4-nitrophenol (36 g, 232.2 mmol) were added to a mixture of 200 mL of diphenylether and 7-chloro-2-(1-methyl-1H-imidazol-4-yl)thieno[3,2-b]pyridine (29 g, 116.1 mmol). The reaction mixture was heated to 200°C and stirred for 3 hours. The resultant was cooled to room temperature, water was added thereto, and stirring was performed. The resulting solid was filtered to obtain 7-(2-fluoro-4-nitrophenoxy)-2-(1-methyl-1H-imidazol-4-yl)thieno[3,2-b]pyridine (16.6 g, yield: 39%).
¹H NMR (400 MHz, DMSO-d₆) δ 8.53 (d, J = 5.6 Hz, 1H), 8.46 (dd, J = 10.4 and 2.8 Hz, 1H), 8.21-8.18 (m, 1H), 7.88 (d, J = 1.2 Hz, 1H), 7.73 (s, 1H), 7.72 (d, J = 12 Hz, 1H), 7.68 (t, J = 8.4 Hz, 1H), 6.87 (d, J = 5.6 Hz, 1H), 3.73 (s, 3H)
MS (ESI) m/z 370.91 (M + H)⁺

### Step 4: Preparation of 3-fluoro-4-((2-(1-methyl-1H-imidazol-4-yl)thieno[3,2-b]pyridin-7-yl)oxy)aniline

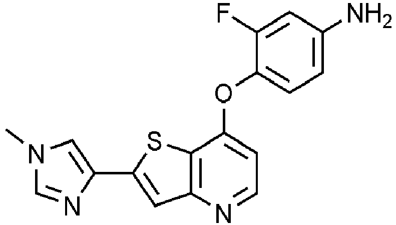

7-(2-Fluoro-4-nitrophenoxy)-2-(1-methyl-1H-imidazol-4-yl)thieno[3,2-b]pyridine (16.2 g, 43.7 mmol), iron (7 g, 131.2 mmol), and ammonium chloride (23.4 g, 437.4 mmol) were added to a mixed liquid of 400 mL of ethanol and 200 mL of water. The temperature of the mixture was elevated to 100°C, and the mixtrue was stirred for 1 hour. The resultant was cooled to room temperature, filtered through a celite pad, and concentrated under reduced pressure. Water was added thereto and stirring was performed. Then, the resultant was filtered to obtain 8.3 g (56%) of 3-fluoro-4-((2-(1-methyl-1H-imidazol-4-yl)thieno[3,2-b]pyridin-7-yl)oxy)aniline.
¹H NMR (400 MHz, DMSO-d₆) δ 8.40 (d, J = 5.2 Hz, 1H), 7.84 (S, 1H), 7.71 (s, 1H), 7.64 (s, 1H), 7.10 (t, J = 88 Hz, 1H), 6.55-6.43 (m, 3H), 5.52 (s, 2H), 3.72 (s, 3H)
MS (ESI) m/z 34111 (M + H)⁺

### Step 5: Preparation of 4-ethoxy-N-(3-fluoro-4-(2-(1-methyl-1H-imidazol-4-yl)thieno[3,2-b]pyridin-7-yloxy)phenyl)-1-(4-fluorophenyl)-2-oxo-1,2-dihydropyridine-3-carboxamide

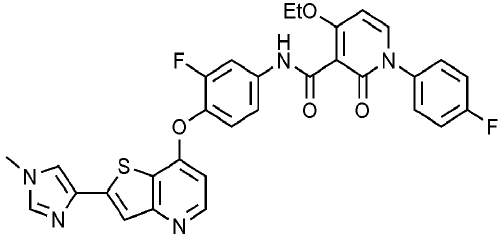

Thionyl chloride (116 g, 97.5 mmol) was added to a mixture of 100 mL of dichloromethane and 4-ethoxy-1 -(4-fluorophenyl)-2-oxo-1,2-dihydropyridine-3-carboxylic acid (10 g, 36.6 mmol). The resultant was stirred at room temperature for 2 hours, and then concentrated under reduced pressure to synthesize 4-ethoxy-1-(4-fluorophenyl)-2-oxo-1,2-dihydropyridine-3-carbonyl chloride. 3-Fluoro-4-((2-(l-methyl-1H-imidazol-4-yl)thieno[3,2-b]pyridin-7-yl)oxy)aniline (8.3 g, 24.4 mmol) was dissolved in 100 mL of dichloromethane, and then triethylamine (4.93 g, 48.8 mmol) was added thereto. Stirring was performed at room temperature for 2 hours. To the reaction mixture was added 4-ethoxy-1-(4-fluorophenyl)-2-oxo-1,2-dihydropyridine-3-carbonyl chloride, which had been obtained by concentratration under reduced pressure, and stirring was performed for 2 hours. The resulting solid was filtered and washed with water, to obtain the off-white solid, 4-ethoxy-N-(3-fluoro-4-(2-(1-methyl-1H-imidazol-4-yl)thieno[3,2-b]pyridin-7-yloxy)phenyl)-1-(4-fluorophenyl)-2-oxo-1,2-dihydropyridine-3-carboxamide (10.4 g, yield: 71%).
¹H NMR (400 MHz, DMSO-d₆) δ 10.61 (s, 1H), 8.42 (d, J = 5.2 Hz, 1H), 7.92 (dd, J = 2.0 and 13.6 Hz, 1H), 7.86 (d, J = 7.6 Hz, 1H), 7.86 (s, 1H), 7.72 (s, 1H), 7.68 (s, 1H), 7.50-7.43 (m, 4H), 7.37 (t, J= 8.8 Hz, 1H), 6.59 (d, J = 5.2 Hz, 1H), 6.52 (d, J = 7.6 Hz, 1H), 4.26 (q, J = 7.2 Hz, 2H), 3.73 (s, 1H), 1.31 (t, J = 7.2 Hz, 3H)
MS (ESI) m/z 600.16 (M + H)⁺

### Example 2: Preparation of 4-ethoxy-N-(3-fluoro-4-(2-(1-methyl-1H-imidazol-4-yl)thieno[3,2-b]pyridine-7-yloxy)phenyl)-1-methyl-2-oxo-1,2-dihydropyridine-3-carboxamide

The following compound was prepared in the same manner as in Example 1. 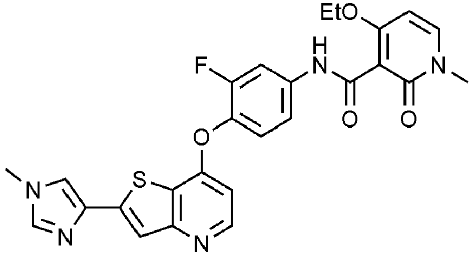 ¹H NMR (400 MHz, DMSO-d₆) δ 10.58 (s, 1H), 8.43 (d, J = 5.6 Hz, 1H), 7.94 (d, J = 13.2 Hz, 1H), 7.87 (t, J = 4.0 Hz, 2H), 7.73 (s, 1H), 7.69 (s, 1H), 7.46 (m, 2H), 6.60 (d, J = 5.2 Hz, 1H), 6.39 (d, J =8.0 Hz, 1H), 4.19 (q, J = 7.2 Hz, 2H), 3.74 (s, 3H), 3.44 (s, 3H), 1.28 (t, J = 7.2 Hz, 3H)
MS (ESI) m/z 520.15 (M + H)⁺

### Example 3: Preparation of 4-ethoxy-N-(3-fluoro-4-(2-(1-methyl-1H-imidazol-2-yl)thieno[3,2-b]pyridine-7-yloxy)phenyl)-l-(4-fluorophenyl)-2-oxo-1,2-dihydropyridine-3-carboxamide

The following compound was prepared in the same manner as in Example 1. 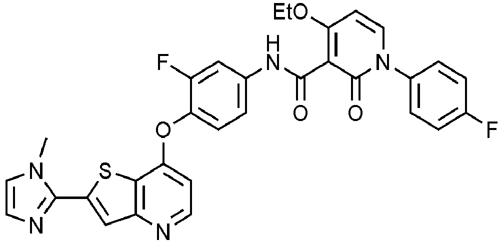 ¹H NMR (400 MHz, CDCl₃) δ 11.65 (s, 1H), 8.47 (d, J = 5.6 Hz, 1H), 7.97 (dd, J = 12.8 Hz, 2.4 Hz, 1H), 7.72-7.68 (m, 3H), 7.54-7.50 (m, 3H), 7.38-7.35 (m, 2H), 7.34-7.31 (m, 1H), 7.24-7.12 (m, 3H), 7.03 (d, J = 0.8 Hz, 1H), 6.53 (d, J = 5.6 Hz, 1H), 6.37 (d, J = 8 Hz, 1H), 4.37 (q, J = 7.2 Hz, 2H), 3.96 (s, 3H), 1.63 (t, J = 7.2 Hz, 3H)
MS (ESI) m/z 599.99 (M + H)⁺

### Example 4: Preparation of 4-ethoxy-N-(3-fluoro-4-(2-(1-methyl-1H-pyrazol-4-yl)thieno[3,2-b]pyridine-7-yloxy)phenyl)-1-(4-fluorophenyl)-2-oxo-1,2-dihydropyridine-3-carboxamide

The following compound was prepared in the same manner as in Example 1. 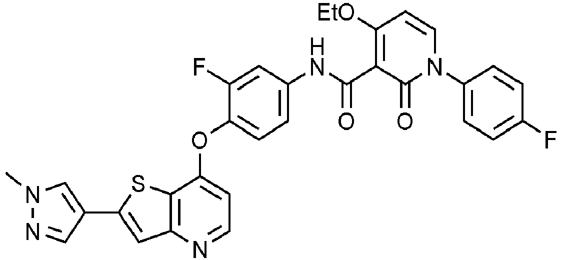 ¹H NMR (400 MHz, DMSO-d₆) δ 10.60 (s, 1H), 8.44 (d, 1H, J = 5.6 Hz, 1H), 8.31 (s, 1H), 7.80 (s, 1H), 7.93 (dd, J = 2.8 Hz, J = 12.8 Hz, 1H), 7.86 (d, J = 8.0 Hz, 1H), 7.69 (s, 1H), 7.47 (m, 5H), 7.37 (t, J = 8.8 Hz, 1H), 6.60 (d, J = 5.6 Hz, 1H), 6.52 (d, J = 8.0 Hz, 1H), 4.27 (q, J = 7.2 Hz, 2H), 3.90 (s, 3H), 1.31(t, J = 7.2 Hz, 3H)
MS (ESI) m/z 600.10 (M + H)⁺

### Example 5: Preparation of 4-ethoxy-N-(3-fluoro-4-(2-(pyridin-2-yl)thieno[3,2-b]pyridin-7-yloxy)phenyl)-1-(4-fluorophenyl)-2-oxo-1,2-dihydropyridine-3-carboxamide

The following compound was prepared in the same manner as in Example 1. 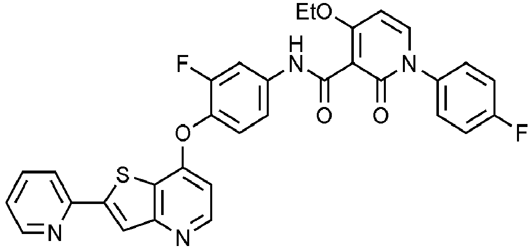 ¹H NMR (400 MHz, MeOH-d₄) δ 8.61 (dd, J = 4.8 Hz, 0.4 Hz, 1H), 8.47 (d, J = 5.6 Hz, 1H), 8.1 (s, 1H), 8.08 (s, 1H), 7.95-7.91 (m, 2H), 7.77 (d, J = 8 Hz, 1H), 7.46-7.37 (m, 5H), 7.27 (t, J = 8.4 Hz, 2H), 6.65 (dd, J = 0.8, 5.6 Hz, 1H), 6.58 (d, J = 7.6 Hz, 1H), 4.32 (q, J = 6.8 Hz, 2H), 1.46 (t, J = 7.2 Hz, 3H)
MS (ESI) m/z 597.16 (M + H)⁺

### Example 6: Preparation of 4-ethoxy-N-(3-fluoro-4-(2-(pyridin-3-yl)thieno[3,2-b]pyridin-7-yloxy)phenyl)-1-(4-fluorophenyl)-2-oxo-1,2-dihydropyridine-3-carboxamide

The following compound was prepared in the same manner as in Example 1. 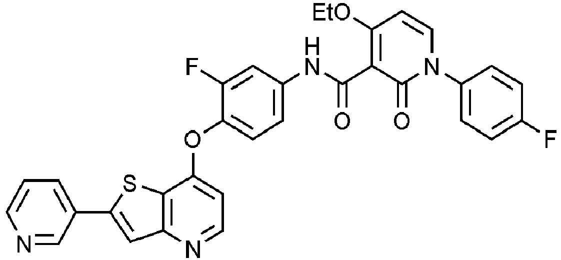 ¹H NMR (400 MHz, CDCl₃) δ 11.68 (s, 1H), 9.02 (d, J = 1.6 Hz, 1H), 8.63 (dd, J = 4.8 Hz, 1.2 Hz, 1H), 8.49 (d, J = 5.6 Hz, 1H), 8.02 (dt, J = 8, 1.6 Hz, 1H), 7.96 (dd, J = 12.4 Hz, 2.4 Hz, 1H), 7.80 (s, 1H), 7.51 (d, J = 7.6 Hz, 1H), 7.41-7.31 (m, 4H), 7.25-7.15 (m, 3H), 6.53 (dd, J = 5.2 Hz, 0.8 Hz 1H), 6.36 (d, J = 8 Hz, 1H), 4.36 (q, J = 6.8 Hz, 2H), 1.58 (t, J = 7.2 Hz, 3H)
MS (ESI) m/z 597.13 (M + H)⁺

### Example 7: Preparation of 4-ethoxy-N-(3-fluoro-4-(2-(thiazol-2-yl)thieno[3,2-b]pyridin-7-yloxy)phenyl)-1-(4-fluorophenyl)-2-oxo-1,2-dihydropyridine-3-carboxamide

The following compound was prepared in the same manner as in Example 1. 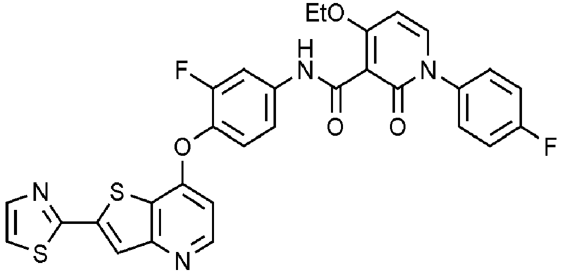 ¹H NMR (400 MHz, CDCl₃) δ 11.65 (s, 1H), 8.49 (d, 1H, J = 5.6 Hz, 1H), 7.96 (dd, J = 2.8 Hz, J = 12.8 Hz, 1H), 7.93 (s, 1H), 7.88 (d, J = 3.6 Hz, 1H), 7.51 (d, J = 7.6 Hz, 1H), 7.42 (d, J = 3.2 Hz, 1H), 7.22-7.38 (m, 6H), 7.18 (t, J = 8.8 Hz, 1H), 6.54 (dd, J = 1.2 Hz, J = 5.6 Hz, 1H), 6.37 (d, J = 8.0Hz, 1H), 4.37 (q, J = 7.2 Hz, 2H), 1.59 (t, J = 7.2 Hz, 3H)
MS (ESI) m/z 603.15 (M + H)⁺

### Example 8: Preparation of 4-ethoxy-N-(4-(2-(1-ethyl-1H-imidazol-4-yl)thieno[3,2-blpyridin-7-yloxy)-3-fluorophenyl)-1-(4-fluorobenzyl)-2-oxo-1,2-dihydropyridine-3-carboxamide

The following compound was prepared in the same manner as in Example 1. 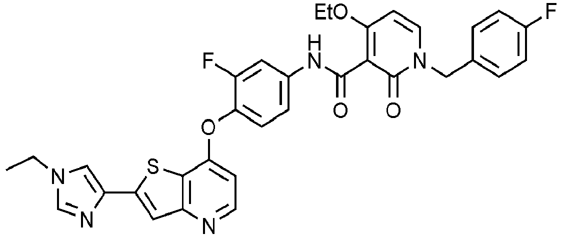 ¹H NMR (400 MHz, CDCl₃) δ 11.76 (s, 1H), 8.43 (d, J = 5.6 Hz, 1H), 7.95 (dd, J = 12.8 and 2.0 Hz, 1H), 7.66 (s, 1H), 7.55 (s, 1H), 7.44 (d, J = 8.0 Hz, 1H), 7.28-7.37 (m, 4H), 7.19 (t, J = 8.8 Hz, 1H), 7.00 (t, J = 8.8 Hz, 1H), 6.47 (d, J = 5.6 Hz, 1H), 6.24 (d, J = 8.0 Hz, 1H), 5.15 (s, 1H), 4.27 (q, J = 7.2 Hz, 2H), 4.05 (q, J = 7.2 Hz, 2H), 1.53 (t, J = 7.2 Hz, 3H)
MS (ESI) m/z 628.24 (M + H)⁺

### Example 9: Preparation of 4-methoxy-N-(3-fluoro-4-(2-(1-(methoxymethyl)-1H-imidazol-4-yl)thieno[3,2-b]pyridin-7-yloxy)phenyl)-1-phenyl-2-oxo-1,2-dihydropyridine-3-carboxamide

The following compound was prepared in the same manner as in Example 1. 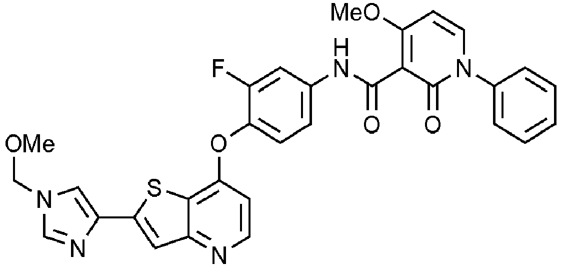 ¹H NMR (400 MHz, DMSO-d₆) δ 10.66 (s, 1H), 8.44 (d, J = 5.6 Hz, 1H), 8.04 (s, 1H), 7.95-7.89 (m, 3H), 7.77 (s, 1H), 7.56-7.40 (m, 7H), 6.61 (d, J = 56 Hz, 1H), 6.53 (d, J = 7.6 Hz, 1H), 5.37 (s, 2H), 3.94 (s, 1H), 3.27 (s, 3H)
MS (ESI) m/z 568.12 (M + H)⁺

### Example 10: Preparation of 4-ethoxy-N-(3-fluoro-4-(2-(piperazin-1-ylmethyl)thieno[3,2-b]pyridin-7-yloxy)phenyl)-1-(4-fluorophenyl)-2-oxo-1,2-dihydropyridine-3-carboxamide hydrochloride

### Step 1: Preparation of 7-chlorothieno[3,2-b]pyridine-2-carbaldehyde

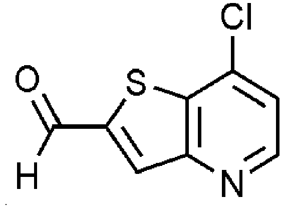

7-Chlorothieno[3,2-b]pyridine (1 g, 5.89 mmol) was dissolved in 20 mL of tetrahydrofuran, and then cooled to -70°C. n-Butyllithium (25M hexane solution) (3.06 mL, 7.66 mmol) was added thereto, and then stirring was performed for 1 hour. Anhydrous N,N-dimethylformamide (0.68 mL, 8.84 mmol) was added thereto at the same temperature and then stirring was performed for 1 hour. The resultant was cooled to room temperature, and then 40 mL of water was added thereto. The resultant was extracted with 40 mL of ethyl acetate, and then dried over anhydrous magnesium sulfate. The resultant was filtered and concentrated under reduced pressure, to obtain 7-chlorothieno[3,2-b]pyridine-2-carbaldehyde (1.17 g, yield: 98%).
¹H NMR (400 MHz, DMSO-d₆) δ 10.24(s, 1H), 8.82(d, J = 4.8 Hz, 1H), 8.64(s, 1H), 7.81(d, J = 4.8 Hz, 1H)
MS (ESI) m/z 197.99 (M + H)⁺

### Step 2: Preparation of 7-(2-fluoro-4-nitrophenoxy)thieno[3,2-b]pyridine-2-carbaldehyde

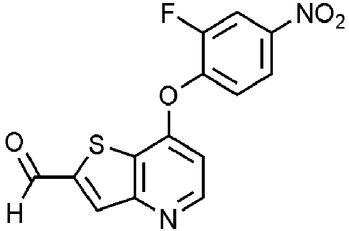

To 20 mL of diphenyl ether was added the compound (1.17 g, 5.91 mmol) obtained in Step 1, 2-fluoro-4-nitrophenol (1.39 g, 8.87 mmol), and potassium carbonate (2.45 g, 17.73 mmol). The resultant was heated to reflux at 170°C for 29 hours. After completion of the reaction, the resultant was cooled to room temperature. 40 mL of water was added thereto and extraction with 40 mL of ethyl acetate was performed. The resultant was dried over anhydrous magnesium sulfate, filtered, concentrated under reduced pressure, and then, separated and purified by silica gel column chromatography, to obtain 7-(2-fluoro-4-nitrophenoxy)thieno[3,2-b]pyridine-2-carbaldehyde (1.05 g, yield: 54%).
¹H NMR (400 MHz, DMSO-d₆) δ 10.26 (s, 1H), 8.75 (d, J = 5.6 Hz, 1H), 8.63 (s, 1H), 8.49 (dd, J = 10.4 Hz, 2.8 Hz, 1H), 8.24 (m, 1H), 7.79 (t, J = 8 Hz, 1H), 7.11 (dd, J = 52 Hz, 0.4 Hz, 1H)
MS (ESI) m/z 351.08 (M + H)⁺

### Step 3: Preparation of tert-butyl 4-((7-(2-fluoro-4-nitrophenoxy)thieno[3,2-b]pyridin-2-yl)methyl)piperazin-1-carboxylate

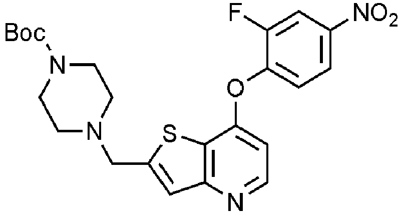

The compound (100 mg, 0.31 mmol) obtained in Step 2 was dissolved in 1 mL of anhydrous dichloromethane, and tert-butyl piperazine-1-carboxylate (64.3 mg, 0.34 mmol) was added thereto. The resultant was stirred at room temperature for 1 hour. Sodium triacetoxy borohydride (95.8 mg, 0.45 mmol) was added thereto and stirring was performed at room temperature for 2 hours. 2 mL of saturated aqueous sodium bicarbonate solution was added thereto, and extraction with 1 mL of dichloromethane was performed. The resultant was dried over anhydrous magnesium sulfate, filtered, and concentrated under reduced pressure, to obtain tert-butyl 4-((7-(2-fluoro-4-nitrophenoxy)thieno[3,2-b]pyridin-2-yl)methyl)piperazine-1-carboxylate (148.5 mg, yield: 97%).
¹H NMR (400 MHz, DMSO-d₆) δ 8.54 (d, J = 5.6 Hz, 1H), 8.46 (dd, J = 10.4 Hz, 24 Hz, 1H), 8.20 (m, 1H) 7.68 (t, J = 5.2 Hz, 1H), 7.52 (s, 1H), 6.88 (d, J = 5.2 Hz, 1H), 3.33 (t, J = 4.8 Hz, 4H), 2.44 (t, J= 4.8 Hz, 4H), 1.39 (s, 9H)
MS (ESI) m/z 489.27 (M + H)⁺

### Step 4: Preparation of tert-butyl 4-((7-(4-amino-2-fluorophenoxy)thieno[3,2-b]pyridin-2-yl)methyl)piperazine-1-carboxylate

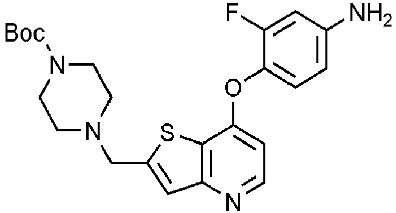

The compound (140 mg, 0.28 mmol) obtained in Step 3 was dissolved in 10 mL of ethanol:water (2:1), and then iron (48 mg, 0.86 mmol) and ammonium chloride (152 mg, 2.85 mmol) were added thereto. Stirring was performed at 110°C for 1 hour. After completion of the reaction, the resultant was cooled to room temperature. Then, ethyl acetate was added thereto and stirring was performed for 10 minutes. The reaction mixture was passed through celite while performing washing with ethyl acetate. To the solution which had passed through celite was added distilled water, and extraction with ethyl acetate was performed. The organic layer was dried over anhydrous magnesium sulfate, filtered, and then removal of solvent was performed under reduced pressure, to obtain tert-butyl 4-((7-(4-amino-2-fluorophenoxy)thieno[3,2-b]pyridine-2-yl)methyl)piperazin-1-carboxylate (120 mg, yield: 93%).
¹H NMR (400 MHz, DMSO-d₆) δ 8.42 (d, J = 5.2 Hz, 1H), 7.43 (s, 1H), 7.08 (t, J = 9.2 Hz, 1H), 6.54-6.49 (m, 2H), 6.44 (dd, J = 8.8 Hz, 2.4 Hz, 1H), 5.51 (bs, 2H), 3.86 (s, 1H), 3.33 (m, 4H), 2.43 (m, 4H), 1.39 (s, 9H)
MS (ESI) m/z 459.31 (M + H)⁺

### Step 5: Preparation of tert-butyl 4-((7-(4-(4-ethoxy-1-(4-fluorophenyl)-2-oxo-1,2-dihydropyridine-3-carboxamido)-2-fluorophenoxy)thieno[3,2-b]pyridin-2-yl)methyl)piperazine-1-carboxylate

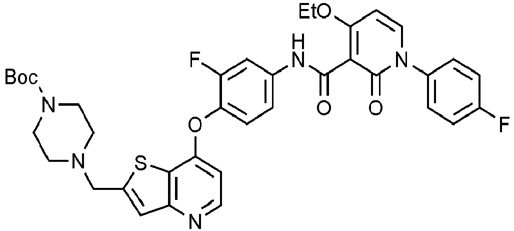

4-Ethoxy-1-(4-fluorophenyl)-2-oxo-1,2-dihydropyridine-3-carboxylic acid (90.6 mg, 0.32 mmol) was dissolved in 4 mL of dichloromethane, and then thionyl chloride (47.5 uL, 0.65 mmol) was added slowly thereto. Stirring was performed at room temperature for 1 hour. Removal of solvent was performed under reduced pressure, and the resultant was dried under vacuum to synthesize 4-ethoxy-1-(4-fluorophenyl)-2-oxo-1,2-dihydropyridine-3-carbonyl chloride.

The compound (100 mg, 0.21 mmol) obtained in Step 4 was dissolved in 2 mL of dichloromethane. Triethylamine (45.6 uL, 0.32 mmol) was added thereto. Stirring was performed at room temperature for 30 minutes. The acid chloride obtained by removal of solvent under reduced pressure was dissolved again in 2 mL of dichloromethane and added to the reaction mixture. Stirring was performed at room temperature for 1 hour. Water and saturated aqueous sodium bicarbonate solution were added thereto, and extraction with dichloromethane was performed. The organic layer was dried over anhydrous magnesium sulfate, filtered and concentrated under reduced pressure, and then separated and purified by silica gel column chromatography, to obtain tert-butyl 4-((7-(4-(4-ethoxy-1-(4-fluorophenyl)-2-oxo-1,2-dihydropyridine-3-carboxamido)-2-fluorophenoxy)thieno[3,2-b]pyridin-2-yl)methyl)piperazine-1-carboxylate (150 mg, yield: 99%).
¹H NMR (400 MHz, DMSO-d₆) δ 10.59 (s, 1H), 8.44 (d, J = 5.2 Hz, 1H), 7.92 (dd, J = 12.8 Hz, 2 Hz, 1H), 7.86 (d, J = 8 Hz, 1H), 7.49-7.41 (m, 5H), 7.39-7.34 (m, 2H), 6.60 (d, J = 5.2 Hz, 1H), 6.51 (d, J =7.6 Hz, 1H), 4.26 (q, J = 6.8 Hz, 2H), 3.87 (s, 1H), 3.35 (bs, 4H), 2.45 (bs, 1H), 1.39 (s, 9H), 1.30 (t, J = 1.4 Hz, 3H)
MS (ESI) m/z 718.44 (M + H)⁺

### Step 6: Preparation of 4-ethoxy-N-(3-fluoro-4-(2-(piperazin-1-ylmethyl)thieno[3,2-b]pyridin-7-yloxy)phenyl)-1-(4-fluorophenyl)-2-oxo-1,2-dihydropyridine-3-carboxamide hydrochloride

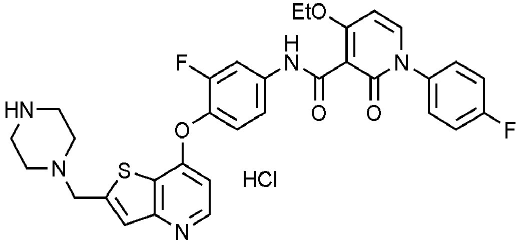

The compound (126 mg, 0.17 mmol) obtained in Step 5 was dissolved in dichloromethane, and then 4N aqueous hydrogen chloride solution in dioxane was added thereto. Stirring was performed at room temperature for 12 hours. After concentration under reduced pressure, diethyl ether was added thereto. The resulting solid was filtered to obtain 4-ethoxy-N-(3-fluoro-4-((2-(piperazin-1-ylmethyl)thieno[3,2-b]pyridin-7-yl)oxy)phenyl)-1-(4-fluorophenyl)-2-oxo-1,2-dihydropyridine-3-carboxamide hydrochloride (123 mg, yield: 95%).
¹H NMR (400 MHz, DMSO-d₆) δ 10.66 (s, 1H), 8.63 (d, J = 6 Hz, 1H), 8.05 (m, 2H), 7.87 (d, J = 7.6 Hz, 1H), 7.55-7.44 (m, 5H), 7.40-7.34 (m, 2H), 6.90 (d, J = 5.2 Hz, 1H), 6.53 (d, J = 8 Hz, 1H), 4.26 (q, J = 6.8 Hz, 2H), 3.23 (bs, 4H), 2.97 (bs, 4H), 1.30 (t, J = 7.2 Hz, 3H)
MS (ESI) m/z 652.02 (M + HCl)⁻

### Example 11: Preparation of 4-ethoxy-N-(3-fluoro-4-(2-((4-methylpiperazin-1-yl)methyl)thieno[3,2-b]pyridine-7-yloxy)phenyl)-1-(4-fluorophenyl)-2-oxo-1,2-dihydropyridine-3-carboxamide

The following compound was prepared in the same manner as in Example 10. 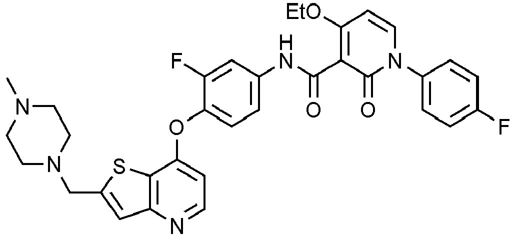 ¹H NMR (400 MHz, CDCl₃) δ 11.65 (s, 1H), 8.45 (d, J = 5.6 Hz, 1H), 7.96 (dd, J = 12.4 and 2.0 Hz, 1H), 8.45 (d, J = 7.6 Hz, 1H), 7.40-7.24 (m, 6H), 7.15 (t, J = 8.8 Hz, 1H), 6.49 (d, J = 4.8 Hz, 1H), 6.39 (d, J = 7.6 Hz, 1H), 4.39 (q, J = 7.2 Hz, 2H), 3.90 (s, 1H), 2.87-2.52 (m, 8H), 1.61 (t, J = 72 Hz, 3H), 1.27 (s, 3H)
MS (ESI) m/z 632.25 (M + H)⁺

### Example 12: Preparation of 4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide hydrochloride

### Step 1: Synthesis of N-{(6-bromopyridin-3-yl)methyl}-2-methoxyethanamine

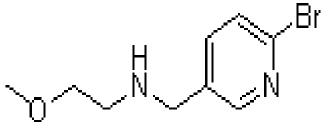

6-Bromopyridine-3-carboxaldehyde (5 g, 26.88 mmol) was dissolved in 1,2-dichloroethane, and then 2-methoxyethylamine (3.5 mL, 40.32 mmol) and acetic acid (1.6 mL, 28.76 mmol) were sequentially added thereto. Stirring was performed for 20 minutes. Then, triacetoxy sodium borohydride (8.5 g, 40.32 mmol) was added thereto and stirring was performed at room temperature for 2 hours. The reaction was terminated with 1N aqueous hydrochloric acid solution, the resultant was adjusted to pH 9 with 2N aqueous sodium hydroxide solution. Then, extraction with dichloromethane was performed. The separated organic layer was dried over anhydrous sodium sulfate, and filtered and concentrated under reduced pressure. The resulting residue was purified by column chromatography to obtain the title compound (4.05 g, yield: 70%, light red oil).
¹H NMR (500 MHz, DMSO-d₆) δ 8.31 (d, J = 5.0 Hz, 1H), 7.70 (dd, J = 10.0 and 5.0 Hz, 1H), 7.58 (d, J = 10.0 Hz, 1H), 3.69 (s, 2H), 3.37 (t, J = 5.0 Hz, 1H), 3.22 (s, 3H), 2.60 (d, J = 5.0 Hz, 2H)

### Step 2: Synthesis of t-butyl [(6-bromopyridin-3-yl)methyl1(2-methoxyethyl)carbamate

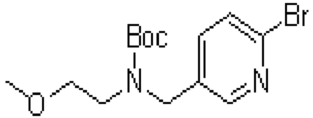

The compound (4.05 g, 16.52 mmol) prepared in Step 1 was dissolved in tetrahydrofuran, and then di-t-butyl dicarbonate (3.9 mL, 17.02 mmol) was added thereto. Stirring was performed at room temperature for 30 minutes. Extraction with ethyl acetate was performed. The separated organic layer was dried over anhydrous sodium sulfate, and filtered and concentrated under reduced pressure. The resulting residue was purified by column chromatography to obtain the title compound (5.52 g, yield: 97%, colorless oil).
¹H NMR (500 MHz, DMSO-d₆) δ 8.27 (s, 1H), 7.64-7.59 (m, 2H), 4.40 (s, 2H), 3.40 (m, 4H), 3.21 (s, 3H), 1.42-1.31 (m, 9H)

### Step 3: Synthesis of t-butyl {[6-(7-chlorothieno[3,2-b]pyridin-2-yl)pyridin-3-yl]methyl}(2-methoxyethyl)carbamate

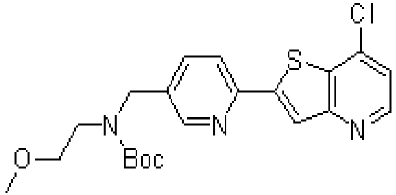

7-Chlorothieno[3,2-b]pyridine (5.4 g, 31.86 mmol) was dissolved in tetrahydrofuran, and then 2.5 M n-butyllithium hexane solution (2.7 mL, 31.86 mmol) was added slowly thereto at -78°C. Stirring was performed for 30 minutes. 1 M zinc chloride diethyl ether solution (31.9 mL, 31.86 mmol) was added slowly thereto, and after 10 minutes, the temperature of the resultant was gradually elevated to room temperature. Then, stirring was performed for 1 hour. Thereafter, tetrakistriphenylphosphinepalladium (920 mg, 0.79 mmol) and the compound (5.5 g, 15.93 mmol) prepared in Step 2 were added thereto, and the resultant was refluxed for 2 hours. The resultant was cooled to room temperature and concentrated under reduced pressure. The resulting residue was filtered through acetonitrile and dried to obtain the title compound (5.2 g, yield: 75%, off-white solid).
¹H NMR (500 MHz, DMSO-d₆) δ 8.66 (d, J = 5.0 Hz, 1H), 8.54 (s, 1H), 8.42 (s, 1H), 8.29 (d, J = 5.0 Hz, 1H), 7.82 (dd, J = 10.0 and 5.0 Hz, 1H), 7.59 (d, J = 5.0 Hz, 1H), 4.50 (s, 2H), 3.45-3.36 (m, 4H), 3.23 (s, 3H), 1.45-1.34 (m, 9H)

### Step 4: Synthesis of t-butyl {[6-[7-(2-fluoro-4-nitrophenoxy)thieno]3,2-b]pyridin-2-yl]pyridin-3-yl]methyl}(2-methoxyethyl)carbamate

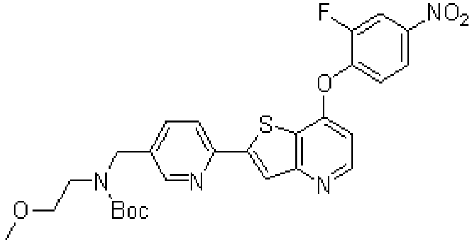

The compound (2.0 g, 4.61 mmol) prepared in Step 3 was dissolved in diphenyl ether, and anhydrous potassium carbonate (765 mg, 5.53 mmol) and 2-fluoro-4-nitrophenol (1.4 g, 9.22 mmol) were added sequentially thereto. Stirring was performed at 160°C for 5 hours. The resultant was cooled to room temperature and extracted with ethyl acetate. The separated organic layer was dried over anhydrous sodium sulfate, and filtered and concentrated under reduced pressure. The resulting residue was dissolved in tetrahydrofuran, and then di-t-butyl dicarbonate (1.06 mL, 4.61 mmol) was added thereto. Stirring was performed at room temperature for 30 minutes. The residue obtained by concentration under reduced pressure was purified by column chromatography to obtain the title compound (1.2 g, yield: 46%, off-white solid).
¹H NMR (500 MHz, DMSO-d₆) δ 8.62 (d, J = 5.0 Hz, 1H), 8.50-8.48 (m, 2H), 8.39 (s, 1H), 8.28 (d, J = 5.0 Hz, 1H), 8.21 (d, J = 10.0 and 5.0 Hz, 1H), 7.80 (d, J = 10.0 Hz, 1H), 7.72 (t, J = 10.0 Hz, 1H), 6.98 (d, J = 5.0 Hz, 1H), 4.48 (s, 2H), 3.43-3.35 (m, 4H), 3.23 (s, 3H), 1.44-1.33 (m, 9H)

### Step 5: Synthesis of t-butyl {[6-[7-(4-amino-2-fluorophenoxy)thieno[3,2-b]pyridin-2-yl]pyridin-3-yl]methyl}2-methoxyethyl)carbamate

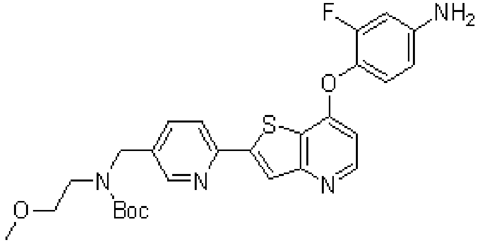

The compound (1.2 g, 2.16 mmol) prepared in Step 4 was dissolved in ethanol and water, and then iron (363 mg, 6.49 mmol) and ammonium chloride (1.16 g, 21.64 mmol) were added sequentially at room temperature. The temperature of the resultant was elevated to 100°C and stirring was performed for 3 hours. After completion of the reaction, the resultant was filtered under a warm state using a celite pad and concentrated under reduced pressure. The resulting residue was extracted with dichloromethane. The separated organic layer was dried over anhydrous sodium sulfate, concentrated under reduced pressure, and then filtered with diethyl ether to obtain the title compound (833 mg, yield: 73%, off-white solid).
¹H NMR (500 MHz, DMSO-d₆) δ 8.51-8.49 (m, 2H), 8.30 (s, 1H), 8.24 (d, J = 5.0 Hz, 1H), 7.78 (dd, J = 10.0 and 5.0 Hz, 1H), 7.13 (t, J = 10.0 Hz, 1H), 6.60 (d, J = 5.0 Hz, 1H), 6.53 (dd, J = 15.0 and 5.0 Hz, 1H), 6.45 (dd, J = 10.0 and 5.0 Hz, 1H), 5.55 (s, 2H), 4.48 (s, 2H), 3.43-3.33 (m, 4H), 3.23 (s, 3H), 1.44-1.34 (m, 9H)

### Step 6: Synthesis of t-butyl[(6-{7-[4-(4-ethoxy-2-oxo-1-phenyl-1,2-dihydropyridine-3-carboxamido)-2-fluorophenoxy]thieno[3,2-b]pyridin-2-yl}pyridin-3-yl)methyl](2-methoxyethyl)carbamate

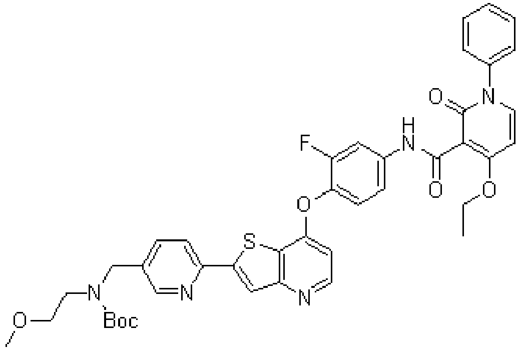

4-Ethoxy-2-oxo-1-phenyl-1,2-dihydropyridine-3-carboxylic acid (0.57 g, 2.2 mmol) of Preparation Example 2, 1-(3-dimethylaminopropyl)-3-ethylcarbodiimide hydrochloride (0.42 g, 2.2 mmol), and hydroxybenzotriazole (0.3 g, 2.2 mmol) were dissolved in dichloromethane, and then triethylamine (0.22 g, 2.2 mmol) and the compound (0.58 g, 1.1 mmol) prepared in Step 5 were added sequentially. Stirring was performed at room temperature for 24 hours or longer. The reaction was terminated with saturated aqueous sodium bicarbonate solution and extraction with dichloromethane was performed. Then, the separated organic layer was dried over anhydrous magnesium sulfate and concentrated under reduced pressure. The resulting residue was purified by column chromatography to obtain the title compound (0.69 g, yield: 82%, off-white solid).
¹H NMR (500 MHz, DMSO-d₆) δ 10.64 (brs, 1H), 8.52-8.51 (m, 2H), 8.33 (s, 1H), 7.95 (d, J = 10.0 Hz, 1H), 7.87 (d, J = 5.0 Hz, 1H), 7.79 (d, J = 10.0 Hz, 1H), 7.56-7.40 (m, 8H), 6.71 (d, J = 5.0 Hz, 1H), 6.52 (d, J = 10.0 Hz, 1H), 4.48 (s, 2H), 4.27 (qt, J = 7.5 Hz, 2H), 3.43-3.36 (m, 4H), 3.23 (s, 3H), 1.14-1.30 (m, 12H)

### Step 7: Synthesis of 4-ethoxy-N-(3-fluoro-4-{[2-(5-{[(2-methoxyethyl)amino]methyl}pyridin-2-yl)thieno[3,2-b]pyridin-7-yl]oxy}phenyl)- 2-oxo-1 -phenyl-1,2-dihydropyridine-3-carboxamide hydrochloride

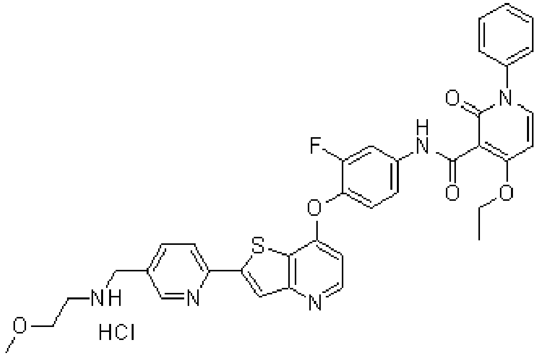

To the compound (0.69 g, 0.9 mmol) prepared in Step 6 was added 10 mL of 4M hydrochloric acid solution in 1,4-dioxane, and stirring was performed at room temperature for 1 hour or longer. The reaction mixture was concentrated under reduced pressure, dissolved in a small amount of ethanol, and then diethyl ether was added thereto. The resulting solid was filtered and dried to obtain the title compound (0.61 g, yield: 96%, off-white solid).
¹H NMR (500 MHz, DMSO-d₆) δ 10.70 (brs, 1H), 9.50 (brs, 2H), 8.80 (s, 1H), 8.69 (d, J = 5.0 Hz, 1H), 8.47 (s, 1H), 8.44 (d, J = 5.0 Hz, 1H), 8.22 (dd, J = 10.0 and 5.0 Hz, 1H), 7.99 (d, J = 10.0 Hz, 1H), 7.88 (d, J = 5.0 Hz, 1H), 7.57-7.40 (m, 7H), 6.97 (d, J = 5.0 Hz, 1H), 6.53 (d, J = 5.0 Hz, 1H), 4.29-4.25 (m, 4H), 3.65 (t, J = 5.0 Hz, 2H), 3.31 (s, 3H), 3.16-3.12 (m, 2H), 1.31 (t, J = 5.0 Hz, 3H)

### Example 13: Preparation of 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3-carboxamide hydrochloride

Preparation was done according to the synthesis route of Example 12, except that 4-ethoxy-1-(4-fluorophenyl)-2-oxo-1,2-dihydropyridine-3-carboxylic acid of Preparation Example 1 was used as starting material of Step 6, to obtain the title compound (yield: 52.8%). 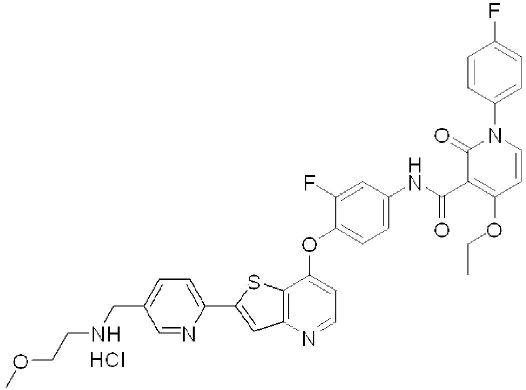 ¹H NMR (500 MHz, DMSO-d₆) δ 10.67 (br s, 1H), 9.32 (br s, 2H), 8.78 (s, 1H), 8.62 (d, J=5.0Hz, 1H), 8.46 (s, 1H), 8.41 (d, J = 10.0 Hz, 1H), 8.18 (d, J = 5.0 Hz, 1H), 7.99-7.96 (m, 1H), 7.90-7.88 (d, J = 10.0 Hz, 1H), 7.53-7.39 (m, 6H), 6.86 (d, J = 5.0 Hz, 1H), 6.55 (d, J = 5.0 Hz, 1H), 4.30-4.26 (m, 4H), 3.64 (m, 2H), 3.33 (s, 3H), 3.19-3.14 (m, 2H), 1.32 (t, J = 5.0 Hz, 3H)

### Example 14: Preparation of N-(3-chloro-4-{[2-(5-{[(2-methoxyethyl)amino]methyl}pyridin-2-yl)thieno[3,2-b]pyridine-7-yl]oxy}phenyl)-1-(4-fluorophenyl)-4-methoxy-2-oxo-1,2-dihydropyridine-3-carboxamide

### hydrochloride

Preparation was done according to the synthesis route of Example 12, except that 1-(4-fluorophenyl)-4-methoxy-2-oxo-1,2-dihydropyridine-3-carboxylic acid of Preparation Example 3 was used as a starting material of Step 6, and 2-chloro-4-nitrophenol was used in place of 2-fluoro-4-nitrophenol in Step 4, to obtain the title compound (yield: 88.6%). 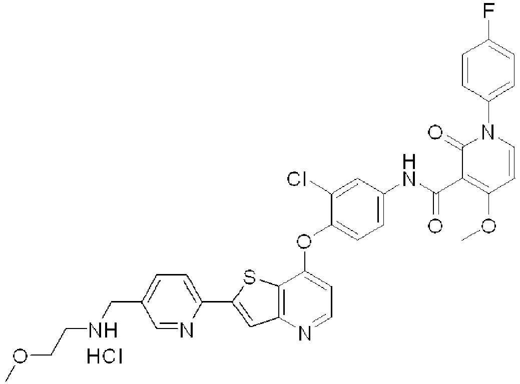 ¹H NMR (500 MHz, DMSO-d₆) δ 10.72 (brs, 1H), 9.44 (brs, 2H), 8.79 (s, 1H), 8.65 (d, J = 5.0 Hz, 1H), 8.45 (s, 1H), 8.42 (d, J = 10.0 Hz, 1H), 8.21-8.17 (m, 2H), 7.92 (d, J = 10.0 Hz, 1H), 7.70 (dd, J = 10.0 and 5.0 Hz, 1H), 7.54 (d, J = 10.0 Hz, 1H), 7.49-7.36 (m, 4H), 6.84 (d, J = 5.0 Hz, 1H), 6.56 (d, J = 5.0 Hz, 1H), 4.27 (t, J = 5.0 Hz, 2H), 3.94 (s, 3H), 3.65 (t, J = 5.0 Hz, 2H), 3.31 (s, 3H), 3.16-3.12 (m, 2H)

### Example 15: Preparation of 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(3-fluorophenyl)-2-oxo-1,2-dihydropyridine-3-carboxamide hydrochloride

Preparation was done according to the synthesis route of Example 12, except that 4-ethoxy-1-(3-fluorophenyl)-2-oxo-1,2-dihydropyridine-3-carboxylic acid of Preparation Example 7 was used as a starting material of Step 6, to obtain the title compound (yield: 73.5%). 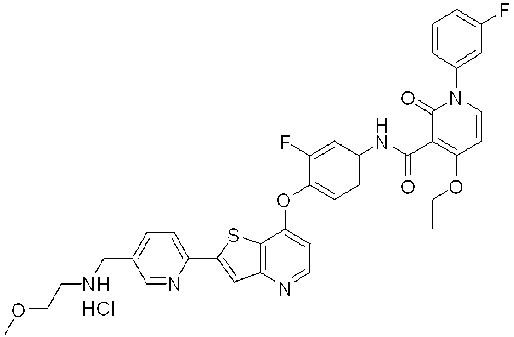 ¹H NMR (500 MHz, DMSO-d₆) δ 10.70(s, 1H), 9.47(brs, 2H), 8.79(s, 1H), 8.66(d, J = 5.0 Hz, 1H), 8.46(s, 1H), 8.43(d, J = 10.0 Hz, 1H), 8.21(dd, J = 10.0 Hz and 5.0 Hz, 1H), 7.98(d, J = 15.0 Hz, 1H), 7.91(d, J = 10.0 Hz, 1H), 7.61-7.53(m, 3H), 7.39-7.33(m, 2H), 7.28(d, J = 10.0 Hz, 1H), 6.93(d, J = 5.0 Hz, 1H), 6.55(d, J = 5.0 Hz, 1H), 4.30-4.26(m, 4H), 3.65(t, J = 5.0 Hz, 2H), 3.31(s, 3H), 3.16-3.12(m, 2H), 1.30(t, J = 5.0 Hz, 3H)

### Example 16: Preparation of N-(3-fluoro-4-{[2-(5-{[(2-methoxyethyl)amino]methyl}pyridin-2-yl)thieno[3,2-b]pyridine-7-yl]oxy}phenyl)-1-(4-fluorophenyl)-4-methyl-2-oxo-1,2-dihydropyridine-3-carboxamide hydrochloride

Preparation was done according to the synthesis route of Example 12, except that 4-methyl-2-oxo-1-(4-fluorophenyl)-1,2-dihydropyridine-3-carboxylic acid of Preparation Example 8 was used as a starting material of Step 6, to obtain the title compound (yield: 67.4%). 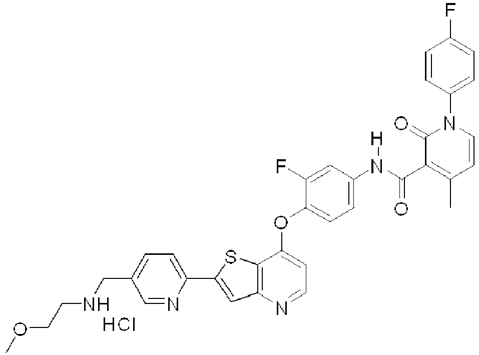 ¹H NMR (500 MHz, DMSO-d₆) δ 10.95 (s, 1H), 9.24 (brs, 2H), 8.75 (s, 1H), 8.58 (d, J = 5.0 Hz, 1H), 8.44 (s, 1H), 8.39 (d, J = 10.0 Hz, 1H), 8.14 (d, J = 10.0 Hz, 1H), 7.98 (d, J = 15.0 Hz, 1H), 7.75 (d, J = 10.0 Hz, 1H), 7.53-7.49 (m, 4H), 7.41-7.37 (m, 2H), 6.79 (d, J = 5.0 Hz, 1H), 6.38 (d, J = 5.0 Hz, 1H), 4.27 (t, J = 5.0 H, 2H), 3.63 (t, J = 5.0 Hz, 2H), 3.32 (s, 3H), 3.17-3.13 (m, 2H), 2.31 (s, 3H)

### Example 17: Preparation of 4-ethoxy-N-(3-fluoro-4-{[2-(5-{[(2-methoxyethyl)(methyl)amino]methyl}pyridin-2-yl)thieno[3,2-b]pyridin-7-yl]oxy}phenyl)-1-(4-fluorophenyl)-2-oxo-1,2-dihydropyridine-3-carboxamide hydrochloride

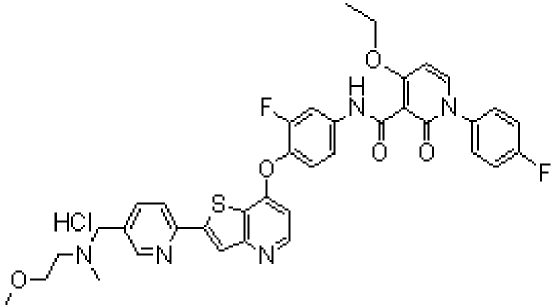

The compound of Example 13 was dissolved in 1 mL of methanol, and formaldehyde (0.02 mL, 0.28 mmol) was added dropwise thereto. Stirring was performed for 1 hour. Then, sodium cyanohydride (12 mg, 0.19 mmol) was added thereto, and stirring was performed for 12 hours. After completion of the reaction, the resultant was extracted with dichloromethane and then washed with saturated aqueous sodium chloride solution. The organic layer was dried over anhydrous sodium sulfate and concentrated under reduced pressure. The resulting residue was purified by column chromatography, and then 0.2 mL of 4 M hydrochloric acid solution in 1,4-dioxane was used to obtain the title compound (17.5 mg, yield: 50.5%, off-white solid).
¹H NMR (500 MHz, DMSO-d₆) δ 10.67 (s, 1H), 10.51 (brs, 1H), 8.80 (s, 1H), 8.60 (d, J = 5.0 Hz, 1H), 8.48 (s, 1H), 8.43 (d, J = 10.0 Hz, 1H), 8.21 (d, J = 5.0 Hz, 1H), 7.96 (d, J = 15.0 Hz, 1H), 7.88 (d, J = 10.0 Hz, 1H), 7.52-7.45 (m, 4H), 7.39-7.36 (m, 2H), 6.83 (d, J = 5.0 Hz, 1H), 6.53 (d, J = 10.0 Hz, 1H), 4.51-4.36 (m, 2H), 4.27 (q, J = 5.0 Hz, 2H), 3.73 (t, J = 5.0 Hz, 2H), 3.38-3.29 (m, 2H), 3.32 (s, 3H), 2.75 (d, J = 5.0 Hz, 3H)

### Example 18: Preparation of 4-ethoxy-N-[3-fluoro-4-({2-[5-(morpholinomethyl)pyridin-2-yl]thieno[3,2-b]pyridine-7-yl]oxy)phenyl]-1-(4-fluorophenyl)-2-oxo-1,2-dihydropyridine-3-carboxamide hydrochloride

### Step 1: Synthesis of N-[4-({2-[5-(1,3-dioxolan-2-yl)pyridin-2-yl]thieno[3,2-b]pyridin-7-yl}oxy)-3-fluorophenyl]-4-ethoxy-1-(4-fluorophenyl)-2-oxo-1,2-dihydropyridine-3-carboxamide

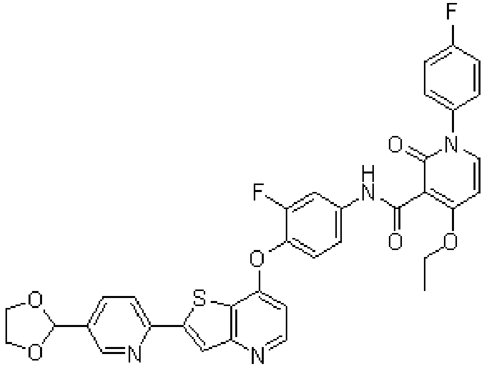

Using, as a starting material, 4-({2-[5-(1,3-dioxolan-2-yl)pyridin-2-yl]thieno[3,2-b]pyridin-7-yl}oxy)-3-fluoroaniline which can be easily prepared using the preparation method of International Patent Publication WO 2009/026717, reaction was allowed to proceed in the same manner as in Step 6 of Example 12, thereby obtaining the title compound (135 mg, yield: 64%, off-white solid).
¹H NMR (500 MHz, DMSO-d₆) δ 10.63 (s, 1H), 8.70 (s, 1H), 8.52 (d, J = 5.0 Hz, 1H), 8.41 (s, 1H), 8.32 (d, J = 5.0 Hz, 1H), 7.99 (dd, J = 10.0 and 5.0 Hz, 1H), 7.94 (d, J = 10.0 Hz, 1H), 7.87 (d, J = 10.0 Hz, 1H), 7.50-7.45 (m, 4H), 7.39-7.35 (m, 2H), 6.71 (d, J = 5.0 Hz, 1H), 6.52 (d, J = 5.0 Hz, 1H), 5.90 (s, 1H), 4.27 (qt, J = 5.0 H, 2H), 4.12-4.07 (m, 2H), 4.04-3.99 (m, 2H), 1.31 (t, J = 5.0 Hz, 3H)

### Step 2: Synthesis of 4-ethoxy-N-{3-fluoro-4-[2-(5-formylpyridin-2-yl)thieno[3,2-b]pyridin-7-yl]oxyphenyl}-1-(4-fluorophenyl)-2-oxo-1,2-dihydropyridine-3-carboxamide

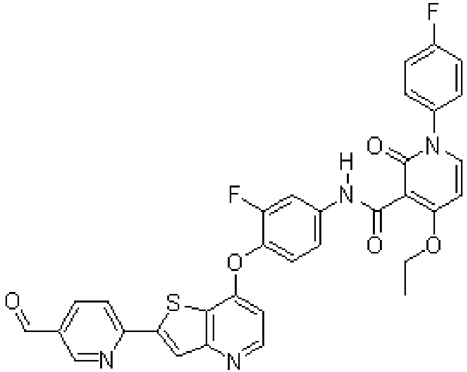

The compound (100 mg, 0.15 mmol) prepared in Step 1 was dissolved in a mixed solution of acetone and water, and then 2 mL of trifluoroacetic acid was added dropwise thereto at room temperature. Stirring was performed at 60°C for 12 hours. The resultant was cooled to room temperature, and the resulting solid was filtered under reduced pressure. Then, the resultant was washed with water and diethyl ether, and dried to obtain the title compound (82 mg, yield: 88%, off-white solid).
¹H NMR (500 MHz, DMSO-d₆) δ 10.64 (s, 1H), 10.14 (s, 1H), 9.15 (s, 1H), 8.59-8.58 (m, 2H), 8.52 (d, J = 5.0 Hz, 1H), 8.39 (d, J = 10.0 Hz, 1H), 7.95 (d, J = 10.0 Hz, 1H), 7.87 (d, J = 10.0 Hz, 1H), 7.51-7.45 (m, 4H), 7.39-7.36 (m, 2H), 6.78 (d, J = 5.0 Hz, 1H), 6.53 (d, J = 10.0 Hz, 1H), 4.27 (qt, J = 5.0 H, 2H), 1.31 (t, J = 5.0 Hz, 3H)

### Step 3: Synthesis of 4-ethoxy-N-[3-fluoro-4-({2-[5-(hydroxymethyl)pyridin-2-yl]thieno[3,2-b]pyridin-7-yl}oxy)phenyl]-1-(4-fluorophenyl)-2-oxo-1,2-dihydropyridine-3-carboxamide

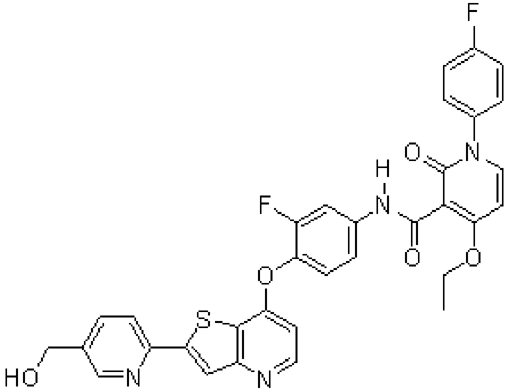

The compound (82 mg, 0.13 mmol) prepared in Step 2 was dissolved in 1,2-dichloroethane, and then triacetoxy sodium borohydride (83 mg, 0.39 mmol) was added thereto. Stirring was performed at room temperature for 12 hours. The reaction was terminated with saturated aqueous sodium bicarbonate solution, and extraction with dichloromethane was performed. Then, the resultant was dried over anhydrous sodium sulfate, and concentrated under reduced pressure. The resulting residue was purified by column chromatography to obtain the title compound (53 mg, yield: 64%, white solid).
¹H NMR (500 MHz, DMSO-d₆) δ 10.63 (s, 1H), 8.58 (s, 1H), 8.51 (d, J = 5.0 Hz, 1H), 8.33 (s, 1H), 8.25 (d, J = 10.0 Hz, 1H), 7.94 (d, J = 10.0 Hz, 1H), 7.89-7.86 (m, 2H), 7.50-7.45 (m, 4H), 7.39-7.36 (m, 2H), 6.69 (d, J = 5.0 Hz, 1H), 6.52 (d, J = 5.0 Hz, 1H), 5.43 (t, J = 5.0 Hz, 1H), 4.59 (d, J = 5.0 Hz, 2H), 4.27 (qt, J = 5.0 H, 2H), 1.31 (t, J = 5.0 Hz, 3H)

### Step 4: Synthesis of N-[4-({2-[5-(chloromethyl)pyridin-2-yl]thieno[3,2-b] pyridin-7-yl}oxy)-3-fluorophenyl]-4-ethoxy-1-(4-fluorophenyl)-2-oxo-1,2-dihydropyridine-3-carboxamide

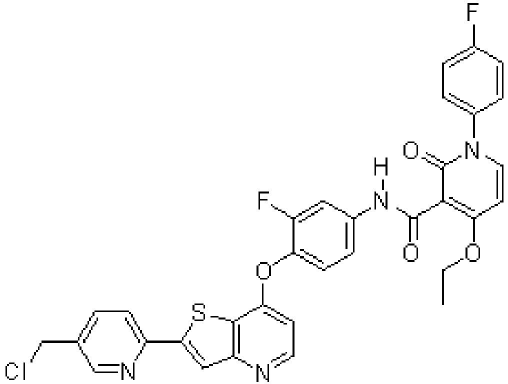

The compound (296 mg, 0.49 mmol) prepared in Step 3 was dissolved in dichloromethane, and then thionyl chloride (0.07 mL, 0.97 mmol) was added thereto. Stirring was performed at room temperature for 3 hours. The reaction was terminated with saturated aqueous sodium bicarbonate solution at 0°C, and extraction with dichloromethane was performed. The resultant was dried over anhydrous sodium sulfate and concentrated under reduced pressure, to obtain the title compound (240 mg, yield: 78%, yellow solid).
¹H NMR (500 MHz, DMSO-d₆) δ 10.63 (s, 1H), 8.72 (s, 1H), 8.52 (d, J = 5.0 Hz, 1H), 8.40 (s, 1H), 8.32 (d, J = 10.0 Hz, 1H), 8.04 (dd, J = 10.0 and 5.0 Hz, 1H), 7.94 (d, J = 15.0 Hz, 1H), 7.87 (d, J = 10.0 Hz, 1H), 7.50-7.46 (m, 4H), 7.39-7.36 (m, 2H), 6.71 (d, J = 5.0 Hz, 1H), 6.53 (d, J = 10.0 Hz, 1H), 4.88 (s, 2H), 4.27 (qt, J = 5.0 H, 2H), 1.31 (t, J = 5.0 Hz, 3H)

### Step 5: Synthesis of 4-ethoxy-N-[3-fluoro-4-({2-[5-(morpholinomethyl)pyridin-2-yl]thieno[3,2-b]pyridine-7-yl}oxy)phenyl]-1-(4-fluorophenyl)-2-oxo-1,2-dihydropyridine-3-carboxamide hydrochloride

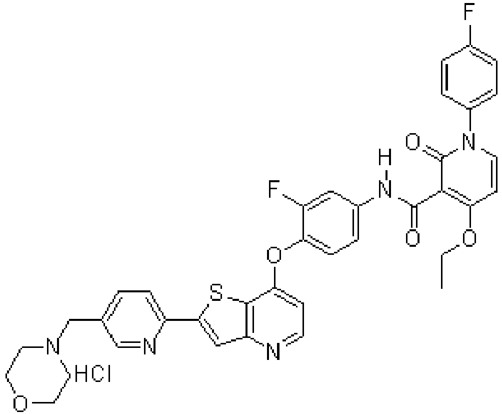

The compound (40 mg, 0.06 mmol) prepared in Step 4 was dissolved in acetonitrile, and then morpholine (0.01 mL, 0.12 mmol) and potassium carbonate (13 mg, 0.09 mmol) were added thereto. Stirring was performed at 80°C for 5 hours. The resultant was cooled to room temperature and concentrated under reduced pressure. Then, extraction with dichloromethane and a small amount of methanol was performed. The separated organic layer was dried over anhydrous sodium sulfate and concentrated under reduced pressure. The resulting residue was purified by column chromatography, and then 0.2 mL of 4M hydrochloric acid solution in 1,4-dioxane was used to obtain the title compound (16.6 mg, yield: 30%, off-white solid).
¹H NMR (500 MHz, DMSO-d₆) δ 10.66 (s, 1H), 8.81 (s, 1H), 8.60 (d, J = 5.0 Hz, 1H), 8.48 (s, 1H), 8.43 (d, J 5.0 Hz, 1H), 8.23 (d, J 5.0 Hz, 1H), 7.96 (d, J 15.0 Hz, 1H), 7.87 (d, J 5.0 Hz, 1H), 7.52-7.45 (m, 4H), 7.39-7.36 (m, 2H), 6.83 (d, J 5.0 Hz, 1H), 6.53 (d, J 10.0 Hz, 1H), 4.44 (s, 2H), 4.26 (qt, J 5.0 H, 2H), 3.96-3.94 (m, 2H), 3.77 (t, J 10.0 Hz, 2H), 3.32-3.30 (m, 2H), 3.14 (qt, J 10.0 2H), 1.31 (t, J 5.0 Hz, 3H)

### Example 19: Preparation of 4-ethoxy-N-[3-fluoro-4-({2-[5-(morpholinomethyl)pyridin-2-yl]thieno[3,2-b]pyridine-7-yl}oxy)phenyl]-2-oxo-1-phenyl-1,2-dihydropyridine-3-carboxamide hydrochloride

Preparation was done according to the synthesis route of Example 18, except that 4-ethoxy-2-oxo-1-phenyl-1,2-dihydropyridine-3-carboxylic acid of Preparation Example 2 was as a starting material in Step 1, to obtain the title compound (yield: 52.3%). 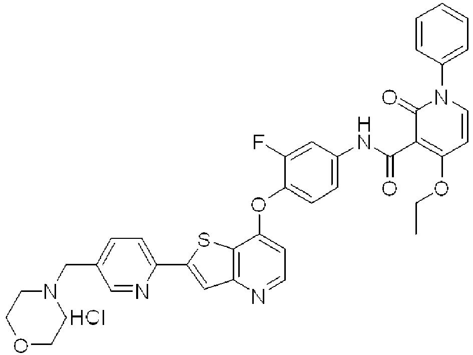 ¹H NMR (500 MHz, DMSO-d₆) δ 10.66 (s, 1H), 8.80 (s, 1H), 8.58 (d, J 5.0 Hz, 1H), 8.47 (s, 1H), 8.43 (d, J 10.0 Hz, 1H), 8.20 (d, J 10.0 Hz, 1H), 7.96 (d, J 15.0 Hz, 1H), 7.87 (d, J 5.0 Hz, 1H), 7.56-7.47 (m, 5H), 7.43-7.40 (m, 2H), 6.80 (d, J 5.0 Hz, 1H), 6.52 (d, J 5.0 Hz, 1H), 4.44 (s, 2H), 4.26 (qt, J 5.0 H, 2H), 3.97-3.95 (m, 2H), 3.74 (t, J 10.0 Hz, 2H), 3.33-3.30 (m, 2H), 3.14 (qt, J 10.0 2H), 1.31 (t, J 5.0 Hz, 3H)

### Example 20: Preparation of 4-ethoxy-N-{3-fluoro-4-[(2-{5-[(4-methylpiperazin-1-yl)methyl]pyridin-2-yl}thieno[3,2-b]]pyridin-7-yl)oxy]phenyl}-1-(4-fluorophenyl)-2-oxo-1,2-dihydropyridine-3-carboxamide hydrochloride

Preparation was done according to the synthesis route of Example 18, except that N-methylpiperazine was used in place of morpholine in Step 5, to obtain the title compound (yield: 22.2%). 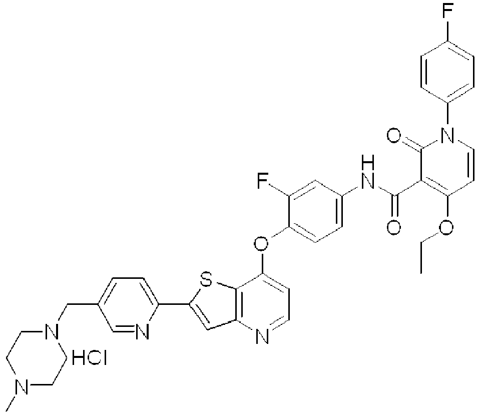 ¹H NMR (500 MHz, DMSO-d₆) δ 10.67 (s, 1H), 8.81 (brs, 1H), 8.60 (d, J 5.0 Hz, 1H), 8.46 (s, 1H), 8.41 (d, J 10.0 Hz, 1H), 8.20 (s, 1H), 7.96 (d, J 15.0 Hz, 1H), 7.87 (d, J 5.0 Hz, 1H), 7.52-7.45 (m, 4H), 7.39-7.36 (m, 2H), 6.84 (d, J 5.0 Hz, 1H), 6.53 (d, J 10.0 Hz, 1H), 4.26 (qt, J 5.0 H, 2H), 4.07 (brs, 8H), 3.56 (brs, 2H), 2.80 (s, 3H), 1.31 (t, J 5.0 Hz, 3H)

### Example 21: Preparation of 4-ethoxy-N-{3-fluoro-4-[(2-{5-[(4-methylpiperazin-1-yl)methyl]pyridin-2-yl}thieno[3,2-b]]pyridin-7-yl)oxy]phenyl}-2-oxo-1-phenyl-1,2-dihydropyridine-3-carboxamide hydrochloride

Preparation was done according to the synthesis route of Example 18, except that N-methylpiperazine was used in place of morpholine in Step 5, to obtain the title compound (yield: 31.4%). 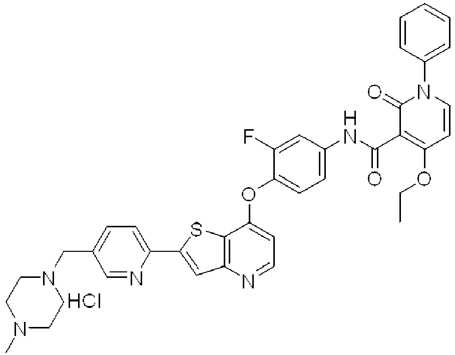 ¹H NMR (500 MHz, DMSO-d₆) δ 10.68 (s, 1H), 8.82 (brs, 1H), 8.61 (d, J 5.0 Hz, 1H), 8.47 (s, 1H), 8.41 (d, J 5.0 Hz, 1H), 8.23 (s, 1H), 7.97 (d, J 15.0 Hz, 1H), 7.87 (d, J 5.0 Hz, 1H), 7.56-7.46 (m, 5H), 7.42-7.40 (m, 2H), 6.86 (d, J 5.0 Hz, 1H), 6.52 (d, J 5.0 Hz, 1H), 4.26 (qt, J 5.0 H, 2H), 3.93 (brs, 8H), 3.58 (brs, 2H), 2.81 (s, 3H), 1.31 (t, J 5.0 Hz, 3H)

In addition, the following known compounds were used for comparison.

### Comparative Example 1

Compound 1 of US 8,536,200 B2, N-{4-[(2-amino-3-chloro-4-pyridinyl)oxy]-3-fluorophenyl}-4-ethoxy-1-(4-fluorophenyl)-2-oxo-1,2-dihydro-3-pyridinecarboxamide; the compound of Comparative Example 1 is BMS-777607 known as a RON inhibitor.

### Comparative Example 2

Compound of Example 73 of US 8,088,794 B2, N-(3-fluoro-4-(7-methoxyquinolin-4-yloxy)phenyl)-1-(2-hydroxy-2-methylpropyl)-5-methyl-3-oxo-2-phenyl-2,3-dihydro-1H-pyrazole-4-carboxamide.

### Comparative Example 3

Compound of Example 1 of US 8,030,302 B2, N-(3-fluoro-4-(1-methyl-6-(1H-pyrazol-4-yl)-1H-indazol-5-yloxy)phenyl)-1-(4-fluorophenyl)-6-methyl-2-oxo-1 ,2-dihydropyridine- 3-carboxamide.

### Experimental Example 1. RON activity inhibition assay: Examples 1 and 5

Enzymatic reaction was allowed to proceed using 2 ng/uL of 250 uM G4Y1 peptide, which serves as a substrate for RON enzyme, and 50 uM ATP in assay buffer (40 mM Tris-HCl, pH 7.5, 20 mM MgCl₂, 0.1 mg/mL bovine serum albumin, 50 uM DTT). The resultant was subjected to treatment with the compounds prepared in the examples and the comparative examples at various concentrations, and reaction was allowed to proceed at room temperature for 1 hour. Then, ADP-Glo™ Reagent and Kinase Detection Reagent were sequentially added thereto, and reaction was allowed to proceed at room temperature for 40 minutes and 30 minutes, respectively.

Subsequently, luminescence was measured using Wallaac Victor 2TM (PerkinElmer Life Sciences, 1420-042). Data were analyzed with the measured RLU values to verify activity of the RON inhibitors. The RLU value of a sample which was not treated with the compound was set as a 100% control, and the activity of the RON inhibitor was evaluated as a percentage of residual activity of the RON enzyme in the sample that was treated with the compound at the concentration to be tested. The concentration of the compound, at which RON enzyme activity inhibition of 50% occurs as compared with the control, was determined as IC₅₀ value (nM) of the RON inhibitor. The results are shown in Table 1.

**[Table 1]**

| **Example No.** | **ADP Glo assay RON, IC₅₀ (nM)** | **Example No.** | **ADP Glo assay RON, IC₅₀ (nM)** |
|---|---|---|---|
| Example 1 | 11.1 | Example 5 | 8.8 |

### Experimental Example 2. RON activity inhibitory capacity assay: Examples 12 to 21

Enzyme activity inhibitory potency (IC₅₀) of the thienopyridine derivatives was measured using fluorescence resonance energy transfer (FRET) technique.

RON kinase (Carna Bioscience) was diluted with kinase assay buffer (50 mM HEPES pH 7.5, 1 mM EGTA, 10 mM MgCl₂, 2 mM DTT, and 0.01% Tween-20) to a concentration of 2x (0.4 nM) the final reaction concentration, and the dilution was added to a 384-well plate at 5 µL/well.

As sample compounds, the compounds synthesized in the examples of the present invention and the compounds of the comparative examples were used. The sample compounds were respectively dissolved in 100% DMSO to obtain 1 mM stock solutions, which were then subjected to eight 10-fold serial dilutions. Each sample compound was diluted with the kinase assay buffer to a concentration of 4x the final reaction concentration, and then the dilution was added to the 384-well plate at 2.5 µL/well. Subsequently, the plate was incubated at room temperature for 15 minutes.

Next, ATP solution (Sigma Aldrich) prepared at 0.25 M concentration was diluted with the kinase reaction buffer to a concentration of 4x (60 µM) the final reaction concentration, and a tyrosine peptide substrate (Perkin-Elmer) was diluted therein to a concentration of 4x (400 nM) the final reaction concentration. The dilution was added to the 384-well plate at 2.5 µL/well. Then, the 384-well plate was incubated at room temperature, so that reaction was allowed to proceed for 60 minutes. After 60 minutes, EDTA (Sigma Aldrich) was diluted with TR-FRET detection buffer (Perkin-Elmer) to a concentration of 4x (40 mM) the final reaction concentration.

A europium-containing anti-phosphotyrosine antibody (Perkin-Elmer) was diluted in the diluted EDTA solution to a concentration of 4x (8 nM) the final reaction concentration, to prepare a stop solution for RON kinase reaction. The prepared stop solution was added to the 384-well plate at 10 µL/well to terminate the reaction, and then the plate was incubated at room temperature for 60 minutes. After 60 minutes, the plate was read on Victor X5 plate reader (Perkin-Elmer). At this time, the excitation wavelength was 340 nm and the emission wavelengths were 620 nm and 665 nm. The IC₅₀ value of the sample compound was calculated using GraphPad Prism™ 5, in which the emission energy at 665 nm was expressed as a function of the concentration of the sample compound to calculate the IC₅₀ value. The results are shown in Table 2.

**[Table 2]**

| **Item** | **IC₅₀ (nM)** | **Item** | **IC₅₀ (nM)** |
|---|---|---|---|
| Comparative Example 1 | 1.11 | Example 16 | 0.23 |
| Comparative Example 2 | 2.89 | Example 17 | 0.88 |
| Comparative Example 3 | 3.74 | Example 18 | 0.39 |
| Example 12 | 0.51 | Example 19 | 0.81 |
| Example 13 | 0.28 | Example 20 | 0.26 |
| Example 14 | 0.08 | Example 21 | 0.23 |
| Example 15 | 0.45 | | |

As shown in Table 2, the compounds of the examples have a very excellent inhibitory effect on the RON tyrosine kinase enzyme.

### Experimental Example 3. Cell growth inhibition assay: Examples 1 to 11

MTS assay was performed to identify whether the compound according to the present invention has a cancer cell proliferation inhibitory effect through inhibition of extracellular signal-regulated kinase activity.

The following assay was performed on MKN45 cell line (cell line expressing active RON), a human gastric cancer cell line. The MKN45 cell line was dispensed at a concentration of 5,000 cells/well in a 96-well plate containing RPMI-1640 medium (Gibco, Invitrogen) containing 10% FBS, and then the plate was incubated for 24 hours at a condition of 5% CO₂ and 37°C. Then, the respective wells were subjected to treatment with the compounds prepared in the examples, each of which was sequentially diluted from its high concentration of 10 µM. For a solvent control, the respective wells were subjected to treatment with dimethyl sulfoxide (DMSO) at the same concentrations (% (v/v)) to those used for the treatment with the compounds. Then, the respective cells were incubated for 48 hours.

In order to identify degree of viability of the cells, a mixture of MTS and phenazine methosulfate (PMS) provided in CellTiter 96 AQuous Non-Radioactive Cell Proliferation Assay Kit (Promega) was added to each cultured cell medium. Additional incubation was performed at a condition of 37°C for 2 hours and 30 minutes. Then, absorbance was measured at 490 nm. Degree of inhibition of cell proliferation depending on the treatment concentrations of each compound was calculated based on the absorbance of the solvent-controlled cells that were not treated with the compound, wherein the concentration of each compound at which proliferation of cancer cells is inhibited by 50% was determined as IC₅₀ (nM) value. The results are shown in Table 3. In the following, A represents < 10 nM, B represents 10 to 50 nM, and C represents > 50 nM.

**[Table 3]**

| **Item** | **Growth inhibitory capacity on MKN45 cells, IC₅₀** | **Item** | **Growth inhibitory capacity on MKN45 cells, IC₅₀** |
|---|---|---|---|
| Example 1 | A | Example 7 | C |
| Example 2 | c | Example 8 | C |
| Example 3 | B | Example 9 | B |
| Example 4 | B | Example 10 | C |
| Example 5 | B | Example 11 | C |
| Example 6 | B | | |

### Experimental Example 4. Measurement of cell killing efficacy: Examples 12 to 21

KM12C (80015, KCLB, MEM + 10% FBS) colon cancer cell line carrying mutant (Δ160) was prepared at 5 × 10⁴ cells/well in 6-well plates. After 24 hours, while performing medium exchange, the cells were subjected to treatment with the sample compounds at respective concentrations (1 µM, 5 µM) for 48 hours.

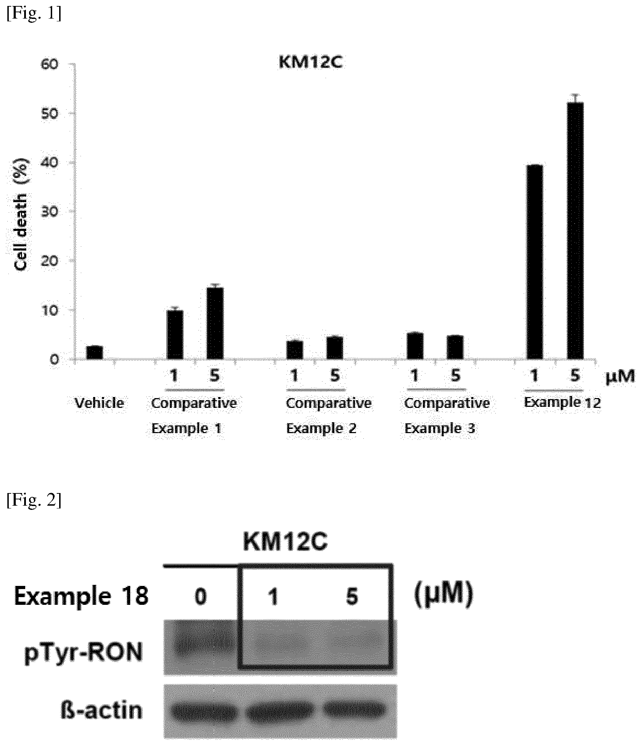

As the sample compounds, the compounds synthesized in the examples of the present invention and the compounds of the comparative examples were used. After 48 hours, the cell suspensions were collected and centrifuged (1,500 rpm, 4 minutes) to obtain the cells, and live cells and dead cells were quantitated (n = 2) through trypan blue (15250-061, Gibco) exclusion assay. Statistical analysis was performed using Excel™ T-test from Microsoft. The results obtained by measuring cell death (%) of the compounds of Comparative Examples 1 to 3 and Example 1 are illustrated in Fig. 1. In addition, cell deaths of the compounds of the examples and the comparative examples were respectively measured, and the results are shown in Table 4 as rates relative to Comparative Example 1.

**[Table 4]**

| **Item** | **Cell death rate (relative to Comparative Example 1)** | |
|---|---|---|
| | **KM12C cells** | |
| | 1 µM | 5 µM |
| Comparative Example 1 | x 1.00 | x 1.00 |
| Comparative Example 2 | x 0.38 | x 0.31 |
| Comparative Example 3 | x 0.54 | x 0.33 |
| Example 12 | x 3.37 | x 2.47 |
| Example 13 | x 2.38 | x 1.32 |
| Example 14 | x 2.26 | x 2.87 |
| Example 15 | x 1.58 | x 1.43 |
| Example 16 | x 0.44 | x 0.69 |
| Example 17 | x 2.48 | x 2.06 |
| Example 18 | x 1.52 | x 1.17 |
| Example 19 | x 1.52 | x 1.11 |
| Example 20 | x 1.99 | x 2.13 |
| Example 21 | x 2.07 | x 1.11 |

As shown in Table 4 and Fig. 1, the compounds of the examples exhibit relatively high anticancer efficacy in the colon cancer cell line (KM12C) carrying RON mutant (Δ160), as compared with the conventional compounds.

### Experimental Example 5. Identification of cytotoxicity using MTS assay

In order to identify the specific cytotoxicity of drugs, cultured cells were treated with 0.05% trypsin/EDTA (0.5% trypsin EDTA (10X), Product No.: 15400-054, Gibco) to be detached from the culture dish, and then 3,000 cells (KM12C) or 2,000 cells (HT29, Colo 320 HSR) were dispensed into each well. After 24 hours, sample compounds were dissolved in DMSO (Product No.: D8418-250ML, SIGMA) as a solvent to make 10 mM stock solutions which were then diluted and used. As the sample compounds, the compounds synthesized in the examples of the present invention and the compounds of the comparative examples were used. The stock solutions were diluted with DMSO, respectively, to eight concentrations (for KM12C) starting from 10 mM by a factor of 1/10 and to nine concentrations (for HT29) starting from 10 mM by a factor of 1/2. The diluted drugs were allowed to have a concentration of 100 µM based on the highest concentration. The cells were treated with the drugs, and then incubated in a 5% CO₂ incubator at 37°C for 72 hours.

After 72 hours, 20 µL of MTS solution (CellTiter 96 Aqueous One Solution Cell Proliferation Assay, Product No.: G3581, Promega) was dispensed into each well and mixed well. Then, incubation was performed in a 5% CO₂ incubator at 37°C for 1 to 4 hours. Subsequently, absorbance values were measured at a wavelength of 490 nm using Victor X5 plate reader (Perkin-Elmer) and IC₅₀ values were calculated therefrom. The results are shown in Table 5.

**[Table 5]**

| **Item** | **KM12C IC₅₀ (µM)** | **Item** | **KM12C IC₅₀ (µM)** |
|---|---|---|---|
| Comparative Example 1 | 0.511 | Example 18 | 0.007 |
| Comparative Example 3 | 0.1173 | Example 19 | 0.003 |
| Example 12 | 0.045 | Example 20 | 0.009 |
| Example 15 | 0.063 | Example 21 | 0.006 |

As shown in Table 5, the compounds of the examples exhibit relatively high anticancer efficacy in the colon cancer cell line (KM12C) carrying RON mutation (Δ160), as compared with the conventional compounds.

### Experimental Example 6. Efficacy analysis of Example 18 in KM12C carrying RON mutant (Δ160)

In MTS assay for Example 18 and Comparative Example 1, KM12C cell line was seeded at 1,000 cells per well into 96-well plates. After 24 hours, the cells were treated with various concentrations (0.000001 uM to 10 uM) of Example 18 and Comparative Example 1 in 100 ul of 10% FBS-MEM medium (Welgene) per well. Subsequently, the cells were incubated for 72 hours. Then, 20 µl of MTS solution (Promega) was added thereto, and incubation was performed for 2 hours. Then, the absorbance was measured at 490 nm. The IC₅₀ values for Example 18 and Comparative Example 1 were calculated using GraphPad Prism™ 5 to investigate cytotoxicity.

The cell death-inducing ability of Example 18 and Comparative Example 1 was measured in KM12C with RON mutant (Δ160), a cetuximab-resistant cell line, and Colo320HSR cell line, a cell line not expressing RON. The two colon cancer cell lines were seeded, respectively, onto 60-mm plates at 2 × 10⁵ cells. Then, after 24 hours, while performing medium exchange, the cells were subjected to treatment with the sample compounds at respective concentrations (1 µM, 5 µM) for 48 hours. After 48 hours, the cells and cell suspensions were collected (1,500 rpm, 3 minutes), and live cells and dead cells were quantitated (n = 2) through trypan blue (15250-061, Gibco) exclusion assay.

The cell killing efficacy of Example 18 and Comparative Example 1 was analyzed, and then the cells were collected. Western blotting was performed to identify inhibitory capacity of Example 18 on active RON. RIPA Lysis Buffer (T&I) was added to the respective cells so as to achieve lysis. Then, BCA kit (Pierce) was used to quantify proteins. The proteins were loaded onto 6% SDS-polyacrylamide with a protein concentration of 40 µg, and electrophoresis was performed at 125 V.

The proteins separated by electrophoresis were transferred to PVDF membrane (Millipore) at 270 mA for 90 minutes using transfer buffer (20% methanol, 25 mM Tris-HCl, 192 mM glycine), and blocking was performed with 5% non-fat skim milk-TBST solution for 1 hour. Subsequently, the proteins were reacted with primary antibody (active RON, beta actin), which was diluted 1:1,000 in 5% non-fat skim milk-TBST solution, at 4°C for 24 hours or 48 hours. Then, washing with TBST was performed three times. The resultant was reacted with anti-rabbit and anti-mouse IgG conjugated horseradish peroxidase (HRP) secondary antibody, which was diluted at 1:1,000, at room temperature for 2 hours. Then, washing with TBST was washed three times. Subsequently, the membrane was treated with the ECL solution (GE Healthcare), and developed by exposure to an X-ray film. Then, the bands were checked.

The results are as shown in Table 6, Table 7, and Fig. 2.

**[Table 6]**

| **Compound No.** | **In vitro enzyme assay (IC₅₀, nM)** | **MTS (IC₅₀, nM)** |
|---|---|---|
| | | **KM12C (RONΔ160)** |
| Comparative Example 1 | 1.11 | 358 |
| Example 18 | 0.39 | 6.9 |

### Comparative Example: BMS777607

**[Table 7]**

| **Compound No. (µM)** | **Colo320HSR (RON-)** | | **KM12C (RONΔ160)** | |
|---|---|---|---|---|
| | **1** | **5** | **1** | **5** |
| Comparative Example 1 | 5.9 | 8.2 | 11.7 | 21.1 |
| Example 18 | NR | NR | 29.0 | 35.2 |

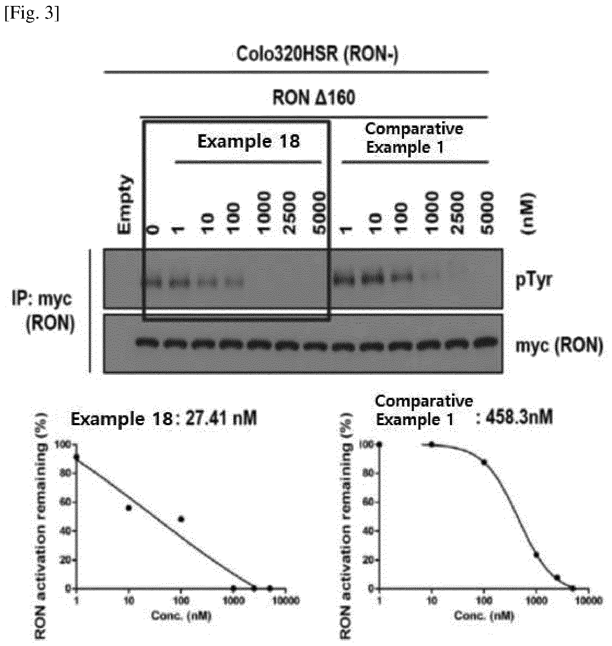

Table 6, Table 7, and Fig. 3 show that cell killing efficacy is observed in a case where KM12C which carries RON mutant (Δ160) and thus expresses active RON, a cetuximab-resistant cell line, is treated with Example 18. It was found that from the RON kinase assay results for Example 18, Example 18 exhibits high inhibition efficacy on kinase as compared with Comparative Example 1. As a result of the comparative analysis through MTS assay, it was identified that Example 18 exhibits cell growth inhibition efficacy as high as 6.9 nM.

As can be seen from the above results, no reactivity with the drug was observed in a case of Colo320HSR which is RON-negative. In addition, in a case where KM12C, a cell line expressing active RON, is treated with Example 18, cell killing efficacy was observed as compared with Comparative Example 1. Here, it was identified, through Western blotting, that expression of active RON decreases (Fig. 2).

### Experimental Example 7. Kinase Assay for RON mutants

As a way capable of replacing RON mutant, each of the mutants (Δ160, Δ155) was transfected into Colo320HSR, a RON-negative cell line, and the cell line was subjected to treatment with Example 18 or Comparative Example 1 at respective concentrations. Then, it was checked, through immunoprecipitation and Western blotting, whether expression of active RON decreases. The detailed method is as follows. The RON-negative Colo320SHR colon cancer cell line was transfected with 12 ug of each of RONΔ160 and RON Δ155 using lipofectamine 2000 (Invitrogen, cat#11668-019). For the same transfection efficiency, after 24 hours, the transformed strains were dispensed into a 60-mm dish. 48 hours after transfection, treatment with drugs of Comparative Example 1 and Example 18 was performed, respectively, in a dose-dependent manner. After 2 hours of drug treatment, the cells were collected and washed with PBS. Then, the washed cells were lysed with RIPA Lysis Buffer (T&I, cat# BRI-9001).

Protein concentrations were quantified using the Bradford (BioRad, cat#5000205) method. Then, 500 ug of protein and 1 ug of myc antibody (Cell Signaling Technology, Inc., cat#2276) were mixed with RIPA buffer, and reacted at 4°C overnight. To the immunoprecipitated solution was added 20 ul of Protein-G agarose beads (Santa Cruz Biotechnology, sc-2002). Then, using a stirrer, reaction was allowed to proceed at 4°C for 2 hours. Washing with RIPA buffer was performed 5 times. Then, 20 ul of 2XSDS sample buffer was added thereto, and then reaction was allowed to proceed at 100°C for 5 minutes.

Electrophoresis was performed using SDS-PAGE, and then proteins were transferred to PVDF membrane. Then, phospho-Tyrosine antibody (Cell Signaling Technology, Inc., cat#9411) and myc antibody (Cell Signaling Technology, Inc., cat#2276) were diluted 1:1,000, respectively, in 5% skim milk TBS-T (T&I, cat#BTT-9120) solution, and then reacted at 4°C overnight. Washing with the TBS-T solution was performed three times for 10 minutes each. Then, anti-mouse-HRP antibody (Cell Signaling Technology, Inc., cat# 7076) was diluted 1:2,000 in 5% skim milk in 1XTBS-T solution, and then reacted at room temperature for 2 hours. Washing with IX TBS-T solution was performed three times, and then the film was developed using the ECL solution (GE Healthcare, cat# RPN2108). The developed film was analyzed with densitometry using Image-J, and then IC₅₀ was calculated using Graphpad Prism (GraphPad Software, Inc.).

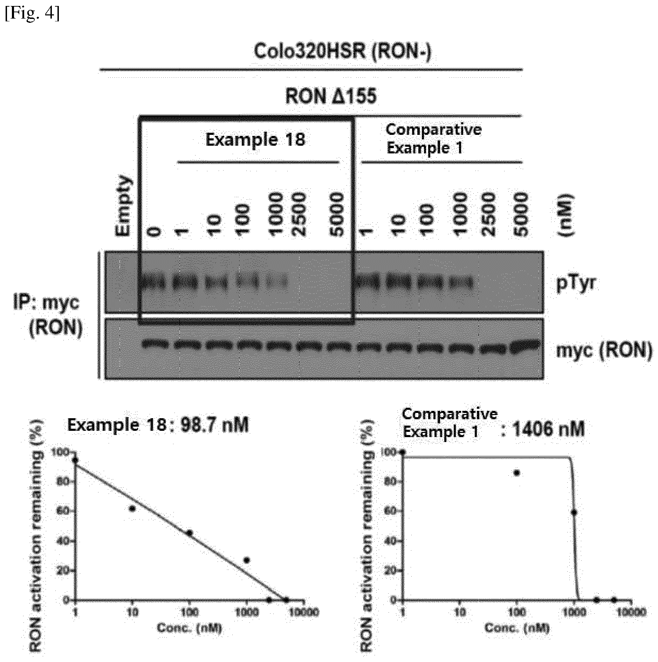

As a result, a rapid decrease with drug treatment was observed in the group treated with Example 18, as compared with Comparative Example 1 (Figs. 3 and 4). Based on these results, the IC50 value was calculated on the basis of the degree of RON activity. As a result, it was identified that Example 18 exhibits high inhibition efficacy as compared with Comparative Example 1.

### Experimental Example 8. Analysis of drug efficacy after overexpression of RON mutants (mt #1: Δ160, mt #2: Δ155) in Colo320HSR cell line

Efficacy analysis for RON mutants was performed by overexpressing RON mutants, and then identifying cell death-inducing ability through Western blotting. Colo320HSR cell line was seeded onto a 100-mm plate, and then a RON mutant #1 (Δ160)- or RON mutant #2 (Δ155)-expressing vector was introduced into the cell line using a transfection solution (Polyplus). After 24 hours, the cell line was seeded onto a 60-mm plate at 2 × 10⁵ cells. Then, after 24 hours, while performing medium exchange, the cells were subjected to treatment with Example 18 at respective concentrations (1 µM, 5 µM) for 48 hours.

After 48 hours, the cells and cell suspensions were collected (1,500 rpm, 3 minutes), and live cells and dead cells were quantitated (n = 2) through trypan blue exclusion assay. Subsequently, the remaining cells were collected and lysed by adding RIPA Lysis Buffer (T&I) to the respective cells for the purpose of identifying, with Western blotting, inhibitory capacity on active RON and cell death-inducing ability. Then, proteins were quantified by BCA kit (Pierce). The proteins were loaded onto 8% or 15% SDS-polyacrylamide with a protein concentration of 40 µg, and electrophoresis was performed at 125 V. The proteins separated by electrophoresis were transferred to PVDF membrane (Millipore) at 270 mA for 90 minutes using transfer buffer (20% methanol, 25 mM Tris-HCl, 192 mM glycine), and then blocking was performed with 5% non-fat skim milk-TBST solution for 1 hour.

Subsequently, the proteins were reacted with primary antibodies (active RON, cleaved caspase-3, beta actin), which were diluted 1:1,000 in 5% non-fat skim milk-TBST solution, at 4°C for 24 hours or 48 hours. Then, washing with TBST was performed three times. The resultant was reacted with anti-rabbit and anti-mouse IgG conjugated horseradish peroxidase (HRP) secondary antibodies, which were diluted at 1:1,000, at room temperature for 2 hours. Then, washing with TBST was washed three times. Subsequently, the membrane was treated with the ECL solution (GE Healthcare), and developed by exposure to an X-ray film. Then, the bands were checked.

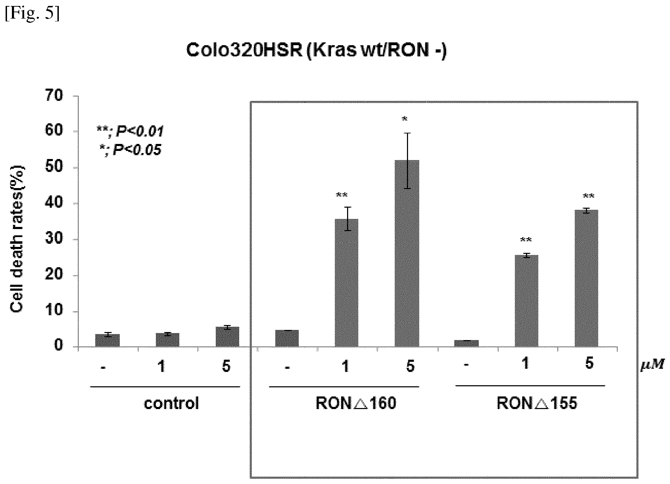

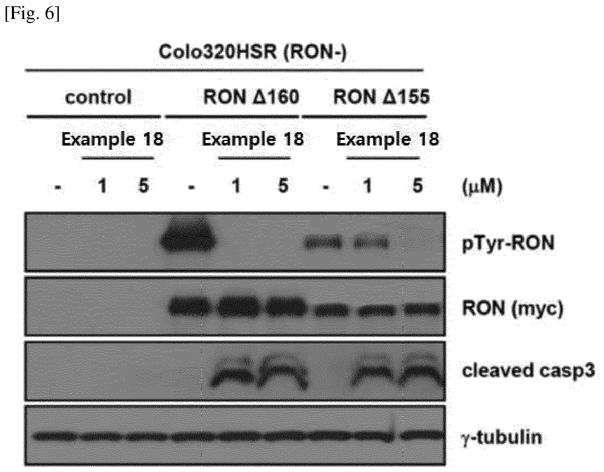

As a result, Colo320SHR, a cell line which is RON-negative and KRAS wt, was transfected with RON mutant #1 (Δ160) or RON mutant #2 (Δ155) using lipofectamine 2000, and drug efficacy of Example 18 was analyzed. The cells were subjected to treatment with Example 18 at respective concentrations, and then collected after 48 hours. Cell deaths were checked through trypan blue exclusion assay. As a result, cell death induction at the respective concentrations is observed in the group transfected with the RON mutant (Fig. 5). Here, changes in protein expression were analyzed through Western blotting. As a result, it was identified that the active RON shows a decreased expression level and cleaved caspase 3 shows an increased expression level (Fig. 6).

### Experimental Example 9. Analysis of action mechanism of Example 18 in KM12C cell line carrying RON mutant (Δ160)

KM12C cell line was seeded at 5 × 10⁵ cells onto a 100-mm plate. Then, after 24 hours, the cells were subjected to treatment with Example 18 at respective concentrations (1 µM, 5 µM) for 48 hours. After 48 hours, the cells and cell suspensions were collected (1,500 rpm, 3 minutes), and changes in expression of related proteins were checked with Western blotting. RIPA Lysis Buffer (T&I) was added to the respective cells so as to achieve lysis. Then, BCA kit (Pierce) was used to quantify proteins. Then, the proteins were loaded onto 8% SDS-polyacrylamide, and electrophoresis was performed at 125 V.

The proteins separated by electrophoresis were transferred to PVDF membrane (Millipore) at 270 mA for 90 minutes using transfer buffer (20% methanol, 25 mM Tris-HCl, 192 mM glycine). Then, blocking was performed with 5% non-fat skim milk-TBST solution for 1 hour. Subsequently, the proteins were reacted with primary antibodies, which were diluted 1:1,000 in 5% non-fat skim milk-TBST solution, at 4°C for 24 hours or 48 hours. Then, washing with TBST was performed three times. The resultant was reacted with anti-rabbit and anti-mouse IgG conjugated HRP secondary antibodies, which were diluted at 1:1,000, at room temperature for 2 hours. Then, washing with TBST was performed three times. Subsequently, the membrane was treated with the ECL solution (GE Healthcare), and developed by exposure to an X-ray film. Then, the bands were checked.

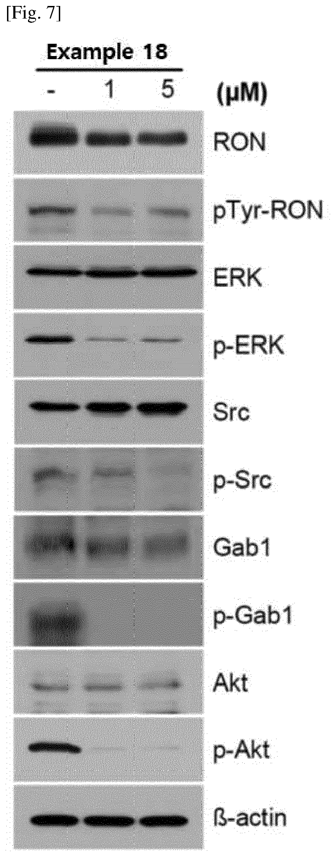

As a result, KM12C which carries RON mutant (Δ160) and thus expresses active RON, a cetuximab-resistant cell line, was subjected to treatment with Example 18 at respective concentrations, and then changes in expression of related proteins were checked through Western blotting. As a result, it was identified that the active RON decreases, and p-ERK, p-Src, p-Akt, and p-Gab1 decrease (Fig. 7).

### Experimental Example 10. Identification of tumor growth inhibition efficacy in an animal model transplanted with KM12C cell line

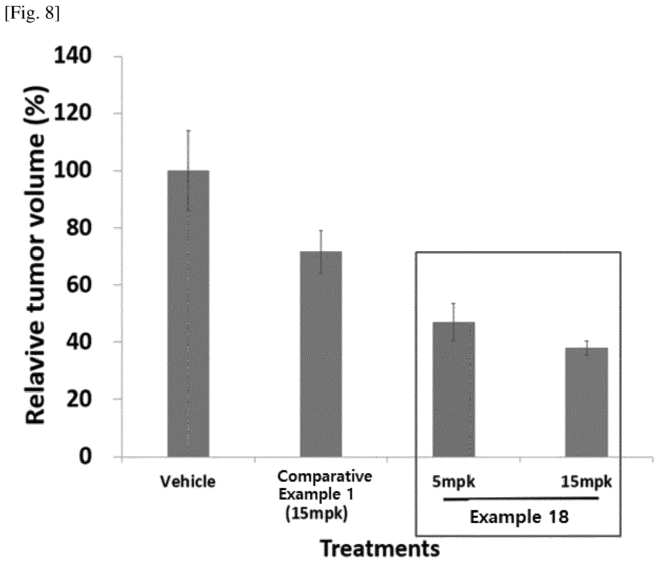

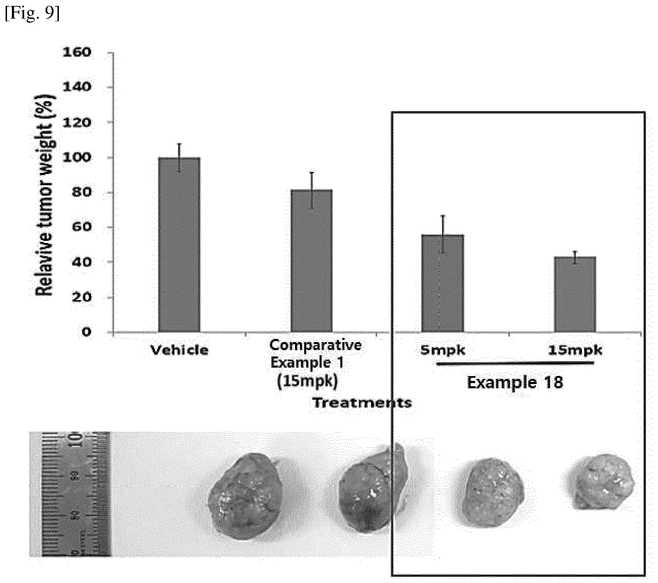

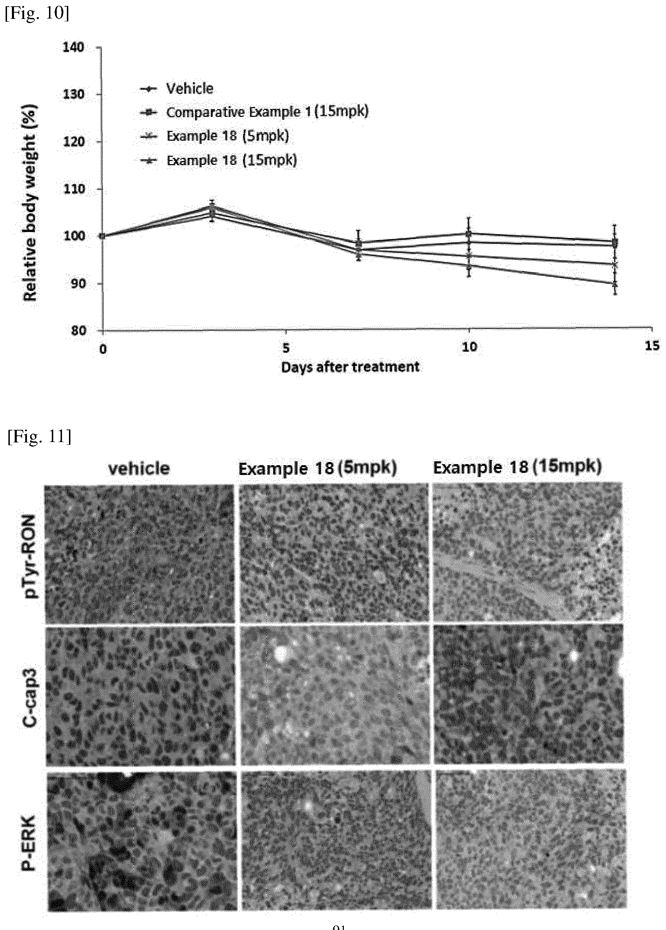

5-Week-old female BALB/c nude mice were purchased and acclimatized for 1 week. Then, human-derived colon cancer cell line KM12C (1.5×10⁶ cells/mice) (cell line expressing active RON/RON mutant (Δ160)) was diluted in PBS, and injected subcutaneously (100 µl) into the right dorsal side of the mice. Comparative Examples 1 and Example 18 were orally administered when the tumor reached a size of about 100 mm³. The drug was administered once a day for 2 weeks, and tumor size and body weight were measured twice a week. As a result, that tumor growth inhibition efficacy with drug administration was observed in the group having received Example 18 (Fig. 8). In addition, after completion of the drug administration, the experimental animals were euthanized, and then the tumors were extracted and weighed. As a result, decreased tumor weight was observed in the Example 18 treatment group as compared with the vehicle group (Fig. 9). Here, it was identified that there is no change in mouse body weight (Fig. 10).

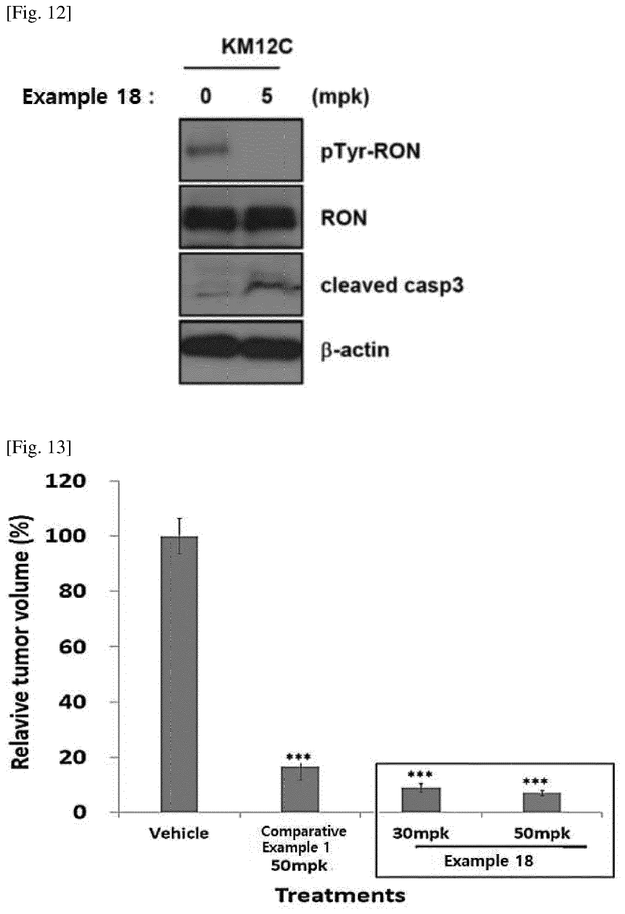

In addition, a portion of the extracted tumor tissue was fixed with 10% formalin, and then a paraffin block was produced therefrom. Then, changes in expression of each protein were checked through immunochemical staining. As a result, it was identified that in the group having received Example 18, expression of the active RON and p-ERK decreases, whereas expression of cleaved caspase 3 is induced (Fig. 11). Tumor tissues were collected from the vehicle group and the 5 mpk (mg/kg) treatment group, and changes in protein expression were compared through Western blotting. As a result, it was identified that the active RON shows a decreased expression level and cleaved caspase 3 shows an increased expression level (Fig. 12).

### Experimental Example 11. Efficacy analysis of Example 18 in an animal model transplanted with KM12C cell line

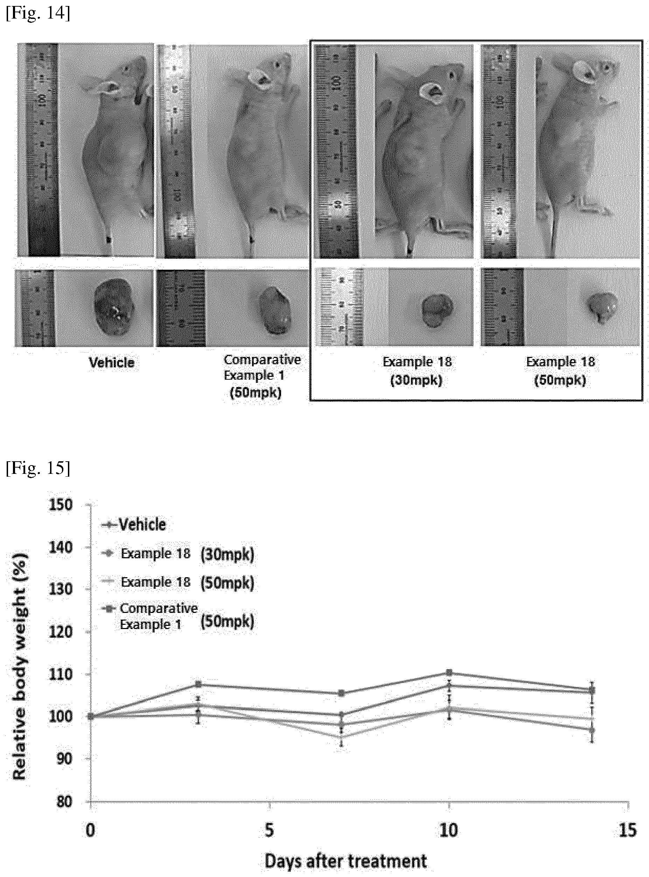

5-Week-old female BALB/c nude mice were purchased and acclimatized for 1 week. Then, human-derived colon cancer cell line KM12C (1.5×10⁶ cells/mice) was diluted in PBS, and injected subcutaneously (100 µl) into the right dorsal side of the mice. Comparative Examples 1 and Example 18 were orally administered when the tumor reached a size of about 100 mm³. The drug was administered at 30 mpk and 50 mpk once a day for 2 weeks, and tumor size and body weight were measured twice a week. As a result, it was identified that both Comparative Example 1 and Example 18 show very high tumor growth inhibition efficacy (Figs. 13 and 14, and Table 8). Here, it was identified there is little change in mouse body weight (Fig. 15). After completion of the drug administration, the experimental animals were euthanized, and then the tumors were extracted and weighed to perform comparative analysis. As a result, high tumor growth inhibition (TGI) efficacy was observed in the drug administration group as compared with Comparative Example 1.

**[Table 8]**

| **Compound** | **Example 18** | | **Comparative Example 1** | |
|---|---|---|---|---|
| Dose (mg/kg) | 30 | 50 | 50 | 30 |
| TGI (%) | 82.4 | 84.8 | 83.7 | 82.4 |

### Experimental Example 12. Identification of effective dose concentration in an animal model transplanted with KM12C cell line

5-Week-old female BALB/c nude mice were purchased and acclimatized for 1 week. Then, human-derived colon cancer cell line KM12C (1.5×10⁶ cells/mice) was diluted in PBS, and injected subcutaneously (100 µl) into the right dorsal side of the mice. Example 18 was orally administered at respective doses when the tumor reached a size of about 100 mm³. The drug was administered once a day for 2 weeks, and tumor size and body weight were measured twice a week to perform comparative analysis. After completion of the drug administration, the experimental animals were euthanized, and then the tumors were extracted and weighed to perform comparative analysis.

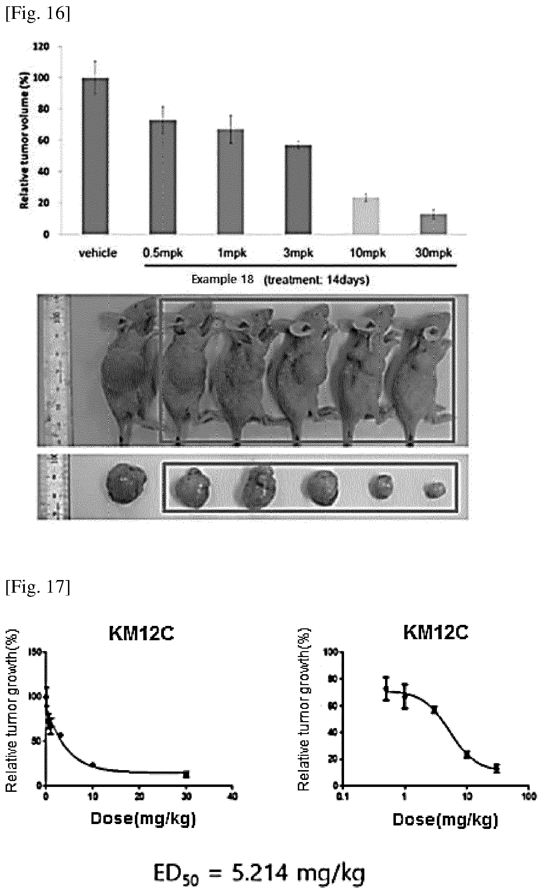

As a result, it was identified that tumor growth is inhibited in a concentration-dependent manner (Fig. 16). Here, it was identified that tumor tissue size and weight also decrease with administration concentrations. Based on these results, it was identified that the effective dose concentration is 5.214 mpk (Fig. 17).

### Experimental Example 13. Efficacy analysis of Example 18 in an animal model transplanted with colon cancer patient tissue tumor cells

5-week-old female BALB/c nude mice were purchased and acclimatized for 1 week. Then, colon cancer patient-derived cancer tissue was transplanted into the right dorsal side of the mice. Example 18 was orally administered when the tumor reached a size of about 100 mm³. The drug was administered once a day for 4 weeks, and tumor size and body weight were measured twice a week to perform comparative analysis. After completion of the drug administration, the experimental animals were euthanized, and then the tumors were extracted and weighed to perform comparative analysis.

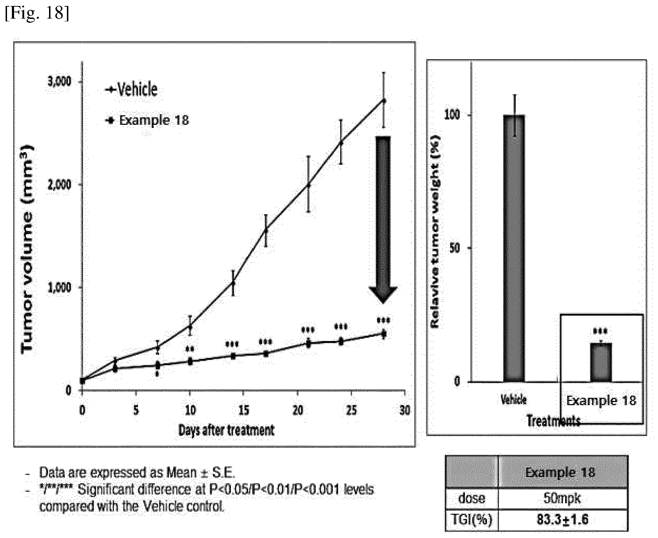

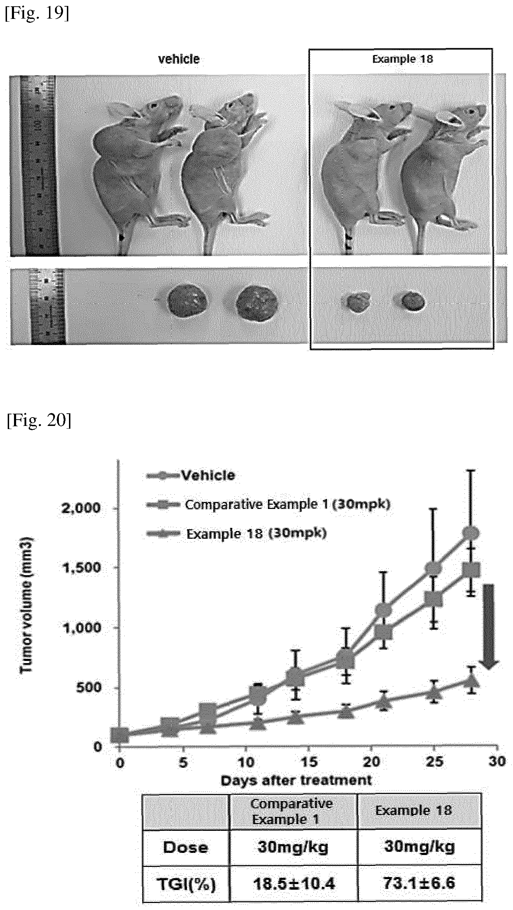

As a result, it was identifiable that tumor growth is inhibited in a case where Example 18 is administered to an animal model (RON mutant: Δ160) into which tumor cells in colon cancer patients have been transplanted. The drug was orally administered on a daily basis, and the dose used was 50 mpk. As a result of drug administration for 4 weeks, it was identified that tumor growth is inhibited by 80% or more (Fig. 18). In addition, after completion of the drug administration, the tumor tissues were extracted and weighed to perform comparative analysis. As a result, it was identified that tumor growth is inhibited by about 83% as compared with the vehicle (Figs. 19 and 18). The lower pictures of Fig. 19 show tumors extracted from representative individuals of the respective groups.

### Experimental Example 14. Efficacy analysis of Example 18 in an animal model transplanted with colon cancer patient tissue tumor cells

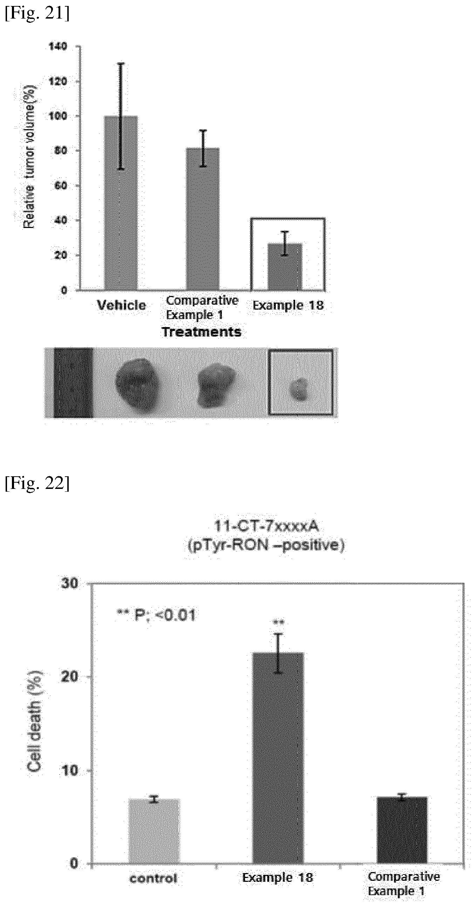

Example 18 and Comparative Example 1 were administered to an animal model transplanted with the same patient tissue (active RON/RON mutant #1: Δ160) cells as in the animal model transplanted with colon cancer patient tissue tumor cells at 30 mpk for 4 weeks. As a result, it was identified that the group having received Example 18 exhibits tumor growth inhibition efficacy of about 73%% (Fig. 20). On the other hand, it was identified that the group having received Comparative Example 1 exhibits low efficacy of about 18% as compared with Example 18 (Fig. 21). The lower pictures of Fig. 21 show tumors extracted from representative individuals of the respective groups.

### Experimental Example 15. Efficacy analysis of Example 18 depending on expression of active RON in colon cancer patient-derived cell lines

The following condition and methods were used to perform the present experiment. Two human colon cancer-derived cell lines (KRAS normal, RON active and inactive) were cultured in REBM (Lonza) culture medium. Then, the respective cell lines were seeded at 5×10⁵ cells onto 24-well plates, in which one sterile 12 mmΦ microscope cover glass was placed on each well, and incubated. The respective colon cancer patient-derived cell lines under incubation were subjected to treatment with Example 18 and Comparative Example 1, respectively, at 5 ug, and then incubated for 48 hours. Each incubated plate was washed twice with PBS, and then fixed with 100% Me-OH at room temperature for 5 minutes.

The Me-OH was removed, and washing with PBS was performed three times for 5 minutes each. Then, 100 ul of 0.1% Triton X-100 (in PBS) was dispensed thereinto and permeabilization was allowed to occur for 5 minutes. Washing with PBS was performed three times for 5 minutes each, and then PBS was added thereto to prevent drying. Parafilm was attached to the 24-well plate cover, and then PBS-T (PBS + 0.1% Tween 20) containing 3% BSA was dropped by 20 µl onto the parafilm. The cover glass on which each colon cancer patient-derived cell line was fixed was placed on 3% BSA with the surface, to which the cells are attached, facing the bottom, so that blocking was performed at room temperature for 30 minutes.

Subsequently, parafilm was attached to a new 24-well plate cover. Primary antibodies (cytokeratin 18, cleaved caspase 3) were diluted in PBS-T (PBS + 0.1% Tween 20) containing 1% BSA, and then the dilution was dropped by 10 ul onto the parafilm. Then, the blocked cover glass was placed thereon with the surface, to which the cells are attached, facing the bottom, and reaction was allowed to proceed at 4°C overnight.

Each cover glass after completion of the primary antibody reaction was placed in a 24-well plate containing PBS-T (PBS + 0.1% Tween 20), with the surface, to which the cells are attached, facing the top, and washing was performed three times for 5 minutes each. Subsequently, parafilm was attached to a new 24-well plate cover. Secondary antibodies (immunofluorescence secondary antibodies) were diluted in PBS-T (PBS + 0.1% Tween 20) containing 1% BSA, and then the dilution was dropped by 10 ul onto the parafilm. Then, the cover glass was placed thereon with the surface, to which the cells are attached, facing the bottom, and reaction was allowed to proceed at room temperature for 2 hours.

Each cover glass after completion of the second antibody reaction was placed in a 24-well plate containing PBS-T (PBS + 0.1% Tween 20), with the surface, to which the cells are attached, facing the top, and washing was performed three times for 5 minutes each. Then, 1 µg/mL Hoechst stain (DAPI) was mixed with PBS-T (PBS + 0.1% Tween 20), and then the mixture was added at 100 ul to each well of a 24-well plate. Reaction was allowed to proceed for 1 minute. Then, washing with PBS-T (PBS + 0.1% Tween 20) was performed twice, and a fluorescence microscope was used to identify the staining. Thereafter, 5 ul of mount solution was dropped on the slide glass to perform staining. Then, the cover glass was placed thereon, and a fluorescence microscope was used to take a fluorescence image.

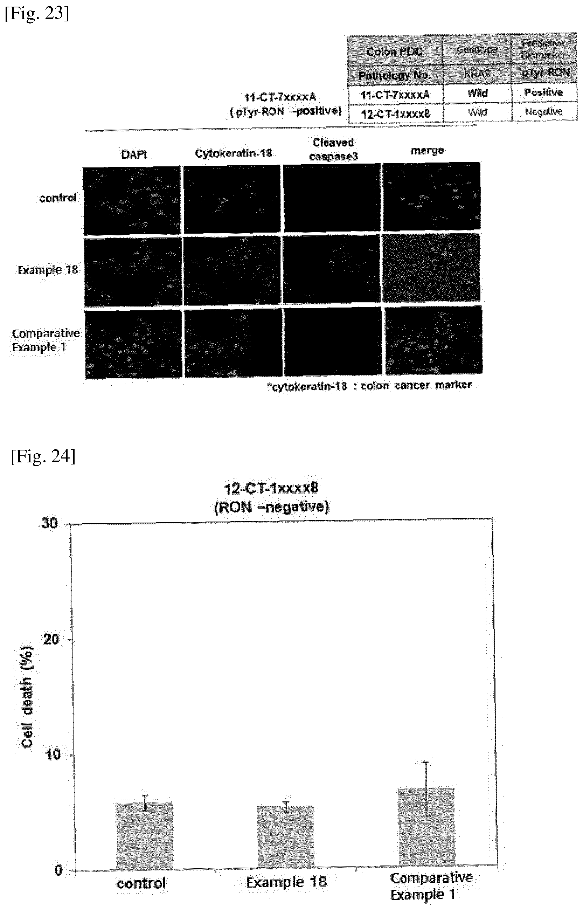

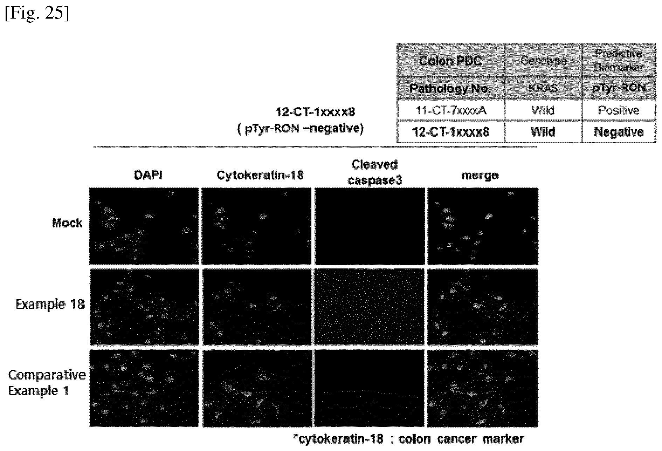

As a result, it was identified that in a case where among the colon cancer patient-derived cell lines, the cell line expressing active RON is subjected to treatment with Example 18 and Comparative Example 1, the group treated with Example 18 exhibits cell death-inducing efficacy (Figs. 22 and 23). In addition, it was identified that in a case where the active RON is negative, no reactivity is caused by the drug treatment (Figs. 24 and 25). Here, cytokeratin 18 was used as a marker for colon cancer.

### Experimental Example 16. Analysis of RON biomarker gene in a colon cancer cell line

35 of human colon cancer cell lines (19 KRAS normal types and 16 KRAS mutant types) were cultured and then collected. The respective cell lines were placed in 1.5-ml tubes at 1×10⁶ to 2×10⁶ cells, to obtain cell pellets. Then, 1 ml of Trizol was added to each sample, and then the cells were pulverized by pipetting. Then, 200 ul of chloroform was added thereto, gentle mixing was performed for 15 seconds, and then the reaction was allowed to proceed at room temperature for 3 minutes. The reaction samples were centrifuged with 12,000xg for 15 minutes at 4°C using a centrifuge. The clear supernatant was placed in a fresh 1.5-ml tube. Then, 0.5 ml of isopropanol was added thereto, gentle mixing was performed for 15 seconds, and then reaction was allowed to proceed at room temperature for 10 minutes.

The reaction sample was centrifuged with 12,000xg for 1 minute at 4 °C using a centrifuge, to identify white RNA pellet at the bottom of the tube. Then, the supernatant was removed. Then, 1 ml of 75% Et-OH was added thereto, mixing was performed for 10 seconds using a vortex mixer, and the resultant was centrifuged with 12,000xg for 10 minutes at 4°C. Subsequently, the RNA pellet was identified, and the supernatant was removed. The tube was inverted and dried at room temperature so that there was no liquid. The moisture-eliminated RNA pellet was dissolved in 50 ul to 100 ul of RNase-free water. Then, the extracted total RNA was measured using nano-drop equipment, and 1 ug of total RNA and 1 ul of oligo dT were mixed in a new tube and reacted at a temperature of 70°C for 5 minutes using a heat block or water bath. Then, the mixture was cooled on ice.

cDNA was synthesized using cDNA synthesis kit tube (AccuPower CycleScript RT PreMix, BIONEER CORPORATION). The synthesized cDNA was subjected to RT-PCR using RT-PCR premix kit tube (AccuPower Gold Multiplex PCR PreMix, BIONEER CORPORATION), and forward primer (10 pmol) and reverse primer (10 pmol) for amplification of RON exon5-6 (RON d5_6) or exon11 (RON d11). Then, the amplified product was electrophoresed, and then sequencing was performed.

**[Table 9]**

| **Item** | **Sequence** |
|---|---|
| RON d5_6 sense primer | 5' -GAGCTGGTCAGGTCACTAAAC-3' (SEQ ID NO: 1) |
| RON d5_6 antisense primer | 5'-CAGACACTCAGTCCCATTGAC-3'(SEQ ID NO: 2) |
| RON d11 sense primer | 5'-ATCTGTGGCCAGCATCTAAC-3'(SEQ ID NO: 3) |
| RON d11 antisense primer | 5'-AAAGGCAGCAGGATACCAAG-3'(SEQ ID NO: 4) |

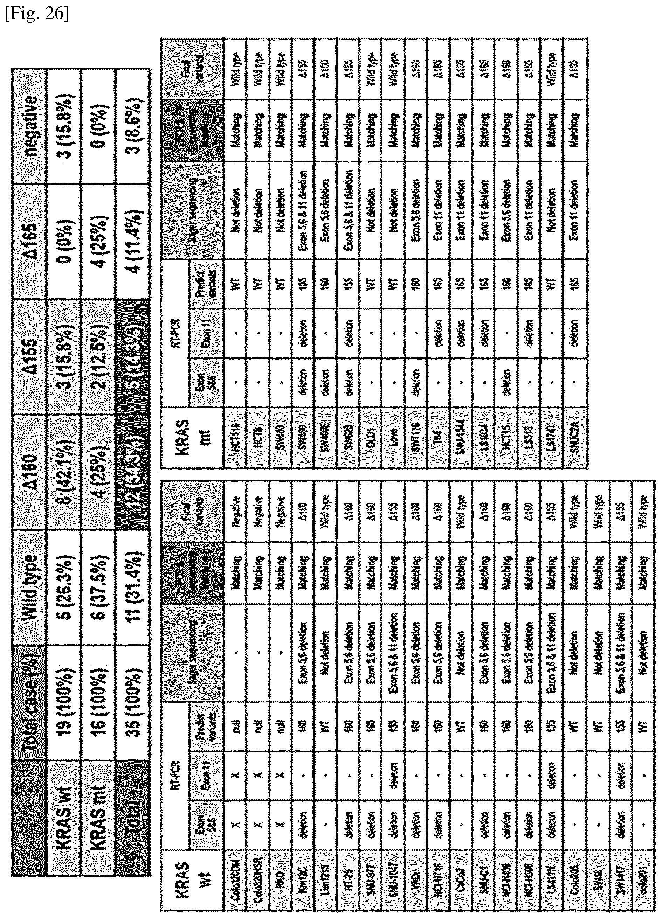

As a result, as illustrated in Fig. 26, it was analyzed that among 35 colon cancer cell lines, the percentages of the RON normal gene (wild type) and the RON active mutant gene (mutant type; Δ160, Δ155, Δ165) are as follows: 11 (31.4%) cell lines carrying RON normal gene, 21 (60%) cell lines carrying RON active mutant gene, and 3 (8.6%) cell lines in which no RON gene is expressed.

In addition, in a case where classification is made based on KRAS genotypes (normal type and mutant type), it was analyzed that among 19 KRAS normal cell lines, there are 5 (26.3%) cell lines carrying RON normal gene, 11 (57.9%) cell lines carrying RON active mutant gene, and 3 (15.8%) cell lines in which no RON gene is expressed. It was analyzed that among 16 KRAS mutant type cell lines, there are 6 (37.5%) cell lines carrying RON normal gene and 10 (62.5%) cell lines carrying RON active mutant gene, and any cell line in which RON gene is not expressed is not found.

### Experimental Example 17. Analysis of RON and KRAS biomarker genes in colon cancer patients: Tissues from Korean patients

401 cancer tissues from Korean colon cancer patients, provided by the Bio-Resource Center (BRC) of Asan Medical Center according to the research plan approved by Asan Medical Center Institutional Review Board (IRB), were cut into sizes of 0.5 cm³ to 1 cm³ and placed in 1.5-ml tubes. Then, 1 ml of Trizol was added thereto, and then the tissues were pulverized for 5 to 10 minutes using a power drill-like homogenizer until no lump was visible. Then, 200 ul of chloroform was added thereto, gentle mixing was performed for 15 seconds, and then reaction was allowed to proceed at room temperature for 3 minutes. The reaction sample was centrifuged with 12,000xg for 15 minutes at 4°C using a centrifuge, and then the clear supernatant was placed in a fresh 1.5-ml tube. 0.5 ml of isopropanol was added thereto, gentle mixing was performed for 15 seconds, and then reaction was allowed to proceed at room temperature for 10 minutes.

cDNA was synthesized from mRNA in the patient tissue using cDNA synthesis kit tube (AccuPower CycleScript RT PreMix, BIONEER CORPORATION). The synthesized cDNA was subjected to RT-PCR using RT-PCR premix kit tube (AccuPower Gold Multiplex PCR PreMix, BIONEER CORPORATION), and forward primer (10 pmol) and reverse primer (10 pmol) for amplification of RON exon5-6 or exon11. Then, the amplified product was electrophoresed, and then sequencing was performed.

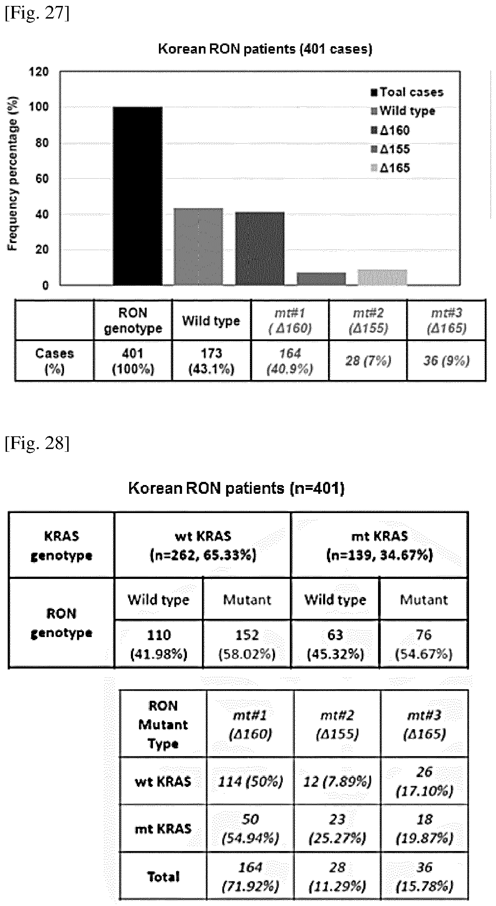

As a result, as illustrated in Fig. 27, it was identified that among 401 colon cancer patient tissues, the percentages of KRAS normal and mutant types, and RON normal gene (wild type) and RON active mutant gene (mutant type; Δ160, Δ155, Δ165) are as follows: 262 (65.33%) KRAS normal patient tissues and 139 (34.67%) KRAS mutant patient tissues. In addition, it was analyzed that among 262 KRAS normal patient tissues, there are 110 (41.98%) tissues carrying RON normal gene and 152 (58.02%) tissues carrying RON active mutant gene, and that among 139 KRAS mutant patient tissues, there are 63 (45.32%) tissues carrying RON normal gene and 76 (54.67%) tissues carrying RON active mutant gene (Fig. 28).

### Experimental Example 18. Analysis of RON mutants in colon cancer patient tissues: Caucasian patient tissues

182 cancer tissues from Caucasian colon cancer patients, purchased through a foreign company (Proteogenex), were cut into sizes of 0.5 cm³ to 1 cm³ and placed in 1.5-ml tubes. 1 ml of Trizol was added thereto, and then the tissues were pulverized for 5 to 10 minutes using a power drill-like homogenizer until no lump was visible. Then, 200 ul of chloroform was added thereto, gentle mixing was performed for 15 seconds, and then reaction was allowed to proceed at room temperature for 3 minutes. The reaction sample was centrifuged with 12,000xg for 15 minutes at 4°C using a centrifuge, and then the clear supernatant was placed in a fresh 1.5-ml tube. 0.5 ml of isopropanol was added thereto, gentle mixing was performed for 15 seconds, and then reaction was allowed to proceed at room temperature for 10 minutes.

cDNA was synthesized from mRNA in the patient tissue using cDNA synthesis kit tube (AccuPower CycleScript RT PreMix, BIONEER CORPORATION). The synthesized cDNA was subjected to RT-PCR using RT-PCR premix kit tube (AccuPower Gold Multiplex PCR PreMix, BIONEER CORPORATION), and forward primer (10 pmol) and reverse primer (10 pmol) for amplification of RON exon5-6 or exon11. Then, the amplified product was electrophoresed, and then sequencing was performed.

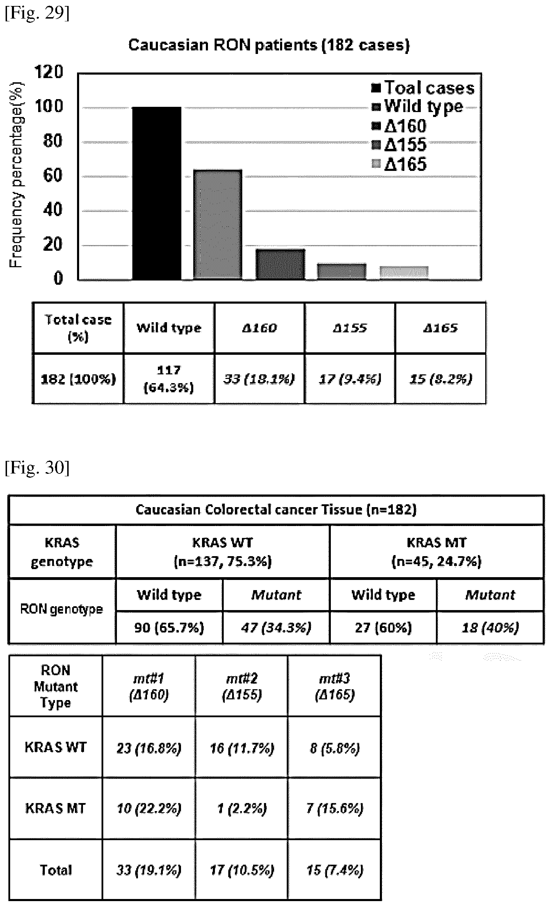

As a result, as illustrated in Fig. 29, it was identified that in a case where KRAS and RON mutants are analyzed in a total of 182 cases of Caucasian patient tissues, RON mutants are present at a percentage of about 34.3% in the patients with normal KRAS. In addition, it was identified that RON mutants are present at a percentage of about 40% in the patient tissues carrying KRAS mutant. As illustrated in the figure, the percentage for each mutant is 18.1% for Δ160, 9.4% for Δ155, and 8.2% for Δ165 (Fig. 30).

### Experimental Example 19. Analysis of RON and KRAS biomarker genes in tissues from an animal model transplanted with tumor cells in colon cancer patients

5 Cancer tissues from an animal model, which had been transplanted with cancer tissues from Korean colon cancer patients, were cut into sizes of 0.5 cm³ to 1 cm³ and placed in 1.5-ml tubes. 1 ml of Trizol was added thereto, and then the tissues were pulverized for 5 to 10 minutes using a power drill-like homogenizer until no lump was visible. Then, 200 ul of chloroform was added thereto, gentle mixing was performed for 15 seconds, and then reaction was allowed to proceed at room temperature for 3 minutes. The reaction sample was centrifuged with 12,000g for 15 minutes at 4°C using a centrifuge. The clear supernatant was placed in a new 1.5-ml tube. 0.5 ml of isopropanol was added thereto, gentle mixing was performed for 15 seconds, and then reaction was allowed to proceed at room temperature for 10 minutes.

cDNA was synthesized from mRNA in the patient tissue using cDNA synthesis kit tube (AccuPower CycleScript RT PreMix, BIONEER CORPORATION). The synthesized cDNA was subjected to RT-PCR using RT-PCR premix kit tube (AccuPower Gold Multiplex PCR PreMix, BIONEER CORPORATION), and forward primer (10 pmol) and reverse primer (10 pmol) for amplification of RON exon5-6 or exon11. Then, the amplified product was electrophoresed, and then sequencing was performed.

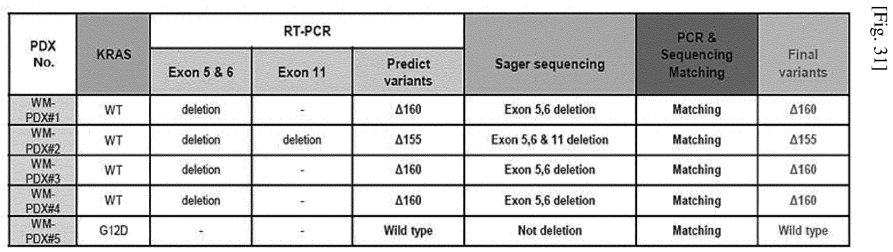

As a result, as illustrated in Fig. 31, it was identified that among 5 tissues from the animal model transplanted with tumor cells in colon cancer patients, the percentages of KRAS normal and mutant types, and RON normal gene (wild type) and RON active mutant gene (mutant type; Δ160, Δ155, Δ165) are as follows: 4 (80%) tissues from the animal model transplanted with tumor cells in KRAS normal colon cancer patients, and 1 (20%) tissue from KRAS mutant patients. On the other hand, among the 4 KRAS normal patient tissues, there was no tissue from the animal model transplanted with tumor cells in colon cancer patients carrying RON normal gene, and all 4 tissues (100%) were tissues from the animal model transplanted with tumor cells in colon cancer patients carrying RON active mutant gene. Regarding 1 tissue from KRAS mutant patients, there was 1 (100%) tissue from the animal model transplanted with tumor cells in colon cancer patients carrying RON normal gene, and no tissue from the animal model transplanted with tumor cells in colon cancer patients carrying RON active mutant gene was identified.

## Claims

1. A pharmaceutical composition for treating a cancer patient who is resistant to a protein kinase inhibitor, the composition comprising as an active ingredient:
a compound represented by Formula 1, or a pharmaceutically acceptable salt thereof: 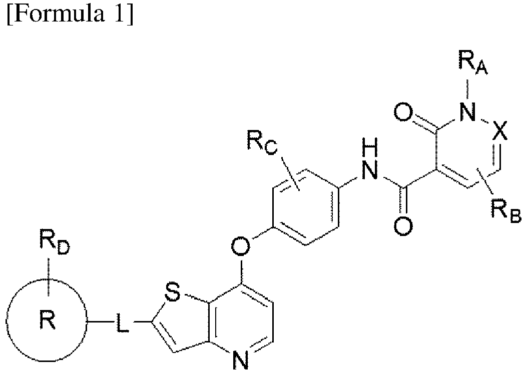
in the formula,
R_{A} is C₁₋₁₀ alkyl, phenyl, or benzyl, where R_{A} optionally has one or more substituents selected from halogen and C₁₋₁₀ alkoxy;
X is -C(-R_{B}')= or -N=;
R_{B} and R_{B}' are each independently H, halogen, C₁₋₁₀ alkyl, or C₁₋₁₀ alkoxy;
R_{C} is H, halogen, or C₁₋₁₀ alkyl;
L is a single bond or C₁₋₆ alkylene;
R is a 5- to 8-membered heterocycle having 1 or 2 heteroatoms selected from N and S;
R_{D} is H, C₁₋₁₀ alkyl, -(CH₂)ₙY-R_{G}, or -CH₂-NR_{E}R_{F};
R_{E} and R_{F} are each independently H, C₁₋₁₀ alkyl, or -(CH₂)ₙ-Y-R_{G}, or R_{E} and R_{F} are linked to each other to form a 4- to 10-membered heterocycle together with the N atom to which they are attached;
wherein n is an integer from 0 to 10; Y is -O-, -C(=O)-, -C (=O)-O-, -S-, or - S(=O)₂-; R_{G} is linear or branched C₁₋₁₀ alkyl, provided that R_{G} is unsubstituted or substituted with one or more substituents selected from the group consisting of halogen, amino, hydroxyl, and C₁₋₆ alkoxy; and
the 4- to 10-membered heterocycle optionally further contains one or two heteroatoms selected from the group consisting of N, O, and S, in addition to the N atom to which R_{E} and R_{F} are attached, and is unsubstituted or substituted with one or more substituents selected from halogen and C₁₋₆ alkyl.

2. The pharmaceutical composition of claim 1, wherein in the compound, R_{A} is methyl, phenyl, halobenzyl, or halophenyl.

3. The pharmaceutical composition of claim 1, wherein in the compound, R_{B} and R_{B}' are each independently H, methyl, methoxy, or ethoxy.

4. The pharmaceutical composition of claim 1, wherein in the compound, X is - C(-R_{B}')=; and
R_{B} and R_{B}' are each independently H, methyl, methoxy, or ethoxy, provided that R_{B} and R_{B}' are not H at the same time.

5. The pharmaceutical composition of claim 1, wherein in the compound, R_{C} is H or halogen.

6. The pharmaceutical composition of claim 1, wherein in the compound, L is a single bond or methylene; and
R is imidazolyl, pyrazolyl, pyridinyl, piperazinyl, or thiazolyl.

7. The pharmaceutical composition of claim 1, wherein in the compound, R_{D} is H, methyl, ethyl, methoxymethyl, or -CH₂-NR_{E}R_{F}; and
R_{E} and R_{F} are each independently H, C₁₋₆ alkyl, or -C₁₋₆ alkylene-O-Ci-io alkyl, provided that R_{E} and R_{F} are not H at the same time.

8. The pharmaceutical composition of claim 1, wherein in the compound, R_{E} and R_{F} together with the N atom to which they are attached form 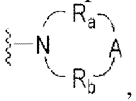 where Rₐ and R_{b} are each independently C₁₋₃ alkylene; and A is -N(-C₁₋₆ alkyl)- or -O-.

9. The pharmaceutical composition of claim 1, wherein in the compound, R_{A} is methyl, phenyl, halobenzyl, or halophenyl;
X is -CH=;
R_{B} is H, methyl, methoxy, or ethoxy;
R_{C} is H or halogen;
L is a single bond or methylene;
R is imidazolyl, pyrazolyl, pyridinyl, thiazolyl, or piperazinyl;
R_{D} is H, methyl, ethyl, methoxymethyl, or -CH₂-NR_{E}R_{F}, where R_{E} is -C₂H₄-O-CH₃ and R_{F} is H or methyl, or R_{E} and R_{F} are linked to each other to form morpholino or methylpiperazinyl together with the N atom to which they are attached.

10. The pharmaceutical composition of claim 1, wherein the compound represented by Formula 1 is the following compound:
1) 4-Ethoxy-N-(3-fluoro-4-(2-(1-methyl-1H-imidazol-4-yl)thieno[3,2-b]pyridin-7-yloxy)phenyl)-1-(4-fluorophenyl)-2-oxo-1,2-dihydropyridine-3-carboxamide;
2) 4-Ethoxy-N-(3-fluoro-4-(2-(1-methyl-1H-imidazol-4-yl)thieno[3,2-b]pyridin-7-yloxy)phenyl)-1-methyl-2-oxo-1,2-dihydropyridine-3-carboxamide;
3) 4-Ethoxy-N-(3-fluoro-4-(2-(1-methyl-1H-imidazol-2-yl)thieno[3,2-b]pyridin-7-yloxy)phenyl)-1-(4-fluorophenyl)-2-oxo-1,2-dihydropyridine-3-carboxamide;
4) 4-Ethoxy-N-(3-fluoro-4-(2-(1-methyl-1H-pyrazol-4-yl)thieno[3,2-b]pyridin-7-yloxy)phenyl)-1-(4-fluorophenyl)-2-oxo-1,2-dihydropyridine-3-carboxamide;
5) 4-Ethoxy-N-(3-fluoro-4-(2-(pyridin-2-yl)thieno[3,2-b]pyridin-7-yloxy)phenyl)-1-(4-fluorophenyl)-2-oxo-1,2-dihydropyridine-3-carboxamide;
6) 4-Ethoxy-N-(3-fluoro-4-(2-(pyridin-3-yl)thieno[3,2-b]pyridin-7-yloxy)phenyl)-1-(4-fluorophenyl)-2-oxo-1,2-dihydropyridine-3-carboxamide;
7) 4-Ethoxy-N-(3-fluoro-4-(2-(thiazol-2-yl)thieno[3,2-b]pyridin-7-yloxy)phenyl)-1-(4-fluorophenyl)-2-oxo-1,2-dihydropyridine-3-carboxamide;
8) 4-Ethoxy-N-(4-(2-(1-ethyl-1H-imidazol-4-yl)thieno[3,2-b]pyridin-7-yloxy)-3-fluorophenyl)-1-(4-fluorobenzyl)-2-oxo-1,2-dihydropyridine-3-carboxamide;
9) 4-Methoxy-N-(3-fluoro-4-(2-(1-(methoxymethyl)-1H-imidazol-4-yl)thieno [3,2-b]pyridine-7-yloxy)phenyl)-1-phenyl-2-oxo-1,2-dihydropyridine-3-carboxamide;
10) 4-Ethoxy-N-(3-fluoro-4-(2-(piperazin-1-ylmethyl)thieno[3,2-b]pyridin-7-yloxy)phenyl)-1-(4-fluorophenyl)-2-oxo-1,2-dihydropyridine-3-carboxamide;
11) 4-Ethoxy-N-(3-fluoro-4-(2-((4-methylpiperazin-1-yl)methyl)thieno[3,2-blpyridin-7-yloxy)phenyl)-1-(4-fluorophenyl)-2-oxo-1,2-dihydropyridine-3-carboxamide;
12) 4-Ethoxy-N-(3-fluoro-4-{ [2-(5-{ [(2-methoxyethyl)amino]methyl}pyridin-2-yl)thieno[3,2-b ]pyridin-7-yl]oxy}phenyl)-2-oxo-1-phenyl-1,2-dihydropyridine-3-carboxamide;
13) 4-Ethoxy-N-(3-fluoro-4-{ [2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3-carboxamide;
14) N-(3-Chloro-4-{ [2-(5-{ [(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3-carboxamide;
15) 4-Ethoxy-N-(3-fluoro-4-{ [2-(5-{ [(2-methoxyethyl)amino]methyl}pyridin-2-yl)thieno[3,2-b]pyridin-7-yl]oxy}phenyl)-1-(3-fluorophenyl)-2-oxo-1,2-dihydropyridine-3-carboxamide;
16) N-(3-Fluoro-4-{ [2-(5-{ [(2-methoxyethyl)amino]methyl}pyridin-2-yl)thieno[3,2-b]pyridine-7-yl]oxy}phenyl)-1-(4-fluorophenyl)-4-methyl-2-oxo-1,2-dihydropyridine-3-carboxamide;
17) 4-Ethoxy-N-(3-fluoro-4-{ [2-(5-{ [(2-methoxyethyl) (methyl)amino]methyl }pyridin-2-yl)thieno[3,2-b]pyridin-7-yl]oxy}phenyl)-1-(4-fluorophenyl)-2-oxo-1,2-dihydropyridine-3-carboxamide;
18) 4-Ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3-carboxamide;
19) 4-Ethoxy-N- [3 -fluoro-4- ({2- [5- (morpholinomethyl)pyridin-2-yl]thieno[3,2-b]pyridin-7-yl}oxy)phenyl]-2-oxo-1-phenyl-1,2-dihydropyridine-3-carboxamide;
20) 4-Ethoxy-N-{3-fluoro-4-[(2-{5-[(4-methylpiperazin-1-yl)methyl]pyridin-2-yl}thieno[3,2-b]pyridin-7-yl)oxy]phenyl}-1-(4-fluorophenyl)-2-oxo-1,2-dihydropyridine-3-carboxamide; or
21) 4-Ethoxy-N-{3-fluoro-4-[(2-{5-[(4-methylpiperazin-1 -yl)methyl]pyridin-2-yl}thieno[3,2-b]pyridin-7-yl)oxy]phenyl}-2-oxo-1-phenyl-1,2-dihydropyridine-3-carboxamide.

11. The pharmaceutical composition of claim 1, wherein the protein kinase is EGFR.

12. The pharmaceutical composition of claim 1, wherein the patient carries active RON (Recepteur d'Origine Nantais).

13. The pharmaceutical composition of claim 1, wherein the active RON is any one selected from the group consisting of RONΔ155, RONΔ160, RONΔ165, and combinations thereof.

14. The pharmaceutical composition of claim 1, wherein the patient carries normal KRAS.

15. The pharmaceutical composition of claim 1, wherein the protein kinase inhibitor is cetuximab.

16. The pharmaceutical composition of claim 1, wherein the cancer is selected from the group consisting of breast cancer, lung cancer, gastric cancer, prostate cancer, uterine cancer, ovarian cancer, kidney cancer, pancreatic cancer, liver cancer, colon cancer, skin cancer, head and neck cancer, and thyroid cancer.

17. The pharmaceutical composition of claim 1, further comprising:
a protein kinase inhibitor.

18. The pharmaceutical composition of claim 1, wherein the protein kinase inhibitor is an EGFR inhibitor.

19. The pharmaceutical composition of claim 18, wherein the EGFR inhibitor is cetuximab.

20. A method for treating a patient who is resistant to a protein kinase inhibitor, comprising steps of:
i) identifying whether a cancer patient carries active RON; and
ii) administering, to a patient carrying active RON, an anticancer agent that comprises, as an active ingredient, the compound represented by Formula I as described above, or a pharmaceutically acceptable salt thereof.


<110>	Wellmarker Bio Co., LTD.

<120>	A biomarker driven therapeutic agent

<130>	PCB903029WMB

<160>	4

<170>	KoPatentIn 3.0

<210>	1
<211>	21
<212>	DNA
<213>	Artificial Sequence

<220>
<223>	RON d5_6 sense primer


<400>	1
gagctggtca ggtcactaaa c	21


<210>	2
<211>	21
<212>	DNA
<213>	Artificial Sequence

<220>
<223>	RON d5_6 antisense primer


<400>	2
cagacactca gtcccattga c	21


<210>	3
<211>	20
<212>	DNA
<213>	Artificial Sequence

<220>
<223>	RON d11 sense primer


<400>	3
atctgtggcc agcatctaac	20


<210>	4
<211>	20
<212>	DNA
<213>	Artificial Sequence

<220>
<223>	RON d11 antisense primer


<400>	4
aaaggcagca ggataccaag	20